(19) Europäisches Patentamt / European Patent Office / Office européen des brevets

(11) **EP 3 288 970 B1**

(12) **EUROPEAN PATENT SPECIFICATION**

(45) Date of publication and mention
of the grant of the patent:
**19.02.2020 Bulletin 2020/08**

(51) Int Cl.:
*C07K 14/705* [(2006.01)]

(21) Application number: **16721211.7**

(22) Date of filing: **26.04.2016**

(86) International application number:
**PCT/GB2016/051162**

(87) International publication number:
**WO 2016/174406 (03.11.2016 Gazette 2016/44)**

(54) **NUCLEIC ACID CONSTRUCT FOR EXPRESSING MORE THAN ONE CHIMERIC ANTIGEN RECEPTOR**

NUKLEINSÄURE ZUR EXPRESSION VON MEHREREN CHIMÄREN ANTIGENREZEPTOREN

CONSTRUCTION D'ACIDE NUCLEIQUE POUR L'EXPRESSION DE PLUSIEURS RECEPTEURS D'ANTIGENES CHIMERIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.04.2015 GB 201507115**

(43) Date of publication of application:
**07.03.2018 Bulletin 2018/10**

(73) Proprietor: **UCL Business Ltd
London W1T 4TP (GB)**

(72) Inventors:
• **PULÉ, Martin
London W12 7RP (GB)**
• **CORDOBA, Shaun
London W12 7RP (GB)**

(74) Representative: **D Young & Co LLP
120 Holborn
London EC1N 2DY (GB)**

(56) References cited:
WO-A1-2014/127261    WO-A1-2014/184143
WO-A1-2015/052538    WO-A1-2015/075468
WO-A1-2016/102965    WO-A2-2013/063419

• **CHRISTOPHER C KLOSS ET AL: "Combinatorial antigen recognition with balanced signaling promotes selective tumor eradication by engineered T cells", NATURE BIOTECHNOLOGY, vol. 31, no. 1, 16 December 2012 (2012-12-16), pages 71-75, XP055130697, ISSN: 1087-0156, DOI: 10.1038/nbt.2459 cited in the application**
• **V. D. FEDOROV ET AL: "PD-1- and CTLA-4-Based Inhibitory Chimeric Antigen Receptors (iCARs) Divert Off-Target Immunotherapy Responses", SCIENCE TRANSLATIONAL MEDICINE, vol. 5, no. 215, 11 December 2013 (2013-12-11), pages 215ra172-215ra172, XP055210508, ISSN: 1946-6234, DOI: 10.1126/scitranslmed.3006597**
• **E. LANITIS ET AL: "Chimeric Antigen Receptor T Cells with Dissociated Signaling Domains Exhibit Focused Antitumor Activity with Reduced Potential for Toxicity In Vivo", CANCER IMMUNOLOGY RESEARCH, vol. 1, no. 1, 7 April 2013 (2013-04-07), pages 43-53, XP055170367, ISSN: 2326-6066, DOI: 10.1158/2326-6066.CIR-13-0008**
• **SCOTT WILKIE ET AL: "Dual Targeting of ErbB2 and MUC1 in Breast Cancer Using Chimeric Antigen Receptors Engineered to Provide Complementary Signaling", JOURNAL OF CLINICAL IMMUNOLOGY, KLUWER ACADEMIC PUBLISHERS-PLENUM PUBLISHERS, NE, vol. 32, no. 5, 17 April 2012 (2012-04-17) , pages 1059-1070, XP035113362, ISSN: 1573-2592, DOI: 10.1007/S10875-012-9689-9 cited in the application**

**(Cont. next page)**

- MICHAEL C JENSEN ET AL: "Designing chimeric antigen receptors to effectively and safely target tumors", CURRENT OPINION IN IMMUNOLOGY, vol. 33, 23 January 2015 (2015-01-23), pages 9-15, XP055217152, ISSN: 0952-7915, DOI: 10.1016/j.coi.2015.01.002
- M. SHARPE ET AL: "Genetically modified T cells in cancer therapy: opportunities and challenges", DISEASE MODELS & MECHANISMS, vol. 8, no. 4, 1 April 2015 (2015-04-01), pages 337-350, XP055288112, GB ISSN: 1754-8403, DOI: 10.1242/dmm.018036
- J. N. KOCHENDERFER ET AL: "Chemotherapy-Refractory Diffuse Large B-Cell Lymphoma and Indolent B-Cell Malignancies Can Be Effectively Treated With Autologous T Cells Expressing an Anti-CD19 Chimeric Antigen Receptor", JOURNAL OF CLINICAL ONCOLOGY, vol. 33, no. 6, 25 August 2014 (2014-08-25), pages 540-549, XP055193969, ISSN: 0732-183X, DOI: 10.1200/JCO.2014.56.2025
- M. SADELAIN ET AL: "The Basic Principles of Chimeric Antigen Receptor Design", CANCER DISCOVERY, vol. 3, no. 4, 1 April 2013 (2013-04-01), pages 388-398, XP055287277, US ISSN: 2159-8274, DOI: 10.1158/2159-8290.CD-12-0548
- SHAUN CORDOBA ET AL: "Chimeric Antigen Receptor Logical AND Gate Based on CD45/CD148 Phosphatases", MOLECULAR THERAPY, vol. 22, no. Suppl. 1, 1 May 2014 (2014-05-01), page S59, XP055170616, ISSN: 1525-0016

SEQUENCE LISTING

<110> UCL Business PLC

<120> NUCLEIC ACID CONSTRUCT

<130> P106292PCT

<150> GB1507115.2
<151> 2015-04-27

<160> 239

<170> PatentIn version 3.5

<210> 1
<211> 7
<212> PRT
<213> Artificial Sequence

<220>
<223> Tyrosinase-related protein (TYRP)-1 intracellular retention signal

<400> 1

```
Asn Gln Pro Leu Leu Thr Asp
1               5
```

<210> 2
<211> 15
<212> PRT
<213> Artificial Sequence

<220>
<223> Adenoviral E3/19K intracellular retention signal

<400> 2

```
Lys Tyr Lys Ser Arg Arg Ser Phe Ile Asp Glu Lys Lys Met Pro
1               5                   10                  15
```

<210> 3
<211> 6
<212> PRT
<213> Artificial Sequence

<220>
<223> Adenoviral E3/19K intracellular retention signal

<400> 3

```
Asp Glu Lys Lys Met Pro
1               5
```

<210> 4
<211> 1350
<212> PRT
<213> Artificial Sequence

<220>
<223> CAR AND gate which recognizes CD19 AND CD33 using a CD148 phosphatase

<400> 4

```
Met Ser Leu Pro Val Thr Ala Leu Leu Leu Pro Leu Ala Leu Leu Leu
1             5             10            15

His Ala Ala Arg Pro Asp Ile Gln Met Thr Gln Thr Thr Ser Ser Leu
            20            25            30

Ser Ala Ser Leu Gly Asp Arg Val Thr Ile Ser Cys Arg Ala Ser Gln
        35            40            45

Asp Ile Ser Lys Tyr Leu Asn Trp Tyr Gln Gln Lys Pro Asp Gly Thr
    50            55            60

Val Lys Leu Leu Ile Tyr His Thr Ser Arg Leu His Ser Gly Val Pro
65            70            75            80

Ser Arg Phe Ser Gly Ser Gly Ser Gly Thr Asp Tyr Ser Leu Thr Ile
            85            90            95

Ser Asn Leu Glu Gln Glu Asp Ile Ala Thr Tyr Phe Cys Gln Gln Gly
        100           105           110

Asn Thr Leu Pro Tyr Thr Phe Gly Gly Gly Thr Lys Leu Glu Ile Thr
        115           120           125

Lys Ala Gly Gly Gly Gly Ser Gly Gly Gly Gly Ser Gly Gly Gly Gly
    130           135           140

Ser Gly Gly Gly Gly Ser Glu Val Lys Leu Gln Glu Ser Gly Pro Gly
145           150           155           160

Leu Val Ala Pro Ser Gln Ser Leu Ser Val Thr Cys Thr Val Ser Gly
            165           170           175

Val Ser Leu Pro Asp Tyr Gly Val Ser Trp Ile Arg Gln Pro Pro Arg
        180           185           190

Lys Gly Leu Glu Trp Leu Gly Val Ile Trp Gly Ser Glu Thr Thr Tyr
        195           200           205

Tyr Asn Ser Ala Leu Lys Ser Arg Leu Thr Ile Ile Lys Asp Asn Ser
    210           215           220

Lys Ser Gln Val Phe Leu Lys Met Asn Ser Leu Gln Thr Asp Asp Thr
```

|     |     |     | 225 |     |     |     | 230 |     |     |     | 235 |     |     |     | 240 |
|-----|-----|-----|-----|-----|-----|-----|-----|-----|-----|-----|-----|-----|-----|-----|-----|

Ala Ile Tyr Tyr Cys Ala Lys His Tyr Tyr Tyr Gly Gly Ser Tyr Ala
            245                 250                 255

Met Asp Tyr Trp Gly Gln Gly Thr Ser Val Thr Val Ser Ser Asp Pro
            260                 265                 270

Thr Thr Thr Pro Ala Pro Arg Pro Pro Thr Pro Ala Pro Thr Ile Ala
            275                 280                 285

Ser Gln Pro Leu Ser Leu Arg Pro Glu Ala Cys Arg Pro Ala Ala Gly
            290                 295                 300

Gly Ala Val His Thr Arg Gly Leu Asp Phe Ala Cys Asp Ile Phe Trp
305                 310                 315                 320

Val Leu Val Val Val Gly Gly Val Leu Ala Cys Tyr Ser Leu Leu Val
            325                 330                 335

Thr Val Ala Phe Ile Ile Phe Trp Val Arg Arg Val Lys Phe Ser Arg
            340                 345                 350

Ser Ala Asp Ala Pro Ala Tyr Gln Gln Gly Gln Asn Gln Leu Tyr Asn
            355                 360                 365

Glu Leu Asn Leu Gly Arg Arg Glu Glu Tyr Asp Val Leu Asp Lys Arg
            370                 375                 380

Arg Gly Arg Asp Pro Glu Met Gly Gly Lys Pro Arg Arg Lys Asn Pro
385                 390                 395                 400

Gln Glu Gly Leu Tyr Asn Glu Leu Gln Lys Asp Lys Met Ala Glu Ala
            405                 410                 415

Tyr Ser Glu Ile Gly Met Lys Gly Glu Arg Arg Arg Gly Lys Gly His
            420                 425                 430

Asp Gly Leu Tyr Gln Gly Leu Ser Thr Ala Thr Lys Asp Thr Tyr Asp
            435                 440                 445

Ala Leu His Met Gln Ala Leu Pro Pro Arg Arg Ala Glu Gly Arg Gly
            450                 455                 460

Ser Leu Leu Thr Cys Gly Asp Val Glu Glu Asn Pro Gly Pro Met Ala
465                 470                 475                 480

```
Val Pro Thr Gln Val Leu Gly Leu Leu Leu Leu Trp Leu Thr Asp Ala
            485             490             495

Arg Cys Asp Ile Gln Met Thr Gln Ser Pro Ser Ser Leu Ser Ala Ser
            500             505             510

Val Gly Asp Arg Val Thr Ile Thr Cys Arg Ala Ser Glu Asp Ile Tyr
            515             520             525

Phe Asn Leu Val Trp Tyr Gln Gln Lys Pro Gly Lys Ala Pro Lys Leu
            530             535             540

Leu Ile Tyr Asp Thr Asn Arg Leu Ala Asp Gly Val Pro Ser Arg Phe
545             550             555             560

Ser Gly Ser Gly Ser Gly Thr Gln Tyr Thr Leu Thr Ile Ser Ser Leu
            565             570             575

Gln Pro Glu Asp Phe Ala Thr Tyr Tyr Cys Gln His Tyr Lys Asn Tyr
            580             585             590

Pro Leu Thr Phe Gly Gln Gly Thr Lys Leu Glu Ile Lys Arg Ser Gly
            595             600             605

Gly Gly Gly Ser Gly Gly Gly Gly Ser Gly Gly Gly Gly Ser Gly Gly
            610             615             620

Gly Gly Ser Arg Ser Glu Val Gln Leu Val Glu Ser Gly Gly Gly Leu
625             630             635             640

Val Gln Pro Gly Gly Ser Leu Arg Leu Ser Cys Ala Ala Ser Gly Phe
            645             650             655

Thr Leu Ser Asn Tyr Gly Met His Trp Ile Arg Gln Ala Pro Gly Lys
            660             665             670

Gly Leu Glu Trp Val Ser Ser Ile Ser Leu Asn Gly Gly Ser Thr Tyr
            675             680             685

Tyr Arg Asp Ser Val Lys Gly Arg Phe Thr Ile Ser Arg Asp Asn Ala
            690             695             700

Lys Ser Thr Leu Tyr Leu Gln Met Asn Ser Leu Arg Ala Glu Asp Thr
705             710             715             720

Ala Val Tyr Tyr Cys Ala Ala Gln Asp Ala Tyr Thr Gly Gly Tyr Phe
            725             730             735
```

44

Asp Tyr Trp Gly Gln Gly Thr Leu Val Thr Val Ser Ser Met Asp Pro
            740                 745             750

Ala Glu Pro Lys Ser Pro Asp Lys Thr His Thr Cys Pro Pro Cys Pro
            755                 760             765

Ala Pro Pro Val Ala Gly Pro Ser Val Phe Leu Phe Pro Pro Lys Pro
            770                 775             780

Lys Asp Thr Leu Met Ile Ala Arg Thr Pro Glu Val Thr Cys Val Val
785                 790                 795                 800

Val Asp Val Ser His Glu Asp Pro Glu Val Lys Phe Asn Trp Tyr Val
            805                 810             815

Asp Gly Val Glu Val His Asn Ala Lys Thr Lys Pro Arg Glu Glu Gln
            820                 825             830

Tyr Asn Ser Thr Tyr Arg Val Val Ser Val Leu Thr Val Leu His Gln
            835                 840             845

Asp Trp Leu Asn Gly Lys Glu Tyr Lys Cys Lys Val Ser Asn Lys Ala
            850                 855             860

Leu Pro Ala Pro Ile Glu Lys Thr Ile Ser Lys Ala Lys Gly Gln Pro
865                 870                 875                 880

Arg Glu Pro Gln Val Tyr Thr Leu Pro Pro Ser Arg Asp Glu Leu Thr
            885                 890             895

Lys Asn Gln Val Ser Leu Thr Cys Leu Val Lys Gly Phe Tyr Pro Ser
            900                 905             910

Asp Ile Ala Val Glu Trp Glu Ser Asn Gly Gln Pro Glu Asn Asn Tyr
            915                 920             925

Lys Thr Thr Pro Pro Val Leu Asp Ser Asp Gly Ser Phe Phe Leu Tyr
            930                 935             940

Ser Lys Leu Thr Val Asp Lys Ser Arg Trp Gln Gln Gly Asn Val Phe
945                 950                 955                 960

Ser Cys Ser Val Met His Glu Ala Leu His Asn His Tyr Thr Gln Lys
            965                 970             975

Ser Leu Ser Leu Ser Pro Gly Lys Lys Asp Pro Lys Ala Val Phe Gly
            980                 985             990

```
Cys Ile Phe Gly Ala Leu Val Ile   Val Thr Val Gly Gly   Phe Ile Phe
        995                 1000                 1005


Trp Arg Lys Lys Arg Lys Asp   Ala Lys Asn Asn Glu   Val Ser Phe
        1010            1015                 1020


Ser Gln Ile Lys Pro Lys Lys   Ser Lys Leu Ile Arg   Val Glu Asn
        1025            1030                 1035


Phe Glu Ala Tyr Phe Lys Lys   Gln Gln Ala Asp Ser   Asn Cys Gly
        1040            1045                 1050


Phe Ala Glu Glu Tyr Glu Asp   Leu Lys Leu Val Gly   Ile Ser Gln
        1055            1060                 1065


Pro Lys Tyr Ala Ala Glu Leu   Ala Glu Asn Arg Gly   Lys Asn Arg
        1070            1075                 1080


Tyr Asn Asn Val Leu Pro Tyr   Asp Ile Ser Arg Val   Lys Leu Ser
        1085            1090                 1095


Val Gln Thr His Ser Thr Asp   Asp Tyr Ile Asn Ala   Asn Tyr Met
        1100            1105                 1110


Pro Gly Tyr His Ser Lys Lys   Asp Phe Ile Ala Thr   Gln Gly Pro
        1115            1120                 1125


Leu Pro Asn Thr Leu Lys Asp   Phe Trp Arg Met Val   Trp Glu Lys
        1130            1135                 1140


Asn Val Tyr Ala Ile Ile Met   Leu Thr Lys Cys Val   Glu Gln Gly
        1145            1150                 1155


Arg Thr Lys Cys Glu Glu Tyr   Trp Pro Ser Lys Gln   Ala Gln Asp
        1160            1165                 1170


Tyr Gly Asp Ile Thr Val Ala   Met Thr Ser Glu Ile   Val Leu Pro
        1175            1180                 1185


Glu Trp Thr Ile Arg Asp Phe   Thr Val Lys Asn Ile   Gln Thr Ser
        1190            1195                 1200


Glu Ser His Pro Leu Arg Gln   Phe His Phe Thr Ser   Trp Pro Asp
        1205            1210                 1215


His Gly Val Pro Asp Thr Thr   Asp Leu Leu Ile Asn   Phe Arg Tyr
```

46

```
            1220                    1225                    1230


      Leu Val  Arg Asp Tyr Met Lys  Gln Ser Pro Pro Glu  Ser Pro Ile
          1235                    1240                    1245


      Leu Val  His Cys Ser Ala Gly  Val Gly Arg Thr Gly  Thr Phe Ile
          1250                    1255                    1260


      Ala Ile  Asp Arg Leu Ile Tyr  Gln Ile Glu Asn Glu  Asn Thr Val
          1265                    1270                    1275


      Asp Val  Tyr Gly Ile Val Tyr  Asp Leu Arg Met His  Arg Pro Leu
          1280                    1285                    1290


      Met Val  Gln Thr Glu Asp Gln  Tyr Val Phe Leu Asn  Gln Cys Val
          1295                    1300                    1305


      Leu Asp  Ile Val Arg Ser Gln  Lys Asp Ser Lys Val  Asp Leu Ile
          1310                    1315                    1320


      Tyr Gln  Asn Thr Thr Ala Met  Thr Ile Tyr Glu Asn  Leu Ala Pro
          1325                    1330                    1335


      Val Thr  Thr Phe Gly Lys Thr  Asn Gly Tyr Ile Ala
          1340                    1345                    1350
```

<210> 5
<211> 1717
<212> PRT
<213> Artificial Sequence

<220>
<223> CAR AND GATE which recognizes CD19 AND CD33 which uses a CD45 phosphatase

<400> 5

```
Met Ser Leu Pro Val Thr Ala Leu Leu Leu Pro Leu Ala Leu Leu Leu
1               5               10              15

His Ala Ala Arg Pro Asp Ile Gln Met Thr Gln Thr Thr Ser Ser Leu
            20              25              30

Ser Ala Ser Leu Gly Asp Arg Val Thr Ile Ser Cys Arg Ala Ser Gln
        35              40              45

Asp Ile Ser Lys Tyr Leu Asn Trp Tyr Gln Gln Lys Pro Asp Gly Thr
    50              55              60

Val Lys Leu Leu Ile Tyr His Thr Ser Arg Leu His Ser Gly Val Pro
```

65 70 75 80

Ser Arg Phe Ser Gly Ser Gly Ser Gly Thr Asp Tyr Ser Leu Thr Ile
85 90 95

Ser Asn Leu Glu Gln Glu Asp Ile Ala Thr Tyr Phe Cys Gln Gln Gly
100 105 110

Asn Thr Leu Pro Tyr Thr Phe Gly Gly Gly Thr Lys Leu Glu Ile Thr
115 120 125

Lys Ala Gly Gly Gly Gly Ser Gly Gly Gly Gly Ser Gly Gly Gly Gly
130 135 140

Ser Gly Gly Gly Gly Ser Glu Val Lys Leu Gln Glu Ser Gly Pro Gly
145 150 155 160

Leu Val Ala Pro Ser Gln Ser Leu Ser Val Thr Cys Thr Val Ser Gly
165 170 175

Val Ser Leu Pro Asp Tyr Gly Val Ser Trp Ile Arg Gln Pro Pro Arg
180 185 190

Lys Gly Leu Glu Trp Leu Gly Val Ile Trp Gly Ser Glu Thr Thr Tyr
195 200 205

Tyr Asn Ser Ala Leu Lys Ser Arg Leu Thr Ile Ile Lys Asp Asn Ser
210 215 220

Lys Ser Gln Val Phe Leu Lys Met Asn Ser Leu Gln Thr Asp Asp Thr
225 230 235 240

Ala Ile Tyr Tyr Cys Ala Lys His Tyr Tyr Tyr Gly Gly Ser Tyr Ala
245 250 255

Met Asp Tyr Trp Gly Gln Gly Thr Ser Val Thr Val Ser Ser Asp Pro
260 265 270

Thr Thr Thr Pro Ala Pro Arg Pro Pro Thr Pro Ala Pro Thr Ile Ala
275 280 285

Ser Gln Pro Leu Ser Leu Arg Pro Glu Ala Cys Arg Pro Ala Ala Gly
290 295 300

Gly Ala Val His Thr Arg Gly Leu Asp Phe Ala Cys Asp Ile Phe Trp
305 310 315 320

```
Val Leu Val Val Val Gly Gly Val Leu Ala Cys Tyr Ser Leu Leu Val
            325             330             335

Thr Val Ala Phe Ile Ile Phe Trp Val Arg Arg Val Lys Phe Ser Arg
            340             345             350

Ser Ala Asp Ala Pro Ala Tyr Gln Gln Gly Gln Asn Gln Leu Tyr Asn
            355             360             365

Glu Leu Asn Leu Gly Arg Arg Glu Glu Tyr Asp Val Leu Asp Lys Arg
    370             375             380

Arg Gly Arg Asp Pro Glu Met Gly Gly Lys Pro Arg Arg Lys Asn Pro
385             390             395             400

Gln Glu Gly Leu Tyr Asn Glu Leu Gln Lys Asp Lys Met Ala Glu Ala
            405             410             415

Tyr Ser Glu Ile Gly Met Lys Gly Glu Arg Arg Arg Gly Lys Gly His
            420             425             430

Asp Gly Leu Tyr Gln Gly Leu Ser Thr Ala Thr Lys Asp Thr Tyr Asp
            435             440             445

Ala Leu His Met Gln Ala Leu Pro Pro Arg Arg Ala Glu Gly Arg Gly
    450             455             460

Ser Leu Leu Thr Cys Gly Asp Val Glu Glu Asn Pro Gly Pro Met Ala
465             470             475             480

Val Pro Thr Gln Val Leu Gly Leu Leu Leu Leu Trp Leu Thr Asp Ala
            485             490             495

Arg Cys Asp Ile Gln Met Thr Gln Ser Pro Ser Ser Leu Ser Ala Ser
            500             505             510

Val Gly Asp Arg Val Thr Ile Thr Cys Arg Ala Ser Glu Asp Ile Tyr
            515             520             525

Phe Asn Leu Val Trp Tyr Gln Gln Lys Pro Gly Lys Ala Pro Lys Leu
    530             535             540

Leu Ile Tyr Asp Thr Asn Arg Leu Ala Asp Gly Val Pro Ser Arg Phe
545             550             555             560

Ser Gly Ser Gly Ser Gly Thr Gln Tyr Thr Leu Thr Ile Ser Ser Leu
            565             570             575
```

Gln Pro Glu Asp Phe Ala Thr Tyr Tyr Cys Gln His Tyr Lys Asn Tyr
580 585 590

Pro Leu Thr Phe Gly Gln Gly Thr Lys Leu Glu Ile Lys Arg Ser Gly
595 600 605

Gly Gly Gly Ser Gly Gly Gly Gly Ser Gly Gly Gly Ser Gly Gly
610 615 620

Gly Gly Ser Arg Ser Glu Val Gln Leu Val Glu Ser Gly Gly Gly Leu
625 630 635 640

Val Gln Pro Gly Gly Ser Leu Arg Leu Ser Cys Ala Ala Ser Gly Phe
645 650 655

Thr Leu Ser Asn Tyr Gly Met His Trp Ile Arg Gln Ala Pro Gly Lys
660 665 670

Gly Leu Glu Trp Val Ser Ser Ile Ser Leu Asn Gly Gly Ser Thr Tyr
675 680 685

Tyr Arg Asp Ser Val Lys Gly Arg Phe Thr Ile Ser Arg Asp Asn Ala
690 695 700

Lys Ser Thr Leu Tyr Leu Gln Met Asn Ser Leu Arg Ala Glu Asp Thr
705 710 715 720

Ala Val Tyr Tyr Cys Ala Ala Gln Asp Ala Tyr Thr Gly Gly Tyr Phe
725 730 735

Asp Tyr Trp Gly Gln Gly Thr Leu Val Thr Val Ser Ser Met Asp Pro
740 745 750

Ala Glu Pro Lys Ser Pro Asp Lys Thr His Thr Cys Pro Pro Cys Pro
755 760 765

Ala Pro Pro Val Ala Gly Pro Ser Val Phe Leu Phe Pro Pro Lys Pro
770 775 780

Lys Asp Thr Leu Met Ile Ala Arg Thr Pro Glu Val Thr Cys Val Val
785 790 795 800

Val Asp Val Ser His Glu Asp Pro Glu Val Lys Phe Asn Trp Tyr Val
805 810 815

Asp Gly Val Glu Val His Asn Ala Lys Thr Lys Pro Arg Glu Glu Gln
820 825 830

```
Tyr Asn Ser Thr Tyr Arg Val Val Ser Val Leu Thr Val Leu His Gln
        835                 840                 845


Asp Trp Leu Asn Gly Lys Glu Tyr Lys Cys Lys Val Ser Asn Lys Ala
    850                 855                 860


Leu Pro Ala Pro Ile Glu Lys Thr Ile Ser Lys Ala Lys Gly Gln Pro
865                 870                 875                 880


Arg Glu Pro Gln Val Tyr Thr Leu Pro Pro Ser Arg Asp Glu Leu Thr
                885                 890                 895


Lys Asn Gln Val Ser Leu Thr Cys Leu Val Lys Gly Phe Tyr Pro Ser
            900                 905                 910


Asp Ile Ala Val Glu Trp Glu Ser Asn Gly Gln Pro Glu Asn Asn Tyr
        915                 920                 925


Lys Thr Thr Pro Pro Val Leu Asp Ser Asp Gly Ser Phe Phe Leu Tyr
    930                 935                 940


Ser Lys Leu Thr Val Asp Lys Ser Arg Trp Gln Gln Gly Asn Val Phe
945                 950                 955                 960


Ser Cys Ser Val Met His Glu Ala Leu His Asn His Tyr Thr Gln Lys
            965                 970                 975


Ser Leu Ser Leu Ser Pro Gly Lys Lys Asp Pro Lys Ala Leu Ile Ala
            980                 985                 990


Phe Leu Ala Phe Leu Ile Ile Val  Thr Ser Ile Ala Leu  Leu Val Val
    995                 1000                1005


Leu Tyr  Lys Ile Tyr Asp Leu  His Lys Lys Arg Ser  Cys Asn Leu
    1010                1015                1020


Asp Glu  Gln Gln Glu Leu Val  Glu Arg Asp Asp Glu  Lys Gln Leu
    1025                1030                1035


Met Asn  Val Glu Pro Ile His  Ala Asp Ile Leu Leu  Glu Thr Tyr
    1040                1045                1050


Lys Arg  Lys Ile Ala Asp Glu  Gly Arg Leu Phe Leu  Ala Glu Phe
    1055                1060                1065


Gln Ser  Ile Pro Arg Val Phe  Ser Lys Phe Pro Ile  Lys Glu Ala
```

```
            1070                    1075                    1080

Arg Lys  Pro Phe Asn Gln Asn  Lys Asn Arg Tyr Val  Asp Ile Leu
    1085                1090                1095

Pro Tyr  Asp Tyr Asn Arg Val  Glu Leu Ser Glu Ile  Asn Gly Asp
    1100                1105                1110

Ala Gly  Ser Asn Tyr Ile Asn  Ala Ser Tyr Ile Asp  Gly Phe Lys
    1115                1120                1125

Glu Pro  Arg Lys Tyr Ile Ala  Ala Gln Gly Pro Arg  Asp Glu Thr
    1130                1135                1140

Val Asp  Asp Phe Trp Arg Met  Ile Trp Glu Gln Lys  Ala Thr Val
    1145                1150                1155

Ile Val  Met Val Thr Arg Cys  Glu Glu Gly Asn Arg  Asn Lys Cys
    1160                1165                1170

Ala Glu  Tyr Trp Pro Ser Met  Glu Glu Gly Thr Arg  Ala Phe Gly
    1175                1180                1185

Asp Val  Val Val Lys Ile Asn  Gln His Lys Arg Cys  Pro Asp Tyr
    1190                1195                1200

Ile Ile  Gln Lys Leu Asn Ile  Val Asn Lys Lys Glu  Lys Ala Thr
    1205                1210                1215

Gly Arg  Glu Val Thr His Ile  Gln Phe Thr Ser Trp  Pro Asp His
    1220                1225                1230

Gly Val  Pro Glu Asp Pro His  Leu Leu Leu Lys Leu  Arg Arg Arg
    1235                1240                1245

Val Asn  Ala Phe Ser Asn Phe  Phe Ser Gly Pro Ile  Val Val His
    1250                1255                1260

Cys Ser  Ala Gly Val Gly Arg  Thr Gly Thr Tyr Ile  Gly Ile Asp
    1265                1270                1275

Ala Met  Leu Glu Gly Leu Glu  Ala Glu Asn Lys Val  Asp Val Tyr
    1280                1285                1290

Gly Tyr  Val Val Lys Leu Arg  Arg Gln Arg Cys Leu  Met Val Gln
    1295                1300                1305
```

53

Val Glu Ala Gln Tyr Ile Leu Ile His Gln Ala Leu Val Glu Tyr
1310              1315              1320

Asn Gln Phe Gly Glu Thr Glu Val Asn Leu Ser Glu Leu His Pro
1325              1330              1335

Tyr Leu His Asn Met Lys Lys Arg Asp Pro Pro Ser Glu Pro Ser
1340              1345              1350

Pro Leu Glu Ala Glu Phe Gln Arg Leu Pro Ser Tyr Arg Ser Trp
1355              1360              1365

Arg Thr Gln His Ile Gly Asn Gln Glu Glu Asn Lys Ser Lys Asn
1370              1375              1380

Arg Asn Ser Asn Val Ile Pro Tyr Asp Tyr Asn Arg Val Pro Leu
1385              1390              1395

Lys His Glu Leu Glu Met Ser Lys Glu Ser Glu His Asp Ser Asp
1400              1405              1410

Glu Ser Ser Asp Asp Asp Ser Asp Ser Glu Glu Pro Ser Lys Tyr
1415              1420              1425

Ile Asn Ala Ser Phe Ile Met Ser Tyr Trp Lys Pro Glu Val Met
1430              1435              1440

Ile Ala Ala Gln Gly Pro Leu Lys Glu Thr Ile Gly Asp Phe Trp
1445              1450              1455

Gln Met Ile Phe Gln Arg Lys Val Lys Val Ile Val Met Leu Thr
1460              1465              1470

Glu Leu Lys His Gly Asp Gln Glu Ile Cys Ala Gln Tyr Trp Gly
1475              1480              1485

Glu Gly Lys Gln Thr Tyr Gly Asp Ile Glu Val Asp Leu Lys Asp
1490              1495              1500

Thr Asp Lys Ser Ser Thr Tyr Thr Leu Arg Val Phe Glu Leu Arg
1505              1510              1515

His Ser Lys Arg Lys Asp Ser Arg Thr Val Tyr Gln Tyr Gln Tyr
1520              1525              1530

Thr Asn Trp Ser Val Glu Gln Leu Pro Ala Glu Pro Lys Glu Leu
1535              1540              1545

54

```
Ile Ser  Met Ile Gln Val Val  Lys Gln Lys Leu Pro  Gln Lys Asn
    1550                1555            1560

Ser Ser  Glu Gly Asn Lys His  His Lys Ser Thr Pro  Leu Leu Ile
    1565                1570            1575

His Cys  Arg Asp Gly Ser Gln  Gln Thr Gly Ile Phe  Cys Ala Leu
    1580                1585            1590

Leu Asn  Leu Leu Glu Ser Ala  Glu Thr Glu Glu Val  Val Asp Ile
    1595                1600            1605

Phe Gln  Val Val Lys Ala Leu  Arg Lys Ala Arg Pro  Gly Met Val
    1610                1615            1620

Ser Thr  Phe Glu Gln Tyr Gln  Phe Leu Tyr Asp Val  Ile Ala Ser
    1625                1630            1635

Thr Tyr  Pro Ala Gln Asn Gly  Gln Val Lys Lys Asn  Asn His Gln
    1640                1645            1650

Glu Asp  Lys Ile Glu Phe Asp  Asn Glu Val Asp Lys  Val Lys Gln
    1655                1660            1665

Asp Ala  Asn Cys Val Asn Pro  Leu Gly Ala Pro Glu  Lys Leu Pro
    1670                1675            1680

Glu Ala  Lys Glu Gln Ala Glu  Gly Ser Glu Pro Thr  Ser Gly Thr
    1685                1690            1695

Glu Gly  Pro Glu His Ser Val  Asn Gly Pro Ala Ser  Pro Ala Leu
    1700                1705            1710

Asn Gln  Gly Ser
    1715
```

<210> 6
<211> 36
<212> PRT
<213> Artificial Sequence

<220>
<223> nucleic acid construct comprising a tyrp-1 retention protein sequence

<400> 6

```
Arg Ala Arg Arg Ser Met Asp Glu Ala Asn Gln Pro Leu Leu Thr Asp
1               5                   10                  15
```

```
        Gln Tyr Gln Cys Tyr Ala Glu Glu Tyr Glu Lys Leu Gln Asn Pro Asn
                    20                  25                  30


        Gln Ser Val Val
                    35
```

<210> 7
<211> 234
<212> PRT
<213> Artificial Sequence

<220>
<223> spacer sequence, hinge-CH2CH3 of human IgG1

<400> 7

```
        Ala Glu Pro Lys Ser Pro Asp Lys Thr His Thr Cys Pro Pro Cys Pro
        1               5                   10                  15

        Ala Pro Pro Val Ala Gly Pro Ser Val Phe Leu Phe Pro Pro Lys Pro
                    20                  25                  30

        Lys Asp Thr Leu Met Ile Ala Arg Thr Pro Glu Val Thr Cys Val Val
                    35                  40                  45

        Val Asp Val Ser His Glu Asp Pro Glu Val Lys Phe Asn Trp Tyr Val
                50                  55                  60

        Asp Gly Val Glu Val His Asn Ala Lys Thr Lys Pro Arg Glu Glu Gln
        65                  70                  75                  80

        Tyr Asn Ser Thr Tyr Arg Val Val Ser Val Leu Thr Val Leu His Gln
                        85                  90                  95

        Asp Trp Leu Asn Gly Lys Glu Tyr Lys Cys Lys Val Ser Asn Lys Ala
                    100                 105                 110

        Leu Pro Ala Pro Ile Glu Lys Thr Ile Ser Lys Ala Lys Gly Gln Pro
                    115                 120                 125

        Arg Glu Pro Gln Val Tyr Thr Leu Pro Pro Ser Arg Asp Glu Leu Thr
            130                 135                 140

        Lys Asn Gln Val Ser Leu Thr Cys Leu Val Lys Gly Phe Tyr Pro Ser
        145                 150                 155                 160

        Asp Ile Ala Val Glu Trp Glu Ser Asn Gly Gln Pro Glu Asn Asn Tyr
                        165                 170                 175
```

```
Lys Thr Thr Pro Pro Val Leu Asp Ser Asp Gly Ser Phe Phe Leu Tyr
        180             185                 190

Ser Lys Leu Thr Val Asp Lys Ser Arg Trp Gln Gln Gly Asn Val Phe
        195             200                 205

Ser Cys Ser Val Met His Glu Ala Leu His Asn His Tyr Thr Gln Lys
    210             215                 220

Ser Leu Ser Leu Ser Pro Gly Lys Lys Asp
225             230
```

<210> 8
<211> 46
<212> PRT
<213> Artificial Sequence

<220>
<223> spacer sequence, human CD8 stalk

<400> 8

```
Thr Thr Thr Pro Ala Pro Arg Pro Pro Thr Pro Ala Pro Thr Ile Ala
1           5               10              15

Ser Gln Pro Leu Ser Leu Arg Pro Glu Ala Cys Arg Pro Ala Ala Gly
        20              25              30

Gly Ala Val His Thr Arg Gly Leu Asp Phe Ala Cys Asp Ile
        35              40              45
```

<210> 9
<211> 20
<212> PRT
<213> Artificial Sequence

<220>
<223> spacer sequence, human IgG1 hinge

<400> 9

```
Ala Glu Pro Lys Ser Pro Asp Lys Thr His Thr Cys Pro Pro Cys Pro
1           5               10              15

Lys Asp Pro Lys
        20
```

<210> 10
<211> 185
<212> PRT
<213> Artificial Sequence

57

<220>
<223> spacer sequence, CD2 ectodomain

<400> 10

```
Lys Glu Ile Thr Asn Ala Leu Glu Thr Trp Gly Ala Leu Gly Gln Asp
1               5                   10              15

Ile Asn Leu Asp Ile Pro Ser Phe Gln Met Ser Asp Asp Ile Asp Asp
            20              25              30

Ile Lys Trp Glu Lys Thr Ser Asp Lys Lys Lys Ile Ala Gln Phe Arg
        35              40              45

Lys Glu Lys Glu Thr Phe Lys Glu Lys Asp Thr Tyr Lys Leu Phe Lys
        50              55              60

Asn Gly Thr Leu Lys Ile Lys His Leu Lys Thr Asp Asp Gln Asp Ile
65              70              75              80

Tyr Lys Val Ser Ile Tyr Asp Thr Lys Gly Lys Asn Val Leu Glu Lys
                85              90              95

Ile Phe Asp Leu Lys Ile Gln Glu Arg Val Ser Lys Pro Lys Ile Ser
            100             105             110

Trp Thr Cys Ile Asn Thr Thr Leu Thr Cys Glu Val Met Asn Gly Thr
        115             120             125

Asp Pro Glu Leu Asn Leu Tyr Gln Asp Gly Lys His Leu Lys Leu Ser
        130             135             140

Gln Arg Val Ile Thr His Lys Trp Thr Thr Ser Leu Ser Ala Lys Phe
145             150             155             160

Lys Cys Thr Ala Gly Asn Lys Val Ser Lys Glu Ser Ser Val Glu Pro
                165             170             175

Val Ser Cys Pro Glu Lys Gly Leu Asp
                180             185
```

<210> 11
<211> 259
<212> PRT
<213> Artificial Sequence

<220>
<223> spacer sequence, CD34 ectodomain

<400> 11

Ser Leu Asp Asn Asn Gly Thr Ala Thr Pro Glu Leu Pro Thr Gln Gly
1               5               10              15

Ser Leu Asp Asn Asn Gly Thr Ala Thr Pro Glu Leu Pro Thr Gln Gly

```
Thr Phe Ser Asn Val Ser Thr Asn Val Ser Tyr Gln Glu Thr Thr Thr
            20                  25              30

Pro Ser Thr Leu Gly Ser Thr Ser Leu His Pro Val Ser Gln His Gly
            35              40              45

Asn Glu Ala Thr Thr Asn Ile Thr Glu Thr Thr Val Lys Phe Thr Ser
            50              55              60

Thr Ser Val Ile Thr Ser Val Tyr Gly Asn Thr Asn Ser Ser Val Gln
65              70              75              80

Ser Gln Thr Ser Val Ile Ser Thr Val Phe Thr Thr Pro Ala Asn Val
            85              90              95

Ser Thr Pro Glu Thr Thr Leu Lys Pro Ser Leu Ser Pro Gly Asn Val
            100             105             110

Ser Asp Leu Ser Thr Thr Ser Thr Ser Leu Ala Thr Ser Pro Thr Lys
            115             120             125

Pro Tyr Thr Ser Ser Ser Pro Ile Leu Ser Asp Ile Lys Ala Glu Ile
    130             135             140

Lys Cys Ser Gly Ile Arg Glu Val Lys Leu Thr Gln Gly Ile Cys Leu
145             150             155             160

Glu Gln Asn Lys Thr Ser Ser Cys Ala Glu Phe Lys Lys Asp Arg Gly
                165             170             175

Glu Gly Leu Ala Arg Val Leu Cys Gly Glu Glu Gln Ala Asp Ala Asp
            180             185             190

Ala Gly Ala Gln Val Cys Ser Leu Leu Leu Ala Gln Ser Glu Val Arg
            195             200             205

Pro Gln Cys Leu Leu Leu Val Leu Ala Asn Arg Thr Glu Ile Ser Ser
    210             215             220

Lys Leu Gln Leu Met Lys Lys His Gln Ser Asp Leu Lys Lys Leu Gly
225             230             235             240

Ile Leu Asp Phe Thr Glu Gln Asp Val Ala Ser His Gln Ser Tyr Ser
            245             250             255

Gln Lys Thr
```

<210> 12
<211> 140
<212> PRT
<213> Artificial Sequence

<220>
<223> comprising CD28 transmembrane domain and CD3 Z endodomain

<400> 12

```
Phe Trp Val Leu Val Val Val Gly Gly Val Leu Ala Cys Tyr Ser Leu
1               5               10                  15

Leu Val Thr Val Ala Phe Ile Ile Phe Trp Val Arg Arg Val Lys Phe
            20              25                  30

Ser Arg Ser Ala Asp Ala Pro Ala Tyr Gln Gln Gly Gln Asn Gln Leu
        35              40                  45

Tyr Asn Glu Leu Asn Leu Gly Arg Arg Glu Glu Tyr Asp Val Leu Asp
    50              55                  60

Lys Arg Arg Gly Arg Asp Pro Glu Met Gly Gly Lys Pro Arg Arg Lys
65              70              75                  80

Asn Pro Gln Glu Gly Leu Tyr Asn Glu Leu Gln Lys Asp Lys Met Ala
                85              90                  95

Glu Ala Tyr Ser Glu Ile Gly Met Lys Gly Glu Arg Arg Arg Gly Lys
            100             105                 110

Gly His Asp Gly Leu Tyr Gln Gly Leu Ser Thr Ala Thr Lys Asp Thr
            115             120                 125

Tyr Asp Ala Leu His Met Gln Ala Leu Pro Pro Arg
    130             135                 140
```

<210> 13
<211> 180
<212> PRT
<213> Artificial Sequence

<220>
<223> comprising CD28 transmembrane domain and CD28 and CD3 Zeta endodomains

<400> 13

```
Phe Trp Val Leu Val Val Val Gly Gly Val Leu Ala Cys Tyr Ser Leu
1               5               10                  15
```

```
Leu Val Thr Val Ala Phe Ile Ile Phe Trp Val Arg Ser Lys Arg Ser
        20              25              30

Arg Leu Leu His Ser Asp Tyr Met Asn Met Thr Pro Arg Arg Pro Gly
        35              40              45

Pro Thr Arg Lys His Tyr Gln Pro Tyr Ala Pro Pro Arg Asp Phe Ala
        50              55              60

Ala Tyr Arg Ser Arg Val Lys Phe Ser Arg Ser Ala Asp Ala Pro Ala
65              70              75              80

Tyr Gln Gln Gly Gln Asn Gln Leu Tyr Asn Glu Leu Asn Leu Gly Arg
                85              90              95

Arg Glu Glu Tyr Asp Val Leu Asp Lys Arg Arg Gly Arg Asp Pro Glu
            100             105             110

Met Gly Gly Lys Pro Arg Arg Lys Asn Pro Gln Glu Gly Leu Tyr Asn
            115             120             125

Glu Leu Gln Lys Asp Lys Met Ala Glu Ala Tyr Ser Glu Ile Gly Met
    130             135             140

Lys Gly Glu Arg Arg Arg Gly Lys Gly His Asp Gly Leu Tyr Gln Gly
145             150             155             160

Leu Ser Thr Ala Thr Lys Asp Thr Tyr Asp Ala Leu His Met Gln Ala
                165             170             175

Leu Pro Pro Arg
            180
```

<210> 14
<211> 216
<212> PRT
<213> Artificial Sequence

<220>
<223> comprising CD28 transmembrane domain and CD28, OX40 and CD3 Zeta endodomains

<400> 14

```
Phe Trp Val Leu Val Val Val Gly Gly Val Leu Ala Cys Tyr Ser Leu
1           5               10              15

Leu Val Thr Val Ala Phe Ile Ile Phe Trp Val Arg Ser Lys Arg Ser
        20              25              30
```

```
Arg Leu Leu His Ser Asp Tyr Met Asn Met Thr Pro Arg Arg Pro Gly
        35                  40              45

Pro Thr Arg Lys His Tyr Gln Pro Tyr Ala Pro Pro Arg Asp Phe Ala
        50                  55              60

Ala Tyr Arg Ser Arg Asp Gln Arg Leu Pro Pro Asp Ala His Lys Pro
65              70              75              80

Pro Gly Gly Gly Ser Phe Arg Thr Pro Ile Gln Glu Glu Gln Ala Asp
                85              90              95

Ala His Ser Thr Leu Ala Lys Ile Arg Val Lys Phe Ser Arg Ser Ala
            100             105             110

Asp Ala Pro Ala Tyr Gln Gln Gly Gln Asn Gln Leu Tyr Asn Glu Leu
            115             120             125

Asn Leu Gly Arg Arg Glu Glu Tyr Asp Val Leu Asp Lys Arg Arg Gly
    130             135             140

Arg Asp Pro Glu Met Gly Gly Lys Pro Arg Arg Lys Asn Pro Gln Glu
145             150             155             160

Gly Leu Tyr Asn Glu Leu Gln Lys Asp Lys Met Ala Glu Ala Tyr Ser
                165             170             175

Glu Ile Gly Met Lys Gly Glu Arg Arg Arg Gly Lys Gly His Asp Gly
            180             185             190

Leu Tyr Gln Gly Leu Ser Thr Ala Thr Lys Asp Thr Tyr Asp Ala Leu
        195             200             205

His Met Gln Ala Leu Pro Pro Arg
210             215
```

<210> 15
<211> 729
<212> PRT
<213> Artificial Sequence

<220>
<223> CD45 trans-membrane and endodomain sequence

<400> 15

```
Ala Leu Ile Ala Phe Leu Ala Phe Leu Ile Ile Val Thr Ser Ile Ala
1               5               10              15
```

```
Leu Leu Val Val Leu Tyr Lys Ile Tyr Asp Leu His Lys Lys Arg Ser
        20              25              30

Cys Asn Leu Asp Glu Gln Gln Glu Leu Val Glu Arg Asp Asp Glu Lys
        35              40              45

Gln Leu Met Asn Val Glu Pro Ile His Ala Asp Ile Leu Leu Glu Thr
        50              55              60

Tyr Lys Arg Lys Ile Ala Asp Glu Gly Arg Leu Phe Leu Ala Glu Phe
65              70              75              80

Gln Ser Ile Pro Arg Val Phe Ser Lys Phe Pro Ile Lys Glu Ala Arg
            85              90              95

Lys Pro Phe Asn Gln Asn Lys Asn Arg Tyr Val Asp Ile Leu Pro Tyr
        100             105             110

Asp Tyr Asn Arg Val Glu Leu Ser Glu Ile Asn Gly Asp Ala Gly Ser
        115             120             125

Asn Tyr Ile Asn Ala Ser Tyr Ile Asp Gly Phe Lys Glu Pro Arg Lys
        130             135             140

Tyr Ile Ala Ala Gln Gly Pro Arg Asp Glu Thr Val Asp Asp Phe Trp
145             150             155             160

Arg Met Ile Trp Glu Gln Lys Ala Thr Val Ile Val Met Val Thr Arg
            165             170             175

Cys Glu Glu Gly Asn Arg Asn Lys Cys Ala Glu Tyr Trp Pro Ser Met
            180             185             190

Glu Glu Gly Thr Arg Ala Phe Gly Asp Val Val Val Lys Ile Asn Gln
            195             200             205

His Lys Arg Cys Pro Asp Tyr Ile Ile Gln Lys Leu Asn Ile Val Asn
        210             215             220

Lys Lys Glu Lys Ala Thr Gly Arg Glu Val Thr His Ile Gln Phe Thr
225             230             235             240

Ser Trp Pro Asp His Gly Val Pro Glu Asp Pro His Leu Leu Leu Lys
            245             250             255

Leu Arg Arg Arg Val Asn Ala Phe Ser Asn Phe Phe Ser Gly Pro Ile
            260             265             270
```

64

```
Val Val His Cys Ser Ala Gly Val Gly Arg Thr Gly Thr Tyr Ile Gly
        275             280             285

Ile Asp Ala Met Leu Glu Gly Leu Glu Ala Glu Asn Lys Val Asp Val
        290             295             300

Tyr Gly Tyr Val Val Lys Leu Arg Arg Gln Arg Cys Leu Met Val Gln
305             310             315             320

Val Glu Ala Gln Tyr Ile Leu Ile His Gln Ala Leu Val Glu Tyr Asn
            325             330             335

Gln Phe Gly Glu Thr Glu Val Asn Leu Ser Glu Leu His Pro Tyr Leu
        340             345             350

His Asn Met Lys Lys Arg Asp Pro Pro Ser Glu Pro Ser Pro Leu Glu
        355             360             365

Ala Glu Phe Gln Arg Leu Pro Ser Tyr Arg Ser Trp Arg Thr Gln His
        370             375             380

Ile Gly Asn Gln Glu Glu Asn Lys Ser Lys Asn Arg Asn Ser Asn Val
385             390             395             400

Ile Pro Tyr Asp Tyr Asn Arg Val Pro Leu Lys His Glu Leu Glu Met
            405             410             415

Ser Lys Glu Ser Glu His Asp Ser Asp Glu Ser Ser Asp Asp Asp Ser
            420             425             430

Asp Ser Glu Glu Pro Ser Lys Tyr Ile Asn Ala Ser Phe Ile Met Ser
        435             440             445

Tyr Trp Lys Pro Glu Val Met Ile Ala Ala Gln Gly Pro Leu Lys Glu
        450             455             460

Thr Ile Gly Asp Phe Trp Gln Met Ile Phe Gln Arg Lys Val Lys Val
465             470             475             480

Ile Val Met Leu Thr Glu Leu Lys His Gly Asp Gln Glu Ile Cys Ala
            485             490             495

Gln Tyr Trp Gly Glu Gly Lys Gln Thr Tyr Gly Asp Ile Glu Val Asp
            500             505             510

Leu Lys Asp Thr Asp Lys Ser Ser Thr Tyr Thr Leu Arg Val Phe Glu
        515             520             525
```

```
Leu Arg His Ser Lys Arg Lys Asp Ser Arg Thr Val Tyr Gln Tyr Gln
    530             535             540

Tyr Thr Asn Trp Ser Val Glu Gln Leu Pro Ala Glu Pro Lys Glu Leu
545             550             555                 560

Ile Ser Met Ile Gln Val Val Lys Gln Lys Leu Pro Gln Lys Asn Ser
                565             570             575

Ser Glu Gly Asn Lys His His Lys Ser Thr Pro Leu Leu Ile His Cys
            580             585             590

Arg Asp Gly Ser Gln Gln Thr Gly Ile Phe Cys Ala Leu Leu Asn Leu
            595             600             605

Leu Glu Ser Ala Glu Thr Glu Glu Val Val Asp Ile Phe Gln Val Val
    610             615             620

Lys Ala Leu Arg Lys Ala Arg Pro Gly Met Val Ser Thr Phe Glu Gln
625             630             635                 640

Tyr Gln Phe Leu Tyr Asp Val Ile Ala Ser Thr Tyr Pro Ala Gln Asn
            645             650             655

Gly Gln Val Lys Lys Asn Asn His Gln Glu Asp Lys Ile Glu Phe Asp
            660             665             670

Asn Glu Val Asp Lys Val Lys Gln Asp Ala Asn Cys Val Asn Pro Leu
            675             680             685

Gly Ala Pro Glu Lys Leu Pro Glu Ala Lys Glu Gln Ala Glu Gly Ser
    690             695             700

Glu Pro Thr Ser Gly Thr Glu Gly Pro Glu His Ser Val Asn Gly Pro
705             710             715                 720

Ala Ser Pro Ala Leu Asn Gln Gly Ser
                725
```

<210> 16
<211> 362
<212> PRT
<213> Artificial Sequence

<220>
<223> CD148 trans-membrane and endodomain sequence

<400> 16

```
Ala Val Phe Gly Cys Ile Phe Gly Ala Leu Val Ile Val Thr Val Gly
1               5               10                  15

Gly Phe Ile Phe Trp Arg Lys Lys Arg Lys Asp Ala Lys Asn Asn Glu
            20              25              30

Val Ser Phe Ser Gln Ile Lys Pro Lys Lys Ser Lys Leu Ile Arg Val
            35              40              45

Glu Asn Phe Glu Ala Tyr Phe Lys Lys Gln Gln Ala Asp Ser Asn Cys
        50              55              60

Gly Phe Ala Glu Glu Tyr Glu Asp Leu Lys Leu Val Gly Ile Ser Gln
65              70              75              80

Pro Lys Tyr Ala Ala Glu Leu Ala Glu Asn Arg Gly Lys Asn Arg Tyr
                85              90              95

Asn Asn Val Leu Pro Tyr Asp Ile Ser Arg Val Lys Leu Ser Val Gln
            100             105             110

Thr His Ser Thr Asp Asp Tyr Ile Asn Ala Asn Tyr Met Pro Gly Tyr
        115             120             125

His Ser Lys Lys Asp Phe Ile Ala Thr Gln Gly Pro Leu Pro Asn Thr
        130             135             140

Leu Lys Asp Phe Trp Arg Met Val Trp Glu Lys Asn Val Tyr Ala Ile
145             150             155             160

Ile Met Leu Thr Lys Cys Val Glu Gln Gly Arg Thr Lys Cys Glu Glu
                165             170             175

Tyr Trp Pro Ser Lys Gln Ala Gln Asp Tyr Gly Asp Ile Thr Val Ala
            180             185             190

Met Thr Ser Glu Ile Val Leu Pro Glu Trp Thr Ile Arg Asp Phe Thr
        195             200             205

Val Lys Asn Ile Gln Thr Ser Glu Ser His Pro Leu Arg Gln Phe His
        210             215             220

Phe Thr Ser Trp Pro Asp His Gly Val Pro Asp Thr Thr Asp Leu Leu
225             230             235             240

Ile Asn Phe Arg Tyr Leu Val Arg Asp Tyr Met Lys Gln Ser Pro Pro
            245             250             255
```

```
Glu Ser Pro Ile Leu Val His Cys Ser Ala Gly Val Gly Arg Thr Gly
            260                 265             270

Thr Phe Ile Ala Ile Asp Arg Leu Ile Tyr Gln Ile Glu Asn Glu Asn
            275                 280             285

Thr Val Asp Val Tyr Gly Ile Val Tyr Asp Leu Arg Met His Arg Pro
            290                 295             300

Leu Met Val Gln Thr Glu Asp Gln Tyr Val Phe Leu Asn Gln Cys Val
305                 310                 315                 320

Leu Asp Ile Val Arg Ser Gln Lys Asp Ser Lys Val Asp Leu Ile Tyr
                325                 330                 335

Gln Asn Thr Thr Ala Met Thr Ile Tyr Glu Asn Leu Ala Pro Val Thr
                340                 345                 350

Thr Phe Gly Lys Thr Asn Gly Tyr Ile Ala
                355                 360
```

<210> 17
<211> 136
<212> PRT
<213> Artificial Sequence

<220>
<223> marker/suicide polypeptide

<400> 17

```
Cys Pro Tyr Ser Asn Pro Ser Leu Cys Ser Gly Gly Gly Gly Ser Glu
1                 5                 10                15

Leu Pro Thr Gln Gly Thr Phe Ser Asn Val Ser Thr Asn Val Ser Pro
            20                  25                  30

Ala Lys Pro Thr Thr Thr Ala Cys Pro Tyr Ser Asn Pro Ser Leu Cys
            35                  40                  45

Ser Gly Gly Gly Gly Ser Pro Ala Pro Arg Pro Pro Thr Pro Ala Pro
            50                  55                  60

Thr Ile Ala Ser Gln Pro Leu Ser Leu Arg Pro Glu Ala Cys Arg Pro
65                  70                  75                  80

Ala Ala Gly Gly Ala Val His Thr Arg Gly Leu Asp Phe Ala Cys Asp
                85                  90                  95
```

```
Ile Tyr Ile Trp Ala Pro Leu Ala Gly Thr Cys Gly Val Leu Leu Leu
            100                 105                 110

Ser Leu Val Ile Thr Leu Tyr Cys Asn His Arg Asn Arg Arg Arg Val
            115                 120                 125

Cys Lys Cys Pro Arg Pro Val Val
            130                 135
```

<210> 18
<211> 8
<212> PRT
<213> Artificial Sequence

<220>
<223> cleavage site

<400> 18

```
Asp Met Glu Ser Asn Pro Gly Pro
1                   5
```

<210> 19
<211> 8
<212> PRT
<213> Artificial Sequence

<220>
<223> cleavage site

<400> 19

```
Asp Val Glu Leu Asn Pro Gly Pro
1                   5
```

<210> 20
<211> 8
<212> PRT
<213> Artificial Sequence

<220>
<223> cleavage site

<400> 20

```
Asp Val Glu Glu Asn Pro Gly Pro
1                   5
```

<210> 21
<211> 8
<212> PRT
<213> Artificial Sequence

<220>
<223> cleavage site

<400> 21

```
                    Asp Ile Glu Leu Asn Pro Gly Pro
                    1                   5
```

<210> 22
<211> 8
<212> PRT
<213> Artificial Sequence

<220>
<223> cleavage site

<400> 22

```
                    Asp Ile Glu Gln Asn Pro Gly Pro
                    1                   5
```

<210> 23
<211> 8
<212> PRT
<213> Artificial Sequence

<220>
<223> cleavage site

<400> 23

```
                    Asp Ser Glu Phe Asn Pro Gly Pro
                    1                   5
```

<210> 24
<211> 19
<212> PRT
<213> Artificial Sequence

<220>
<223> cleavage sequence

<400> 24

```
        Leu Leu Asn Phe Asp Leu Leu Lys Leu Ala Gly Asp Val Glu Ser Asn
        1               5                   10                      15

        Pro Gly Pro
```

<210> 25
<211> 19
<212> PRT
<213> Artificial Sequence

<220>
<223> cleavage sequence

<400> 25

Leu Leu Asn Phe Asp Leu Leu Lys Leu Ala Gly Asp Val Gln Ser Asn

1               5                       10                      15

Pro Gly Pro

<210> 26
<211> 19
<212> PRT
<213> Artificial Sequence

<220>
<223> cleavage sequence

<400> 26

Leu Leu Asn Phe Asp Leu Leu Lys Leu Ala Gly Asp Val Glu Ile Asn
1               5                       10                      15

Pro Gly Pro

<210> 27
<211> 19
<212> PRT
<213> Artificial Sequence

<220>
<223> cleavage sequence

<400> 27

Leu Leu Asn Phe Asp Leu Leu Lys Leu Ala Gly Asp Val Glu Phe Asn
1               5                       10                      15

Pro Gly Pro

<210> 28
<211> 19
<212> PRT
<213> Artificial Sequence

<220>
<223> cleavage sequence

<400> 28

Leu Leu Asn Phe Asp Leu Leu Lys Leu Ala Gly Asp Val Glu Ser His
1               5                       10                      15

Pro Gly Pro

<210> 29
<211> 19

<212> PRT
<213> Artificial Sequence

<220>
<223> cleavage sequence

<400> 29

```
Leu Leu Asn Phe Asp Leu Leu Lys Leu Ala Gly Asp Val Glu Ser Glu
1               5                   10                  15

Pro Gly Pro
```

<210> 30
<211> 19
<212> PRT
<213> Artificial Sequence

<220>
<223> cleavage sequence

<400> 30

```
Leu Leu Asn Phe Asp Leu Leu Lys Leu Ala Gly Asp Val Glu Ser Gln
1               5                   10                  15

Pro Gly Pro
```

<210> 31
<211> 19
<212> PRT
<213> Artificial Sequence

<220>
<223> cleavage sequence

<400> 31

```
Leu Leu Asn Phe Asp Leu Leu Lys Leu Ala Gly Asp Val Glu Ser Asn
1               5                   10                  15

Pro Gly Gly
```

<210> 32
<211> 20
<212> PRT
<213> Artificial Sequence

<220>
<223> 2A-like sequence

<400> 32

```
Tyr His Ala Asp Tyr Tyr Lys Gln Arg Leu Ile His Asp Val Glu Met
```

<pre>
                 1                 5                      10                          15
</pre>

<pre>
          Asn Pro Gly Pro
                         20
</pre>

```
<210> 33
<211> 20
<212> PRT
<213> Artificial Sequence

<220>
<223> 2A-like sequence

<400> 33
```

<pre>
          His Tyr Ala Gly Tyr Phe Ala Asp Leu Leu Ile His Asp Ile Glu Thr
          1                 5                      10                  15
</pre>

<pre>
          Asn Pro Gly Pro
                      20
</pre>

```
<210> 34
<211> 20
<212> PRT
<213> Artificial Sequence

<220>
<223> 2A-like sequence

<400> 34
```

<pre>
          Gln Cys Thr Asn Tyr Ala Leu Leu Lys Leu Ala Gly Asp Val Glu Ser
          1                 5                      10                  15
</pre>

<pre>
          Asn Pro Gly Pro
                      20
</pre>

```
<210> 35
<211> 19
<212> PRT
<213> Artificial Sequence

<220>
<223> 2A-like sequence

<400> 35
```

<pre>
          Ala Thr Asn Phe Ser Leu Leu Lys Gln Ala Gly Asp Val Glu Glu Asn
          1                 5                      10                  15
</pre>

<pre>
          Pro Gly Pro
</pre>

```
<210> 36
<211> 19
```

<212> PRT
<213> Artificial Sequence

<220>
<223> 2A-like sequence

<400> 36

```
        Ala Ala Arg Gln Met Leu Leu Leu Leu Ser Gly Asp Val Glu Thr Asn
        1               5                   10                  15


        Pro Gly Pro
```

<210> 37
<211> 20
<212> PRT
<213> Artificial Sequence

<220>
<223> 2A-like sequence

<400> 37

```
        Arg Ala Glu Gly Arg Gly Ser Leu Leu Thr Cys Gly Asp Val Glu Glu
        1               5                   10                  15


        Asn Pro Gly Pro
                        20
```

<210> 38
<211> 20
<212> PRT
<213> Artificial Sequence

<220>
<223> 2A-like sequence

<400> 38

```
        Thr Arg Ala Glu Ile Glu Asp Glu Leu Ile Arg Ala Gly Ile Glu Ser
        1               5                   10                  15


        Asn Pro Gly Pro
                        20
```

<210> 39
<211> 20
<212> PRT
<213> Artificial Sequence

<220>
<223> 2A-like sequence

<400> 39

Thr Arg Ala Glu Ile Glu Asp Glu Leu Ile Arg Ala Asp Ile Glu Ser

1            5                 10                15

Asn Pro Gly Pro
20

<210> 40
<211> 20
<212> PRT
<213> Artificial Sequence

<220>
<223> 2A-like sequence

<400> 40

Ala Lys Phe Gln Ile Asp Lys Ile Leu Ile Ser Gly Asp Val Glu Leu
1            5                 10                15

Asn Pro Gly Pro
20

<210> 41
<211> 20
<212> PRT
<213> Artificial Sequence

<220>
<223> 2A-like sequence

<400> 41

Ser Ser Ile Ile Arg Thr Lys Met Leu Val Ser Gly Asp Val Glu Glu
1            5                 10                15

Asn Pro Gly Pro
20

<210> 42
<211> 20
<212> PRT
<213> Artificial Sequence

<220>
<223> 2A-like sequence

<400> 42

```
Cys Asp Ala Gln Arg Gln Lys Leu Leu Leu Ser Gly Asp Ile Glu Gln
1               5                   10                  15

Asn Pro Gly Pro
            20
```

<210> 43
<211> 20
<212> PRT
<213> Artificial Sequence

<220>
<223> 2A-like sequence

<400> 43

```
Tyr Pro Ile Asp Phe Gly Gly Phe Leu Val Lys Ala Asp Ser Glu Phe
1               5                   10                  15

Asn Pro Gly Pro
            20
```

<210> 44
<211> 57
<212> PRT
<213> Artificial Sequence

<220>
<223> cleavage sequence

<400> 44

```
Val Thr Glu Leu Leu Tyr Arg Met Lys Arg Ala Glu Thr Tyr Cys Pro
1               5                   10                  15

Arg Pro Leu Ala Ile His Pro Thr Glu Ala Arg His Lys Gln Lys Ile
            20                  25                  30

Val Ala Pro Val Lys Gln Thr Leu Asn Phe Asp Leu Leu Lys Leu Ala
            35                  40                  45

Gly Asp Val Glu Ser Asn Pro Gly Pro
        50                  55
```

<210> 45
<211> 40
<212> PRT
<213> Artificial Sequence

<220>
<223> cleavage sequence

<400> 45

```
        Leu Leu Ala Ile His Pro Thr Glu Ala Arg His Lys Gln Lys Ile Val
        1               5               10              15
```

```
        Ala Pro Val Lys Gln Thr Leu Asn Phe Asp Leu Leu Lys Leu Ala Gly
                        20              25              30
```

```
        Asp Val Glu Ser Asn Pro Gly Pro
                    35              40
```

<210> 46
<211> 33
<212> PRT
<213> Artificial Sequence

<220>
<223> cleavage sequence

<400> 46

```
        Glu Ala Arg His Lys Gln Lys Ile Val Ala Pro Val Lys Gln Thr Leu
        1               5               10              15
```

```
        Asn Phe Asp Leu Leu Lys Leu Ala Gly Asp Val Glu Ser Asn Pro Gly
                        20              25              30
```

```
        Pro
```

<210> 47
<211> 24
<212> PRT
<213> Artificial Sequence

<220>
<223> cleavage sequence

<400> 47

```
        Ala Pro Val Lys Gln Thr Leu Asn Phe Asp Leu Leu Lys Leu Ala Gly
        1               5               10              15
```

```
        Asp Val Glu Ser Asn Pro Gly Pro
                        20
```

<210> 48
<211> 5
<212> PRT
<213> Artificial Sequence

<220>
<223> linker

<400> 48

```
                            Ser Gly Gly Gly Ser
                            1                   5
```

<210> 49
<211> 4
<212> PRT
<213> Artificial Sequence

<220>
<223> intracellular retention signal

<220>
<221> misc_feature
<222> (4)..(4)
<223> Xaa can be any naturally occurring amino acid

<400> 49

```
                                     Asn Pro Phe Xaa
                                     1
```

<210> 50
<211> 4
<212> PRT
<213> Artificial Sequence

<220>
<223> intracellular retention signal

<400> 50

```
                            Lys Asp Glu Leu
                            1
```

<210> 51
<211> 4
<212> PRT
<213> Artificial Sequence

<220>
<223> intracellular retention signal

<220>
<221> misc_feature
<222> (3)..(4)
<223> Xaa can be any naturally occurring amino acid

<400> 51

```
                            Lys Lys Xaa Xaa
                            1
```

<210> 52
<211> 5
<212> PRT
<213> Artificial Sequence

<220>

<223> intracellular retention signal

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<400> 52

```
Lys Xaa Lys Xaa Xaa
1               5
```

<210> 53
<211> 4
<212> PRT
<213> Artificial Sequence

<220>
<223> intracellular retention signal

<220>
<221> misc_feature
<222> (3)..(3)
<223> Xaa can be any naturally occurring amino acid

<400> 53

```
Asn Pro Xaa Tyr
1
```

<210> 54
<211> 4
<212> PRT
<213> Artificial Sequence

<220>
<223> intracellular retention signal

<220>
<221> misc_feature
<222> (2)..(3)
<223> Xaa can be any naturally occurring amino acid

<220>
<221> MISC_FEATURE
<222> (4)..(4)
<223> Xaa is an amino acid with a bulky hydrophobic side chain

<400> 54

```
Tyr Xaa Xaa Xaa
1
```

<210> 55
<211> 8
<212> PRT
<213> Artificial Sequence

<220>
<223> intracellular retention signal

<220>
<221> misc_feature
<222> (3)..(5)
<223> Xaa can be any naturally occurring amino acid

<400> 55

```
Asp Glu Xaa Xaa Xaa Leu Leu Ile
1               5
```

<210> 56
<211> 5
<212> PRT
<213> Artificial Sequence

<220>
<223> intracellular retention signal

<220>
<221> misc_feature
<222> (2)..(3)
<223> Xaa can be any naturally occurring amino acid

<400> 56

```
Asp Xaa Xaa Leu Leu
1               5
```

<210> 57
<211> 4
<212> PRT
<213> Artificial Sequence

<220>
<223> intracellular retention signal

<400> 57

```
Asp Pro Phe Trp
1
```

<210> 58
<211> 5
<212> PRT
<213> Artificial Sequence

<220>
<223> intracellular retention signal

<220>

<221> misc_feature
<222> (2)..(2)
<223> Xaa can be any naturally occurring amino acid

<220>
<221> misc_feature
<222> (4)..(4)
<223> Xaa can be any naturally occurring amino acid

<400> 58

```
Phe Xaa Asp Xaa Phe
1               5
```

<210> 59
<211> 6
<212> PRT
<213> Artificial Sequence

<220>
<223> intracellular retention signal

<220>
<221> MISC_FEATURE
<222> (2)..(2)
<223> Xaa is an amino acid with a bulky hydrophobic side chain

<220>
<221> misc_feature
<222> (3)..(3)
<223> Xaa can be any naturally occurring amino acid

<220>
<221> MISC_FEATURE
<222> (4)..(4)
<223> Xaa is an amino acid with a bulky hydrophobic side chain

<400> 59

```
Leu Xaa Xaa Xaa Asp Glu
1               5
```

<210> 60
<211> 5
<212> PRT
<213> Artificial Sequence

<220>
<223> intracellular retention signal

<400> 60

```
Leu Leu Asp Leu Leu
1               5
```

<210> 61
<211> 5
<212> PRT

<213> Artificial Sequence

<220>
<223> intracellular retention signal

<400> 61

```
                                        Pro Trp Asp Leu Trp
                                        1                 5
```

<210> 62
<211> 20
<212> PRT
<213> Mus musculus

<400> 62

```
        Met Glu Thr Asp Thr Leu Ile Leu Trp Val Leu Leu Leu Leu Val Pro
        1               5                 10                  15


        Gly Ser Thr Gly
                    20
```

<210> 63
<211> 21
<212> PRT
<213> Artificial Sequence

<220>
<223> signal peptide derived from Human CD8a

<400> 63

```
        Met Ser Leu Pro Val Thr Ala Leu Leu Leu Pro Leu Ala Leu Leu Leu
        1               5                 10                  15


        His Ala Ala Arg Pro
                    20
```

<210> 64
<211> 248
<212> PRT
<213> Artificial Sequence

<220>
<223> scFv aCD19

<400> 64

```
Asp Ile Gln Met Thr Gln Thr Thr Ser Ser Leu Ser Ala Ser Leu Gly
1               5               10              15

Asp Arg Val Thr Ile Ser Cys Arg Ala Ser Gln Asp Ile Ser Lys Tyr
            20              25              30

Leu Asn Trp Tyr Gln Gln Lys Pro Asp Gly Thr Val Lys Leu Leu Ile
            35              40              45

Tyr His Thr Ser Arg Leu His Ser Gly Val Pro Ser Arg Phe Ser Gly
    50              55              60

Ser Gly Ser Gly Thr Asp Tyr Ser Leu Thr Ile Ser Asn Leu Glu Gln
65              70              75              80

Glu Asp Ile Ala Thr Tyr Phe Cys Gln Gln Gly Asn Thr Leu Pro Tyr
            85              90              95

Thr Phe Gly Gly Gly Thr Lys Leu Glu Ile Thr Lys Ala Gly Gly Gly
        100             105             110

Gly Ser Gly Gly Gly Gly Ser Gly Gly Gly Ser Gly Gly Gly Gly
        115             120             125

Ser Glu Val Lys Leu Gln Glu Ser Gly Pro Gly Leu Val Ala Pro Ser
    130             135             140

Gln Ser Leu Ser Val Thr Cys Thr Val Ser Gly Val Ser Leu Pro Asp
145             150             155             160

Tyr Gly Val Ser Trp Ile Arg Gln Pro Pro Arg Lys Gly Leu Glu Trp
            165             170             175

Leu Gly Val Ile Trp Gly Ser Glu Thr Thr Tyr Tyr Asn Ser Ala Leu
            180             185             190

Lys Ser Arg Leu Thr Ile Ile Lys Asp Asn Ser Lys Ser Gln Val Phe
        195             200             205

Leu Lys Met Asn Ser Leu Gln Thr Asp Asp Thr Ala Ile Tyr Tyr Cys
    210             215             220

Ala Lys His Tyr Tyr Tyr Gly Gly Ser Tyr Ala Met Asp Tyr Trp Gly
225             230             235             240

Gln Gly Thr Ser Val Thr Val Ser
            245
```

<210> 65
<211> 47
<212> PRT
<213> Homo sapiens

<400> 65

```
Pro Thr Thr Thr Pro Ala Pro Arg Pro Pro Thr Pro Ala Pro Thr Ile
1               5               10              15

Ala Ser Gln Pro Leu Ser Leu Arg Pro Glu Ala Cys Arg Pro Ala Ala
            20              25              30

Gly Gly Ala Val His Thr Arg Gly Leu Asp Phe Ala Cys Asp Ile
            35              40              45
```

<210> 66
<211> 27
<212> PRT
<213> Homo sapiens

<400> 66

```
Phe Trp Val Leu Val Val Val Gly Gly Val Leu Ala Cys Tyr Ser Leu
1               5               10              15

Leu Val Thr Val Ala Phe Ile Ile Phe Trp Val
            20              25
```

<210> 67
<211> 113
<212> PRT
<213> Homo sapiens

<400> 67

```
Arg Arg Val Lys Phe Ser Arg Ser Ala Asp Ala Pro Ala Tyr Gln Gln
1               5                   10                  15

Gly Gln Asn Gln Leu Tyr Asn Glu Leu Asn Leu Gly Arg Arg Glu Glu
            20                  25                  30

Tyr Asp Val Leu Asp Lys Arg Arg Gly Arg Asp Pro Glu Met Gly Gly
                35                  40                  45

Lys Pro Arg Arg Lys Asn Pro Gln Glu Gly Leu Tyr Asn Glu Leu Gln
        50                  55                  60

Lys Asp Lys Met Ala Glu Ala Tyr Ser Glu Ile Gly Met Lys Gly Glu
65                  70                  75                  80

Arg Arg Arg Gly Lys Gly His Asp Gly Leu Tyr Gln Gly Leu Ser Thr
                85                  90                  95

Ala Thr Lys Asp Thr Tyr Asp Ala Leu His Met Gln Ala Leu Pro Pro
                100                 105                 110

Arg
```

<210> 68
<211> 20
<212> PRT
<213> Artificial Sequence

<220>
<223> 2A peptide

<400> 68

```
Arg Ala Glu Gly Arg Gly Ser Leu Leu Thr Cys Gly Asp Val Glu Glu
1               5                   10                  15

Asn Pro Gly Pro
            20
```

<210> 69
<211> 18
<212> PRT
<213> Artificial Sequence

<220>
<223> signal peptide derived from mouse Ig kappa

<400> 69

```
Met Ala Val Pro Thr Gln Val Leu Gly Leu Leu Leu Leu Trp Leu Thr
1               5               10              15
```

Asp Ala

<210> 70
<211> 254
<212> PRT
<213> Artificial Sequence

<220>
<223> scFv aCD33

<400> 70

```
Arg Cys Asp Ile Gln Met Thr Gln Ser Pro Ser Ser Leu Ser Ala Ser
1               5               10              15

Val Gly Asp Arg Val Thr Ile Thr Cys Arg Ala Ser Glu Asp Ile Tyr
            20              25              30

Phe Asn Leu Val Trp Tyr Gln Gln Lys Pro Gly Lys Ala Pro Lys Leu
            35              40              45

Leu Ile Tyr Asp Thr Asn Arg Leu Ala Asp Gly Val Pro Ser Arg Phe
            50              55              60

Ser Gly Ser Gly Ser Gly Thr Gln Tyr Thr Leu Thr Ile Ser Ser Leu
65              70              75              80

Gln Pro Glu Asp Phe Ala Thr Tyr Tyr Cys Gln His Tyr Lys Asn Tyr
                85              90              95

Pro Leu Thr Phe Gly Gln Gly Thr Lys Leu Glu Ile Lys Arg Ser Gly
```

```
                100                    105                      110

        Gly Gly Gly Ser Gly Gly Gly Gly Ser Gly Gly Gly Gly Ser Gly Gly
                115               120              125

        Gly Gly Ser Arg Ser Glu Val Gln Leu Val Glu Ser Gly Gly Gly Leu
                130               135              140

        Val Gln Pro Gly Gly Ser Leu Arg Leu Ser Cys Ala Ala Ser Gly Phe
        145               150              155              160

        Thr Leu Ser Asn Tyr Gly Met His Trp Ile Arg Gln Ala Pro Gly Lys
                        165              170              175

        Gly Leu Glu Trp Val Ser Ser Ile Ser Leu Asn Gly Gly Ser Thr Tyr
                    180              185              190

        Tyr Arg Asp Ser Val Lys Gly Arg Phe Thr Ile Ser Arg Asp Asn Ala
                195              200              205

        Lys Ser Thr Leu Tyr Leu Gln Met Asn Ser Leu Arg Ala Glu Asp Thr
                210              215              220

        Ala Val Tyr Tyr Cys Ala Ala Gln Asp Ala Tyr Thr Gly Gly Tyr Phe
        225               230              235              240

        Asp Tyr Trp Gly Gln Gly Thr Leu Val Thr Val Ser Ser Met
                        245              250
```

<210> 71
<211> 231
<212> PRT
<213> Artificial Sequence

<220>
<223> hinge and Fc derived from human IgG1

<400> 71

```
Glu Pro Lys Ser Pro Asp Lys Thr His Thr Cys Pro Pro Cys Pro Ala
1               5               10              15

Pro Pro Val Ala Gly Pro Ser Val Phe Leu Phe Pro Pro Lys Pro Lys
        20              25              30

Asp Thr Leu Met Ile Ala Arg Thr Pro Glu Val Thr Cys Val Val Val
        35              40              45

Asp Val Ser His Glu Asp Pro Glu Val Lys Phe Asn Trp Tyr Val Asp
        50              55              60

Gly Val Glu Val His Asn Ala Lys Thr Lys Pro Arg Glu Glu Gln Tyr
65              70              75              80

Asn Ser Thr Tyr Arg Val Val Ser Val Leu Thr Val Leu His Gln Asp
                85              90              95

Trp Leu Asn Gly Lys Glu Tyr Lys Cys Lys Val Ser Asn Lys Ala Leu
        100             105             110

Pro Ala Pro Ile Glu Lys Thr Ile Ser Lys Ala Lys Gly Gln Pro Arg
        115             120             125

Glu Pro Gln Val Tyr Thr Leu Pro Pro Ser Arg Asp Glu Leu Thr Lys
    130             135             140

Asn Gln Val Ser Leu Thr Cys Leu Val Lys Gly Phe Tyr Pro Ser Asp
145             150             155             160

Ile Ala Val Glu Trp Glu Ser Asn Gly Gln Pro Glu Asn Asn Tyr Lys
            165             170             175

Thr Thr Pro Pro Val Leu Asp Ser Asp Gly Ser Phe Phe Leu Tyr Ser
        180             185             190

Lys Leu Thr Val Asp Lys Ser Arg Trp Gln Gln Gly Asn Val Phe Ser
        195             200             205

Cys Ser Val Met His Glu Ala Leu His Asn His Tyr Thr Gln Lys Ser
    210             215             220

Leu Ser Leu Ser Pro Gly Lys
225             230
```

<210> 72
<211> 4

<212> PRT
<213> Artificial Sequence

<220>
<223> linker

<400> 72

```
                              Lys Asp Pro Lys
                              1
```

<210> 73
<211> 21
<212> PRT
<213> Homo sapiens

<400> 73

```
        Ala Val Phe Gly Cys Ile Phe Gly Ala Leu Val Ile Val Thr Val Gly
        1               5                   10                  15

        Gly Phe Ile Phe Trp
                        20
```

<210> 74
<211> 341
<212> PRT
<213> Homo sapiens

<400> 74

Arg Lys Lys Arg Lys Asp Ala Lys Asn Asn Glu Val Ser Phe Ser Gln
1               5                   10                  15

Ile Lys Pro Lys Lys Ser Lys Leu Ile Arg Val Glu Asn Phe Glu Ala
            20                  25                  30

Tyr Phe Lys Lys Gln Gln Ala Asp Ser Asn Cys Gly Phe Ala Glu Glu
        35                  40                  45

Tyr Glu Asp Leu Lys Leu Val Gly Ile Ser Gln Pro Lys Tyr Ala Ala
    50                  55                  60

Glu Leu Ala Glu Asn Arg Gly Lys Asn Arg Tyr Asn Asn Val Leu Pro
65                  70                  75                  80

Tyr Asp Ile Ser Arg Val Lys Leu Ser Val Gln Thr His Ser Thr Asp
            85                  90                  95

Asp Tyr Ile Asn Ala Asn Tyr Met Pro Gly Tyr His Ser Lys Lys Asp
            100                 105                 110

Phe Ile Ala Thr Gln Gly Pro Leu Pro Asn Thr Leu Lys Asp Phe Trp
        115                 120                 125

Arg Met Val Trp Glu Lys Asn Val Tyr Ala Ile Ile Met Leu Thr Lys
    130                 135                 140

Cys Val Glu Gln Gly Arg Thr Lys Cys Glu Glu Tyr Trp Pro Ser Lys
145                 150                 155                 160

Gln Ala Gln Asp Tyr Gly Asp Ile Thr Val Ala Met Thr Ser Glu Ile
            165                 170                 175

```
Val Leu Pro Glu Trp Thr Ile Arg Asp Phe Thr Val Lys Asn Ile Gln
            180             185             190

Thr Ser Glu Ser His Pro Leu Arg Gln Phe His Phe Thr Ser Trp Pro
            195             200             205

Asp His Gly Val Pro Asp Thr Thr Asp Leu Leu Ile Asn Phe Arg Tyr
    210             215             220

Leu Val Arg Asp Tyr Met Lys Gln Ser Pro Pro Glu Ser Pro Ile Leu
225             230             235             240

Val His Cys Ser Ala Gly Val Gly Arg Thr Gly Thr Phe Ile Ala Ile
            245             250             255

Asp Arg Leu Ile Tyr Gln Ile Glu Asn Glu Asn Thr Val Asp Val Tyr
        260             265             270

Gly Ile Val Tyr Asp Leu Arg Met His Arg Pro Leu Met Val Gln Thr
        275             280             285

Glu Asp Gln Tyr Val Phe Leu Asn Gln Cys Val Leu Asp Ile Val Arg
    290             295             300

Ser Gln Lys Asp Ser Lys Val Asp Leu Ile Tyr Gln Asn Thr Thr Ala
305             310             315             320

Met Thr Ile Tyr Glu Asn Leu Ala Pro Val Thr Thr Phe Gly Lys Thr
            325             330             335

Asn Gly Tyr Ile Ala
            340
```

<210> 75
<211> 22
<212> PRT
<213> Homo sapiens

<400> 75

```
Ala Leu Ile Ala Phe Leu Ala Phe Leu Ile Ile Val Thr Ser Ile Ala
1               5               10              15

Leu Leu Val Val Leu Tyr
            20
```

<210> 76
<211> 4
<212> PRT

<213> Mus musculus

<400> 76

Met Glu Thr Asp
1

<210> 77
<211> 11
<212> PRT
<213> Mus musculus

<400> 77

Thr Leu Ile Leu Trp Val Leu Leu Leu Val
1               5                   10

<210> 78
<211> 5
<212> PRT
<213> Mus musculus

<400> 78

Pro Gly Ser Thr Gly
1                   5

<210> 79
<211> 25
<212> PRT
<213> Homo sapiens

<400> 79

Met Asn Arg Gly Val Pro Phe Arg His Leu Leu Leu Val Leu Gln Leu
1               5               10                  15

Ala Leu Leu Pro Ala Ala Thr Gln Gly
            20              25

<210> 80
<211> 23
<212> PRT
<213> Homo sapiens

<400> 80

Met Tyr Leu Trp Leu Lys Leu Leu Ala Phe Gly Phe Ala Phe Leu Asp
1               5               10                  15

Thr Glu Val Phe Val Thr Gly
            20

<210> 81
<211> 21
<212> PRT

<213> Homo sapiens

<400> 81

```
Met Ala Leu Pro Val Thr Ala Leu Leu Leu Pro Leu Ala Leu Leu Leu
1               5               10              15

His Ala Ala Arg Pro
            20
```

<210> 82
<211> 21
<212> PRT
<213> Homo sapiens

<400> 82

```
Met Arg Pro Arg Leu Trp Leu Leu Leu Ala Ala Gln Leu Thr Val Leu
1               5               10              15

His Gly Asn Ser Val
            20
```

<210> 83
<211> 24
<212> PRT
<213> Homo sapiens

<400> 83

```
Met Ser Phe Pro Cys Lys Phe Val Ala Ser Phe Leu Leu Ile Phe Asn
1               5               10              15

Val Ser Ser Lys Gly Ala Val Ser
            20
```

<210> 84
<211> 24
<212> PRT
<213> Homo sapiens

<400> 84

```
Met Pro Met Gly Ser Leu Gln Pro Leu Ala Thr Leu Tyr Leu Leu Gly
1               5               10              15

Met Leu Val Ala Ser Cys Leu Gly
            20
```

<210> 85
<211> 25
<212> PRT
<213> Homo sapiens

<400> 85

```
Met Ala Gly Pro Pro Arg Leu Leu Leu Leu Pro Leu Leu Leu Ala Leu
1               5               10              15

Ala Arg Gly Leu Pro Gly Ala Leu Ala
        20              25
```

<210> 86
<211> 24
<212> PRT
<213> Homo sapiens

<400> 86

```
Met Ser Ala Pro Lys Leu Leu Ser Leu Gly Cys Ile Phe Phe Pro Leu
1               5               10              15

Leu Leu Phe Gln Gln Ala Arg Ala
        20
```

<210> 87
<211> 19
<212> PRT
<213> Homo sapiens

<400> 87

```
Met Arg Phe Phe Val Pro Leu Phe Leu Val Gly Ile Leu Phe Pro Ala
1               5               10              15

Ile Leu Ala
```

<210> 88
<211> 23
<212> PRT
<213> Homo sapiens

<400> 88

```
Met Pro Gly Phe Leu Val Arg Ile Leu Pro Leu Leu Leu Val Leu Leu
1               5               10              15

Leu Leu Gly Pro Thr Arg Gly
        20
```

<210> 89
<211> 24
<212> PRT
<213> Homo sapiens

<400> 89

```
Met Lys Arg Phe Leu Phe Leu Leu Leu Thr Ile Ser Leu Leu Val Met
1               5               10              15

Val Gln Ile Gln Thr Gly Leu Ser
            20
```

<210> 90
<211> 21
<212> PRT
<213> Homo sapiens

<400> 90

```
Met Gln Gly Pro Pro Leu Leu Thr Ala Ala His Leu Leu Cys Val Cys
1               5               10              15

Thr Ala Ala Leu Ala
            20
```

<210> 91
<211> 22
<212> PRT
<213> Homo sapiens

<400> 91

```
Met Gly Pro Thr Ser Gly Pro Ser Leu Leu Leu Leu Leu Leu Thr His
1               5               10              15

Leu Pro Leu Ala Leu Gly
            20
```

<210> 92
<211> 18
<212> PRT
<213> Homo sapiens

<400> 92

```
Met Gln Pro Ile Leu Leu Leu Leu Ala Phe Leu Leu Leu Pro Arg Ala
1               5               10              15

Asp Ala
```

<210> 93
<211> 20
<212> PRT
<213> Homo sapiens

<400> 93

Met Glu Ala Pro Ala Gln Leu Leu Phe Leu Leu Leu Leu Trp Leu Pro
1               5               10              15

Asp Thr Thr Arg
              20

<210> 94
<211> 20
<212> PRT
<213> Homo sapiens

<400> 94

Met Val Leu Gln Thr Gln Val Phe Ile Ser Leu Leu Leu Trp Ile Ser
1               5               10              15

Gly Ala Tyr Gly
              20

<210> 95
<211> 19
<212> PRT
<213> Homo sapiens

<400> 95

Met Ala Trp Ala Pro Leu Leu Leu Thr Leu Leu Ala His Cys Thr Asp
1               5               10              15

Cys Trp Ala

<210> 96
<211> 22
<212> PRT
<213> Homo sapiens

<400> 96

Met Leu Lys Pro Ser Leu Pro Phe Thr Ser Leu Leu Phe Leu Gln Leu
1               5               10              15

Pro Leu Leu Gly Val Gly
              20

<210> 97
<211> 20
<212> PRT
<213> Homo sapiens

<400> 97

```
        Met Ala Trp Ala Ser Arg Leu Gly Leu Leu Leu Ala Leu Leu Leu Pro
        1               5               10              15


        Val Val Gly Ala
                    20
```

<210> 98
<211> 23
<212> PRT
<213> Homo sapiens

<400> 98

```
        Met Gly Val Pro Arg Pro Gln Pro Trp Ala Leu Gly Leu Leu Leu Phe
        1               5               10              15


        Leu Leu Pro Gly Ser Leu Gly
                    20
```

<210> 99
<211> 4
<212> PRT
<213> Artificial Sequence

<220>
<223> ITAM motif

<220>
<221> misc_feature
<222> (2)..(3)
<223> Xaa can be any naturally occurring amino acid

<220>
<221> MISC_FEATURE
<222> (4)..(4)
<223> Xaa may be Leu or Ile

<400> 99

```
                        Tyr Xaa Xaa Xaa
                        1
```

<210> 100
<211> 4
<212> PRT
<213> Artificial Sequence

<220>
<223> basic amino acid furin target sequence

<220>
<221> misc_feature
<222> (2)..(2)
<223> Xaa can be any naturally occurring amino acid

<220>

<221> MISC_FEATURE
<222> (3)..(3)
<223> Xaa may be Arg or Lys

<400> 100

```
                                        Arg Xaa Xaa Arg
                                        1
```

<210> 101
<211> 7
<212> PRT
<213> Artificial Sequence

<220>
<223> consensus Tobacco Etch Virus (TEV) cleavage site

<400> 101

```
                              Glu Asn Leu Tyr Phe Gln Ser
                              1               5
```

<210> 102
<211> 8
<212> PRT
<213> Artificial Sequence

<220>
<223> motif involved in cleavage activity

<220>
<221> misc_feature
<222> (2)..(2)
<223> Xaa can be any naturally occurring amino acid

<220>
<221> misc_feature
<222> (4)..(4)
<223> Xaa can be any naturally occurring amino acid

<400> 102

```
                         Asp Xaa Glu Xaa Asn Pro Gly Pro
                         1               5
```

<210> 103
<211> 8
<212> PRT
<213> Artificial Sequence

<220>
<223> cleavage site

<220>
<221> MISC_FEATURE
<222> (2)..(2)
<223> Xaa may be Ile, Val, Met or Ser

<220>
<221> MISC_FEATURE
<222> (4)..(4)
<223> Xaa may be Thr, Met, Ser, Leu, Glu, Gln or Phe

<400> 103

```
                        Asp Xaa Glu Xaa Asn Pro Gly Pro
                        1               5
```

<210> 104
<211> 4
<212> PRT
<213> Artificial Sequence

<220>
<223> C-terminal signal, motif of postassium channels

<220>
<221> misc_feature
<222> (2)..(2)
<223> Xaa can be any naturally occurring amino acid

<400> 104

```
                            Arg Xaa Arg Arg
                            1
```

<210> 105
<211> 12
<212> PRT
<213> Homo sapiens

<400> 105

```
                    Ile Asn Phe Asp Asn Pro Val Tyr Gln Lys Thr Thr
                    1               5                   10
```

<210> 106
<211> 12
<212> PRT
<213> Homo sapiens

<400> 106

```
                    Val Glu Ile Gly Asn Pro Thr Tyr Lys Met Tyr Glu
                    1               5                   10
```

<210> 107
<211> 12
<212> PRT
<213> Homo sapiens

<400> 107

```
                    Thr Asn Phe Thr Asn Pro Val Tyr Ala Thr Leu Tyr
                    1               5                   10
```

<210> 108
<211> 12
<212> PRT
<213> Drosophila sp.

<400> 108

```
Gly Asn Phe Ala Asn Pro Val Tyr Glu Ser Met Tyr
 1               5                   10
```

<210> 109
<211> 12
<212> PRT
<213> Caenorhabditis elegans

<400> 109

```
Thr Thr Phe Thr Asn Pro Val Tyr Glu Leu Glu Asp
 1               5                   10
```

<210> 110
<211> 12
<212> PRT
<213> Caenorhabditis elegans

<400> 110

```
Leu Arg Val Asp Asn Pro Leu Tyr Asp Pro Asp Ser
 1               5                   10
```

<210> 111
<211> 12
<212> PRT
<213> Homo sapiens

<400> 111

```
Ile Ile Phe Glu Asn Pro Met Tyr Ser Ala Arg Asp
 1               5                   10
```

<210> 112
<211> 12
<212> PRT
<213> Homo sapiens

<400> 112

```
Thr Asn Phe Glu Asn Pro Ile Tyr Ala Gln Met Glu
 1               5                   10
```

<210> 113
<211> 12
<212> PRT
<213> Homo sapiens

<400> 113

```
Asp Thr Gly Glu Asn Pro Ile Tyr Lys Ser Ala Val
1               5                   10
```

<210> 114
<211> 11
<212> PRT
<213> Homo sapiens

<400> 114

```
Thr Thr Val Val Asn Pro Lys Tyr Glu Gly Lys
1               5                   10
```

<210> 115
<211> 12
<212> PRT
<213> Drosophila sp.

<400> 115

```
Trp Asp Thr Glu Asn Pro Ile Tyr Lys Gln Ala Thr
1               5                   10
```

<210> 116
<211> 11
<212> PRT
<213> Drosophila sp.

<400> 116

```
Ser Thr Phe Lys Asn Pro Met Tyr Ala Gly Lys
1               5                   10
```

<210> 117
<211> 12
<212> PRT
<213> Homo sapiens

<400> 117

```
His Gly Tyr Glu Asn Pro Thr Tyr Arg Phe Leu Glu
1               5                   10
```

<210> 118
<211> 12
<212> PRT
<213> Homo sapiens

<400> 118

```
Asn Gly Tyr Glu Asn Pro Thr Tyr Lys Phe Phe Glu
1               5                   10
```

<210> 119
<211> 12
<212> PRT

<213> Drosophila sp.

<400> 119

```
                    Asn Gly Tyr Glu Asn Pro Thr Tyr Lys Tyr Phe Glu
                    1               5                   10
```

<210> 120
<211> 12
<212> PRT
<213> Homo sapiens

<400> 120

```
                    Tyr Ala Ser Ser Asn Pro Glu Tyr Leu Ser Ala Ser
                    1               5                   10
```

<210> 121
<211> 12
<212> PRT
<213> Homo sapiens

<400> 121

```
                    Gly Ser Val Gln Asn Pro Val Tyr His Asn Gln Pro
                    1               5                   10
```

<210> 122
<211> 12
<212> PRT
<213> Homo sapiens

<400> 122

```
                    Thr Ala Val Gly Asn Pro Glu Tyr Leu Asn Thr Val
                    1               5                   10
```

<210> 123
<211> 12
<212> PRT
<213> Homo sapiens

<400> 123

```
                    Ile Ser Leu Asp Asn Pro Asp Tyr Gln Gln Asp Phe
                    1               5                   10
```

<210> 124
<211> 11
<212> PRT
<213> Homo sapiens

<400> 124

```
                    Arg Lys Arg Ser His Ala Gly Tyr Gln Thr Ile
                    1               5                   10
```

<210> 125

<211> 10
<212> PRT
<213> Homo sapiens

<400> 125

```
Lys His His His Ala Gly Tyr Glu Gln Phe
1               5               10
```

<210> 126
<211> 11
<212> PRT
<213> Gallus gallus

<400> 126

```
Lys Lys His His Asn Thr Gly Tyr Glu Gln Phe
1               5               10
```

<210> 127
<211> 11
<212> PRT
<213> Gallus gallus

<400> 127

```
Arg Arg Lys Ser Arg Thr Gly Tyr Gln Ser Val
1               5               10
```

<210> 128
<211> 11
<212> PRT
<213> Gallus gallus

<400> 128

```
Arg Arg Lys Ser Tyr Ala Gly Tyr Gln Thr Leu
1               5               10
```

<210> 129
<211> 12
<212> PRT
<213> Drosophila sp.

<400> 129

```
Arg Arg Arg Ser Thr Ser Arg Gly Tyr Met Ser Phe
1               5               10
```

<210> 130
<211> 11
<212> PRT
<213> Lumbricus terrestris

<400> 130

```
Arg Lys Arg Ser Arg Arg Gly Tyr Glu Ser Val
1               5               10
```

<210> 131
<211> 10
<212> PRT
<213> Homo sapiens

<400> 131

```
Lys Ser Ile Arg Ser Gly Tyr Glu Val Met
1               5                   10
```

<210> 132
<211> 12
<212> PRT
<213> Homo sapiens

<400> 132

```
His Cys Gly Gly Pro Arg Pro Gly Tyr Glu Thr Leu
1               5                   10
```

<210> 133
<211> 12
<212> PRT
<213> Mus musculus

<400> 133

```
His Cys Arg Thr Arg Arg Ala Glu Tyr Glu Thr Leu
1               5                   10
```

<210> 134
<211> 10
<212> PRT
<213> Homo sapiens

<400> 134

```
Arg Arg Arg Pro Ser Ala Tyr Gln Ala Leu
1               5                   10
```

<210> 135
<211> 9
<212> PRT
<213> Homo sapiens

<400> 135

```
Arg Arg Arg Ser Tyr Gln Asn Ile Pro
1               5
```

<210> 136
<211> 10
<212> PRT
<213> Homo sapiens

<400> 136

```
                    Lys Lys His Cys Ser Tyr Gln Asp Ile Leu
                    1               5               10
```

<210> 137
<211> 10
<212> PRT
<213> Mus musculus

<400> 137

```
                    Arg Arg Arg Ser Ala Tyr Gln Asp Ile Arg
                    1               5               10
```

<210> 138
<211> 11
<212> PRT
<213> Rattus norvegicus

<400> 138

```
                    Arg Lys Arg Arg Arg Ser Tyr Gln Asp Ile Met
                    1               5               10
```

<210> 139
<211> 13
<212> PRT
<213> Rattus norvegicus

<400> 139

```
                    Lys Phe Cys Lys Ser Lys Glu Arg Asn Tyr His Thr Leu
                    1               5               10
```

<210> 140
<211> 14
<212> PRT
<213> Drosophila sp.

<400> 140

```
                    Lys Phe Tyr Lys Ala Arg Asn Glu Arg Asn Tyr His Thr Leu
                    1               5               10
```

<210> 141
<211> 14
<212> PRT
<213> Homo sapiens

<400> 141

```
                    Lys Ile Arg Leu Arg Cys Gln Ser Ser Gly Tyr Gln Arg Ile
                    1               5               10
```

<210> 142
<211> 14
<212> PRT
<213> Mus musculus

<400> 142

        Lys Ile Arg Gln Arg His Gln Ser Ser Ala Tyr Gln Arg Ile
        1                5                10

<210> 143
<211> 15
<212> PRT
<213> Homo sapiens

<400> 143

        His Phe Cys Leu Tyr Arg Lys Arg Pro Gly Tyr Asp Gln Leu Asn
        1                5                10              15

<210> 144
<211> 11
<212> PRT
<213> Homo sapiens

<400> 144

        Ser Leu Ser Arg Gly Ser Gly Tyr Lys Glu Ile
        1                5                10

<210> 145
<211> 13
<212> PRT
<213> Homo sapiens

<400> 145

        Arg Arg Leu Arg Lys Gly Tyr Thr Pro Leu Met Glu Thr
        1                5                10

<210> 146
<211> 14
<212> PRT
<213> Homo sapiens

<400> 146

        Arg Arg Ala Gly His Ser Ser Tyr Thr Pro Leu Pro Gly Ser
        1                5                10

<210> 147
<211> 18
<212> PRT
<213> Dictyostelium sp.

<400> 147

        Lys Lys Leu Arg Gln Gln Lys Gln Gln Gly Tyr Gln Ala Ile Ile Asn
        1                5                10              15

        Asn Glu

<210> 148
<211> 15
<212> PRT
<213> Dictyostelium sp.

<400> 148

```
Arg Ser Lys Ser Asn Gln Asn Gln Ser Tyr Asn Leu Ile Gln Leu
1               5               10              15
```

<210> 149
<211> 17
<212> PRT
<213> Dictyostelium sp.

<400> 149

```
Arg Lys Thr Phe Tyr Asn Asn Asn Gln Tyr Asn Gly Tyr Asn Ile Ile
1               5               10              15

Asn
```

<210> 150
<211> 10
<212> PRT
<213> Homo sapiens

<400> 150

```
Pro Leu Ser Tyr Thr Arg Phe Ser Leu Ala
1               5               10
```

<210> 151
<211> 11
<212> PRT
<213> Homo sapiens

<400> 151

```
Met Thr Lys Glu Tyr Gln Asp Leu Gln His Leu
1               5               10
```

<210> 152
<211> 10
<212> PRT
<213> Homo sapiens

<400> 152

```
Ser Tyr Lys Tyr Ser Lys Val Asn Lys Glu
1               5               10
```

<210> 153
<211> 10
<212> PRT
<213> Homo sapiens

<400> 153

```
Pro Ala Ala Tyr Arg Gly Val Gly Asp Asp
1               5                   10
```

<210> 154
<211> 10
<212> PRT
<213> Homo sapiens

<400> 154

```
Thr Gly Val Tyr Val Lys Met Pro Pro Thr
1               5                   10
```

<210> 155
<211> 10
<212> PRT
<213> Homo sapiens

<400> 155

```
Leu Ile Ser Tyr Lys Gly Leu Pro Pro Glu
1               5                   10
```

<210> 156
<211> 11
<212> PRT
<213> Rattus norvegicus

<400> 156

```
Ala Ser Asp Tyr Gln Arg Leu Asn Leu Lys Leu
1               5                   10
```

<210> 157
<211> 11
<212> PRT
<213> Human immunodeficiency virus type 1

<400> 157

```
Arg Gln Gly Tyr Ser Pro Leu Ser Phe Gln Thr
1               5                   10
```

<210> 158
<211> 19
<212> PRT
<213> Homo sapiens

<400> 158

```
Arg Met Gln Ala Gln Pro Pro Gly Tyr Arg His Val Ala Asp Gly Glu
1               5                   10                  15

Asp His Ala
```

<210> 159
<211> 7
<212> PRT
<213> Artificial Sequence

<220>
<223> Dileucine-based sorting signal

<220>
<221> MISC_FEATURE
<222> (1)..(1)
<223> Xaa may be Asp or Glu

<220>
<221> misc_feature
<222> (2)..(4)
<223> Xaa can be any naturally occurring amino acid

<220>
<221> MISC_FEATURE
<222> (7)..(7)
<223> Xaa may be Leu or Ile

<400> 159

```
Xaa Xaa Xaa Xaa Leu Leu Xaa
1               5
```

<210> 160
<211> 9
<212> PRT
<213> Homo sapiens

<400> 160

```
Ser Asp Lys Gln Thr Leu Leu Pro Asn
1               5
```

<210> 161
<211> 9
<212> PRT
<213> Rattus norvegicus

<400> 161

```
Asp Glu Arg Ala Pro Leu Ile Arg Thr
1               5
```

<210> 162
<211> 9
<212> PRT
<213> Homo sapiens

<400> 162

```
Gln Glu Lys Asp Pro Leu Leu Lys Asn
1               5
```

<210> 163
<211> 9
<212> PRT
<213> Coturnix sp.

<400> 163

```
Thr Glu Arg Asn Pro Leu Leu Lys Ser
1               5
```

<210> 164
<211> 9
<212> PRT
<213> Homo sapiens

<400> 164

```
Gly Glu Asn Ser Pro Leu Leu Ser Gly
1               5
```

<210> 165
<211> 9
<212> PRT
<213> Homo sapiens

<400> 165

```
Glu Glu Lys Gln Pro Leu Leu Met Glu
1               5
```

<210> 166
<211> 9
<212> PRT
<213> Oryzias latipes

<400> 166

```
Gly Glu Arg Gln Pro Leu Leu Gln Ser
1               5
```

<210> 167
<211> 9
<212> PRT
<213> Gallus gallus

<400> 167

```
Pro Glu Ile Gln Pro Leu Leu Thr Glu
1               5
```

<210> 168
<211> 9
<212> PRT
<213> Carassius auratus

<400> 168

```
                     Glu Gly Arg Gln Pro Leu Leu Gly Asp
                     1               5
```

<210> 169
<211> 9
<212> PRT
<213> Homo sapiens

<400> 169

```
                     Glu Ala Asn Gln Pro Leu Leu Thr Asp
                     1               5
```

<210> 170
<211> 9
<212> PRT
<213> Gallus gallus

<400> 170

```
                     Glu Leu His Gln Pro Leu Leu Thr Asp
                     1               5
```

<210> 171
<211> 9
<212> PRT
<213> Danio rerio

<400> 171

```
                     Arg Glu Phe Glu Pro Leu Leu Asn Ala
                     1               5
```

<210> 172
<211> 9
<212> PRT
<213> Homo sapiens

<400> 172

```
                     Glu Glu Lys Met Ala Ile Leu Met Asp
                     1               5
```

<210> 173
<211> 9
<212> PRT
<213> Homo sapiens

<400> 173

```
                     Glu Glu Lys Leu Ala Ile Leu Ser Gln
                     1               5
```

<210> 174
<211> 9
<212> PRT
<213> Mus musculus

<400> 174

```
                              Ser Glu Arg Asp Val Leu Leu Asp Glu
                              1               5
```

<210> 175
<211> 9
<212> PRT
<213> Homo sapiens

<400> 175

```
                              Ser Glu Arg Arg Asn Leu Leu Glu Asp
                              1               5
```

<210> 176
<211> 9
<212> PRT
<213> Rattus norvegicus

<400> 176

```
                              Asp Asp Ser Gly Asp Leu Leu Pro Gly
                              1               5
```

<210> 177
<211> 9
<212> PRT
<213> Homo sapiens

<400> 177

```
                              Ser Gln Ile Lys Arg Leu Leu Ser Glu
                              1               5
```

<210> 178
<211> 9
<212> PRT
<213> Felis catus

<400> 178

```
                              Ser His Ile Lys Arg Leu Leu Ser Glu
                              1               5
```

<210> 179
<211> 9
<212> PRT
<213> Mus musculus

<400> 179

```
                              Arg Arg Thr Pro Ser Leu Leu Glu Gln
                              1               5
```

<210> 180
<211> 9
<212> PRT

<213> Homo sapiens

<400> 180

```
                              His Arg Thr Pro Ser Leu Leu Glu Gln
                              1               5
```

<210> 181
<211> 10
<212> PRT
<213> Rattus norvegicus

<400> 181

```
                              Glu Pro Arg Gly Ser Arg Leu Leu Val Arg
                              1               5                   10
```

<210> 182
<211> 19
<212> PRT
<213> Homo sapiens

<400> 182

```
              Met Asp Asp Gln Arg Asp Leu Ile Ser Asn Asn Glu Gln Leu Pro Met
              1               5                   10                  15


              Leu Gly Arg
```

<210> 183
<211> 19
<212> PRT
<213> Mus musculus

<400> 183

```
              Met Asp Asp Gln Arg Asp Leu Ile Ser Asn His Glu Gln Leu Pro Ile
              1               5                   10                  15


              Leu Gly Asn
```

<210> 184
<211> 20
<212> PRT
<213> Gallus gallus

<400> 184

```
              Met Ala Glu Glu Gln Arg Asp Leu Ile Ser Ser Asp Gly Ser Ser Gly
              1               5                   10                  15


              Val Leu Pro Ile
                          20
```

<210> 185

<211> 23
<212> PRT
<213> Danio rerio

<400> 185

```
Met Glu Pro Asp His Gln Asn Glu Ser Leu Ile Gln Arg Val Pro Ser
1               5                   10                  15

Ala Glu Thr Ile Leu Gly Arg
            20
```

<210> 186
<211> 23
<212> PRT
<213> Danio rerio

<400> 186

```
Met Ser Ser Glu Gly Asn Glu Thr Pro Leu Ile Ser Asp Gln Ser Ser
1               5                   10                  15

Val Asn Met Gly Pro Gln Pro
            20
```

<210> 187
<211> 24
<212> PRT
<213> Trypanosoma sp.

<400> 187

```
Arg Pro Arg Arg Arg Thr Glu Glu Asp Glu Leu Leu Pro Glu Glu Ala
1               5                   10                  15

Glu Gly Leu Ile Asp Pro Gln Asn
            20
```

<210> 188
<211> 19
<212> PRT
<213> Homo sapiens

<400> 188

```
Pro Asp Lys His Ser Leu Leu Val Gly Asp Phe Arg Glu Asp Asp Asp
1               5                   10                  15

Thr Ala Leu
```

<210> 189
<211> 9
<212> PRT
<213> Homo sapiens

<400> 189

```
                              Thr Glu Arg Glu Arg Leu Leu Asn Phe
                              1               5
```

<210> 190
<211> 8
<212> PRT
<213> Homo sapiens

<400> 190

```
                              Val Glu Thr Asp Asp Leu Ile Leu
                              1               5
```

<210> 191
<211> 7
<212> PRT
<213> Caenorhabditis elegans

<400> 191

```
                              Phe Glu Asn Asp Ser Leu Leu
                              1               5
```

<210> 192
<211> 9
<212> PRT
<213> Saccharomyces cerevisiae

<400> 192

```
                              Asn Glu Gln Ser Pro Leu Leu His Asn
                              1               5
```

<210> 193
<211> 8
<212> PRT
<213> Saccharomyces cerevisiae

<400> 193

```
                              Ser Glu Gln Thr Arg Leu Val Pro
                              1               5
```

<210> 194
<211> 8
<212> PRT
<213> Saccharomyces cerevisiae

<400> 194

```
                              Glu Val Asp Leu Asp Leu Leu Lys
                              1               5
```

<210> 195
<211> 13
<212> PRT

<213> Homo sapiens

<400> 195

```
                    Ser Phe His Asp Asp Ser Asp Glu Asp Leu Leu His Ile
                    1               5               10
```

<210> 196
<211> 13
<212> PRT
<213> Bos taurus

<400> 196

```
                    Thr Phe His Asp Asp Ser Asp Glu Asp Leu Leu His Val
                    1               5               10
```

<210> 197
<211> 13
<212> PRT
<213> Oryctolagus cuniculus

<400> 197

```
                    Ser Phe His Asp Asp Ser Asp Glu Asp Leu Leu Asn Ile
                    1               5               10
```

<210> 198
<211> 13
<212> PRT
<213> Gallus gallus

<400> 198

```
                    Ser Phe His Asp Asp Ser Asp Glu Asp Leu Leu Asn Val
                    1               5               10
```

<210> 199
<211> 13
<212> PRT
<213> Homo sapiens

<400> 199

```
                    Glu Glu Ser Glu Glu Arg Asp Asp His Leu Leu Pro Met
                    1               5               10
```

<210> 200
<211> 13
<212> PRT
<213> Gallus gallus

<400> 200

```
                    Asp Glu Ser Glu Glu Arg Asp Asp His Leu Leu Pro Met
```

               1                  5                  10

<210> 201
<211> 12
<212> PRT
<213> Homo sapiens

<400> 201

```
Gly Tyr His Asp Asp Ser Asp Glu Asp Leu Leu Glu
1               5               10
```

<210> 202
<211> 13
<212> PRT
<213> Homo sapiens

<400> 202

```
Ile Thr Gly Phe Ser Asp Asp Val Pro Met Val Ile Ala
1               5               10
```

<210> 203
<211> 13
<212> PRT
<213> Hydra sp.

<400> 203

```
Ile Asn Arg Phe Ser Asp Asp Glu Pro Leu Val Val Ala
1               5               10
```

<210> 204
<211> 13
<212> PRT
<213> Homo sapiens

<400> 204

```
Met Leu Glu Ala Ser Asp Asp Glu Ala Leu Leu Val Cys
1               5               10
```

<210> 205
<211> 13
<212> PRT
<213> Homo sapiens

<400> 205

```
Lys Asn Glu Thr Ser Asp Asp Glu Ala Leu Leu Leu Cys
1               5               10
```

<210> 206
<211> 12
<212> PRT
<213> Mus musculus

<400> 206

```
            Trp Val Val Glu Ala Glu Asp Glu Pro Leu Leu Ala
            1               5                   10
```

<210> 207
<211> 12
<212> PRT
<213> Homo sapiens

<400> 207

```
            Trp Val Ala Glu Ala Glu Asp Glu Pro Leu Leu Thr
            1               5                   10
```

<210> 208
<211> 12
<212> PRT
<213> Homo sapiens

<400> 208

```
            His Asp Asp Phe Ala Asp Asp Ile Ser Leu Leu Lys
            1               5                   10
```

<210> 209
<211> 13
<212> PRT
<213> Mus musculus

<400> 209

```
            Gly Arg Asp Ser Pro Glu Asp His Ser Leu Leu Val Asn
            1               5                   10
```

<210> 210
<211> 12
<212> PRT
<213> Peromyscus maniculatus

<400> 210

```
            Val Arg Cys His Pro Glu Asp Asp Arg Leu Leu Gly
            1               5                   10
```

<210> 211
<211> 13
<212> PRT
<213> Homo sapiens

<400> 211

```
            His Arg Val Ser Gln Asp Asp Leu Asp Leu Leu Thr Ser
            1               5                   10
```

<210> 212
<211> 13
<212> PRT

<213> Homo sapiens

<400> 212

| Ala | Ser | Val | Ser | Leu | Leu | Asp | Asp | Glu | Leu | Met | Ser | Leu |
|-----|-----|-----|-----|-----|-----|-----|-----|-----|-----|-----|-----|-----|
| 1   |     |     |     | 5   |     |     |     |     | 10  |     |     |     |

<210> 213
<211> 13
<212> PRT
<213> Homo sapiens

<400> 213

| Ala | Ser | Ser | Gly | Leu | Asp | Asp | Leu | Asp | Leu | Leu | Gly | Lys |
|-----|-----|-----|-----|-----|-----|-----|-----|-----|-----|-----|-----|-----|
| 1   |     |     |     | 5   |     |     |     |     | 10  |     |     |     |

<210> 214
<211> 13
<212> PRT
<213> Homo sapiens

<400> 214

| Val | Gln | Asn | Pro | Ser | Ala | Asp | Arg | Asn | Leu | Leu | Asp | Leu |
|-----|-----|-----|-----|-----|-----|-----|-----|-----|-----|-----|-----|-----|
| 1   |     |     |     | 5   |     |     |     |     | 10  |     |     |     |

<210> 215
<211> 13
<212> PRT
<213> Homo sapiens

<400> 215

| Asn | Ala | Leu | Ser | Trp | Leu | Asp | Glu | Glu | Leu | Leu | Cys | Leu |
|-----|-----|-----|-----|-----|-----|-----|-----|-----|-----|-----|-----|-----|
| 1   |     |     |     | 5   |     |     |     |     | 10  |     |     |     |

<210> 216
<211> 13
<212> PRT
<213> Drosophila sp.

<400> 216

| Thr | Val | Asp | Ser | Ile | Asp | Asp | Val | Pro | Leu | Leu | Ser | Asp |
|-----|-----|-----|-----|-----|-----|-----|-----|-----|-----|-----|-----|-----|
| 1   |     |     |     | 5   |     |     |     |     | 10  |     |     |     |

<210> 217
<211> 13
<212> PRT
<213> Mus musculus

<400> 217

| Gln | Glu | Glu | Cys | Pro | Ser | Asp | Ser | Glu | Glu | Asp | Glu | Gly |
|-----|-----|-----|-----|-----|-----|-----|-----|-----|-----|-----|-----|-----|
| 1   |     |     |     | 5   |     |     |     |     | 10  |     |     |     |

<210> 218

<211> 13
<212> PRT
<213> Mus musculus

<400> 218

```
Arg Asp Arg Asp Tyr Asp Glu Asp Asp Glu Asp Asp Ile
1               5                   10
```

<210> 219
<211> 15
<212> PRT
<213> Mus musculus

<400> 219

```
Leu Asp Glu Thr Glu Asp Asp Glu Leu Glu Tyr Asp Asp Glu Ser
1               5                   10                  15
```

<210> 220
<211> 10
<212> PRT
<213> Homo sapiens

<400> 220

```
Lys Asp Pro Asp Glu Val Glu Thr Glu Ser
1               5                   10
```

<210> 221
<211> 13
<212> PRT
<213> Homo sapiens

<400> 221

```
His Glu Phe Gln Asp Glu Thr Asp Thr Glu Glu Glu Thr
1               5                   10
```

<210> 222
<211> 14
<212> PRT
<213> Homo sapiens

<400> 222

```
Gln Glu Lys Glu Asp Asp Gly Ser Glu Ser Glu Glu Glu Tyr
1               5                   10
```

<210> 223
<211> 9
<212> PRT
<213> Homo sapiens

<400> 223

```
Gly Glu Asp Glu Glu Ser Glu Ser Asp
1               5
```

<210> 224
<211> 12
<212> PRT
<213> Homo sapiens

<400> 224

```
Gly Glu Asp Ser Asp Glu Glu Pro Asp His Glu Glu
1               5                   10
```

<210> 225
<211> 11
<212> PRT
<213> Homo sapiens

<400> 225

```
Leu Glu Asp Asp Ser Asp Glu Glu Glu Asp Phe
1               5                   10
```

<210> 226
<211> 9
<212> PRT
<213> Human cytomegalovirus

<400> 226

```
Lys Asp Ser Asp Glu Glu Glu Asn Val
1               5
```

<210> 227
<211> 13
<212> PRT
<213> Herpesvirus 3

<400> 227

```
Phe Glu Asp Ser Glu Ser Thr Asp Thr Glu Glu Glu Phe
1               5                   10
```

<210> 228
<211> 9
<212> PRT
<213> Human immunodeficiency virus type 1

<400> 228

```
Leu Glu Ala Gln Glu Glu Glu Glu Val
1               5
```

<210> 229
<211> 22
<212> PRT
<213> Saccharomyces cerevisiae

<400> 229

```
        Ala Asp Asp Leu Glu Ser Gly Leu Gly Ala Glu Asp Asp Leu Glu Gln
        1                   5                   10                  15

        Asp Glu Gln Leu Glu Gly
                        20
```

<210> 230
<211> 12
<212> PRT
<213> Saccharomyces cerevisiae

<400> 230

```
                Thr Glu Ile Asp Glu Ser Phe Glu Met Thr Asp Phe
                1                   5                   10
```

<210> 231
<211> 18
<212> PRT
<213> Saccharomyces cerevisiae

<400> 231

```
        Thr Glu Pro Glu Glu Val Glu Asp Phe Asp Phe Asp Leu Ser Asp Glu
        1                   5                   10                  15

        Asp His
```

<210> 232
<211> 15
<212> PRT
<213> Saccharomyces cerevisiae

<400> 232

```
            Phe Glu Ile Glu Glu Asp Asp Val Pro Thr Leu Glu Glu Glu His
            1                   5                   10                  15
```

<210> 233
<211> 707
<212> PRT
<213> Homo sapiens

<400> 233

```
        Lys Ile Tyr Asp Leu His Lys Lys Arg Ser Cys Asn Leu Asp Glu Gln
        1                   5                   10                  15

        Gln Glu Leu Val Glu Arg Asp Asp Glu Lys Gln Leu Met Asn Val Glu
                        20                  25                  30
```

Pro Ile His Ala Asp Ile Leu Leu Glu Thr Tyr Lys Arg Lys Ile Ala
        35              40                  45

Asp Glu Gly Arg Leu Phe Leu Ala Glu Phe Gln Ser Ile Pro Arg Val
        50              55                  60

Phe Ser Lys Phe Pro Ile Lys Glu Ala Arg Lys Pro Phe Asn Gln Asn
65              70                  75                  80

Lys Asn Arg Tyr Val Asp Ile Leu Pro Tyr Asp Tyr Asn Arg Val Glu
                85                  90                  95

Leu Ser Glu Ile Asn Gly Asp Ala Gly Ser Asn Tyr Ile Asn Ala Ser
            100                 105                 110

Tyr Ile Asp Gly Phe Lys Glu Pro Arg Lys Tyr Ile Ala Ala Gln Gly
        115                 120                 125

Pro Arg Asp Glu Thr Val Asp Asp Phe Trp Arg Met Ile Trp Glu Gln
        130                 135                 140

Lys Ala Thr Val Ile Val Met Val Thr Arg Cys Glu Glu Gly Asn Arg
145                 150                 155                 160

Asn Lys Cys Ala Glu Tyr Trp Pro Ser Met Glu Glu Gly Thr Arg Ala
                165                 170                 175

Phe Gly Asp Val Val Val Lys Ile Asn Gln His Lys Arg Cys Pro Asp
                180                 185                 190

Tyr Ile Ile Gln Lys Leu Asn Ile Val Asn Lys Lys Glu Lys Ala Thr
        195                 200                 205

Gly Arg Glu Val Thr His Ile Gln Phe Thr Ser Trp Pro Asp His Gly
        210                 215                 220

Val Pro Glu Asp Pro His Leu Leu Leu Lys Leu Arg Arg Arg Val Asn
225                 230                 235                 240

Ala Phe Ser Asn Phe Phe Ser Gly Pro Ile Val Val His Cys Ser Ala
                245                 250                 255

Gly Val Gly Arg Thr Gly Thr Tyr Ile Gly Ile Asp Ala Met Leu Glu
                260                 265                 270

Gly Leu Glu Ala Glu Asn Lys Val Asp Val Tyr Gly Tyr Val Val Lys
        275                 280                 285

123

```
Leu Arg Arg Gln Arg Cys Leu Met Val Gln Val Glu Ala Gln Tyr Ile
    290             295             300

Leu Ile His Gln Ala Leu Val Glu Tyr Asn Gln Phe Gly Glu Thr Glu
    305             310             315             320

Val Asn Leu Ser Glu Leu His Pro Tyr Leu His Asn Met Lys Lys Arg
                325             330             335

Asp Pro Pro Ser Glu Pro Ser Pro Leu Glu Ala Glu Phe Gln Arg Leu
            340             345             350

Pro Ser Tyr Arg Ser Trp Arg Thr Gln His Ile Gly Asn Gln Glu Glu
            355             360             365

Asn Lys Ser Lys Asn Arg Asn Ser Asn Val Ile Pro Tyr Asp Tyr Asn
    370             375             380

Arg Val Pro Leu Lys His Glu Leu Glu Met Ser Lys Glu Ser Glu His
385             390             395             400

Asp Ser Asp Glu Ser Ser Asp Asp Asp Ser Asp Ser Glu Glu Pro Ser
                405             410             415

Lys Tyr Ile Asn Ala Ser Phe Ile Met Ser Tyr Trp Lys Pro Glu Val
                420             425             430

Met Ile Ala Ala Gln Gly Pro Leu Lys Glu Thr Ile Gly Asp Phe Trp
            435             440             445

Gln Met Ile Phe Gln Arg Lys Val Lys Val Ile Val Met Leu Thr Glu
    450             455             460

Leu Lys His Gly Asp Gln Glu Ile Cys Ala Gln Tyr Trp Gly Glu Gly
465             470             475             480

Lys Gln Thr Tyr Gly Asp Ile Glu Val Asp Leu Lys Asp Thr Asp Lys
                485             490             495

Ser Ser Thr Tyr Thr Leu Arg Val Phe Glu Leu Arg His Ser Lys Arg
            500             505             510

Lys Asp Ser Arg Thr Val Tyr Gln Tyr Gln Tyr Thr Asn Trp Ser Val
            515             520             525

Glu Gln Leu Pro Ala Glu Pro Lys Glu Leu Ile Ser Met Ile Gln Val
    530             535             540
```

```
Val Lys Gln Lys Leu Pro Gln Lys Asn Ser Ser Glu Gly Asn Lys His
545             550             555             560

His Lys Ser Thr Pro Leu Leu Ile His Cys Arg Asp Gly Ser Gln Gln
            565             570             575

Thr Gly Ile Phe Cys Ala Leu Leu Asn Leu Leu Glu Ser Ala Glu Thr
            580             585             590

Glu Glu Val Val Asp Ile Phe Gln Val Val Lys Ala Leu Arg Lys Ala
        595             600             605

Arg Pro Gly Met Val Ser Thr Phe Glu Gln Tyr Gln Phe Leu Tyr Asp
    610             615             620

Val Ile Ala Ser Thr Tyr Pro Ala Gln Asn Gly Gln Val Lys Lys Asn
625             630             635             640

Asn His Gln Glu Asp Lys Ile Glu Phe Asp Asn Glu Val Asp Lys Val
            645             650             655

Lys Gln Asp Ala Asn Cys Val Asn Pro Leu Gly Ala Pro Glu Lys Leu
            660             665             670

Pro Glu Ala Lys Glu Gln Ala Glu Gly Ser Glu Pro Thr Ser Gly Thr
            675             680             685

Glu Gly Pro Glu His Ser Val Asn Gly Pro Ala Ser Pro Ala Leu Asn
    690             695             700

Gln Gly Ser
    705
```

<210> 234
<211> 8
<212> PRT
<213> Artificial Sequence

<220>
<223> cleavage site

<400> 234

```
Asp Ile Glu Thr Asn Pro Gly Pro
1               5
```

<210> 235
<211> 8
<212> PRT
<213> Artificial Sequence

<220>
<223> cleavage site

<400> 235

```
                              Asp Val Glu Thr Asn Pro Gly Pro
                              1                   5
```

<210> 236
<211> 8
<212> PRT
<213> Artificial Sequence

<220>
<223> cleavage site

<400> 236

```
                              Asp Val Glu Met Asn Pro Gly Pro
                              1                   5
```

<210> 237
<211> 8
<212> PRT
<213> Artificial Sequence

<220>
<223> cleavage site

<400> 237

```
                              Asp Val Glu Ser Asn Pro Gly Pro
                              1                   5
```

<210> 238
<211> 4
<212> PRT
<213> Artificial Sequence

<220>
<223> ER signal peptide

<400> 238

```
                              Lys Lys Phe Phe
                              1
```

<210> 239
<211> 4
<212> PRT
<213> Artificial Sequence

<220>
<223> intracellular retention signal

<400> 239

```
His Asp Glu Leu
1
```

**Description**

FIELD OF THE INVENTION

The present invention relates to constructs and approaches for expressing more than one chimeric antigen receptor (CAR) at the surface of a cell. The cell may be capable of specifically recognising a target cell, due to a differential pattern of expression (or non-expression) of two or more antigens by the target cell. The constructs of the invention enable modulation of the relative expression of the two or more CARs at the cell surface by a method involving co-expression of the CARs from a single vector.

BACKGROUND TO THE INVENTION

A number of immunotherapeutic agents have been described for use in cancer treatment, including therapeutic monoclonal antibodies (mAbs), immunoconjugated mAbs, radioconjugated mAbs and bi-specific T-cell engagers.

Typically these immunotherapeutic agents target a single antigen: for instance, Rituximab targets CD20; Myelotarg targets CD33; and Alemtuzumab targets CD52.

However, it is relatively rare for the presence (or absence) of a single antigen effectively to describe a cancer, which can lead to a lack of specificity.

Most cancers cannot be differentiated from normal tissues on the basis of a single antigen. Hence, considerable "on-target off-tumour" toxicity occurs whereby normal tissues are damaged by the therapy. For instance, whilst targeting CD20 to treat B-cell lymphomas with Rituximab, the entire normal B-cell compartment is depleted, whilst targeting CD52 to treat chronic lymphocytic leukaemia, the entire lymphoid compartment is depleted, whilst targeting CD33 to treat acute myeloid leukaemia, the entire myeloid compartment is damaged etc.

The predicted problem of "on-target off-tumour" toxicity has been borne out by clinical trials. For example, an approach targeting ERBB2 caused death to a patient with colon cancer metastatic to the lungs and liver. ERBB2 is over-expressed in colon caner in some patients, but it is also expressed on several normal tissues, including heart and normal vasculature.

For some cancers, targeting the presence of two cancer antigens may be more selective and therefore effective than targeting one. For example, B-chronic lymphocytic leukaemia (B-CLL) is a common leukaemia which is currently treated by targeting CD19. This treats the lymphoma but also depletes the entire B-cell compartment such that the treatment has a considerable toxic effect. B-CLL has an unusual phenotype in that CD5 and CD19 are co-expressed. By targeting only cells which express CD5 and CD19, it would be possible to considerably reduce on-target off-tumour toxicity.

There is thus a need for immunotherapeutic agents which are capable of more targeting to reflect the complex pattern of marker expression that is associated with many cancers.

*Chimeric Antigen Receptors (CARs)*

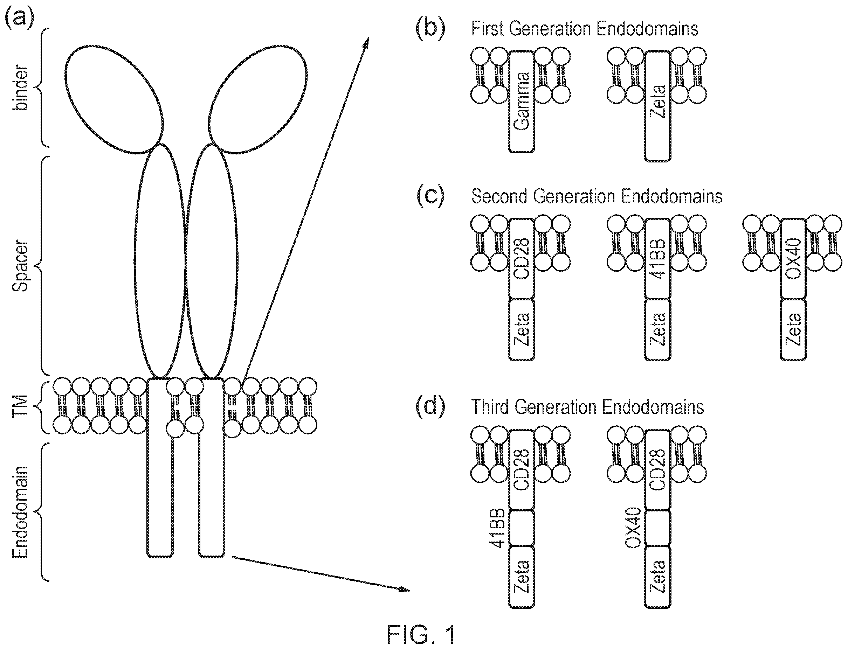

Chimeric antigen receptors are proteins which graft the specificity of a monoclonal antibody (mAb) to the effector function of a T-cell. Their usual form is that of a type I transmembrane domain protein with an antigen recognizing amino terminus, a spacer, a transmembrane domain all connected to a compound endodomain which transmits T-cell survival and activation signals (see Figure 1A).

The most common form of these molecules are fusions of single-chain variable fragments (scFv) derived from monoclonal antibodies which recognize a target antigen, fused via a spacer and a trans-membrane domain to a signaling endodomain. Such molecules result in activation of the T-cell in response to recognition by the scFv of its target. When T cells express such a CAR, they recognize and kill target cells that express the target antigen. Several CARs have been developed against tumour associated antigens, and adoptive transfer approaches using such CAR-expressing T cells are currently in clinical trial for the treatment of various cancers.

However, the use of CAR-expressing T cells is also associated with on-target, off tumour toxicity. For example, a CAR-based approach targeting carboxy anyhdrase-IX (CAIX) to treat renal cell carcinoma resulted in liver toxicity which is thought to be caused by the specific attack on bile duct epithelial cells (Lamers et al (2013) Mol. Ther. 21:904-912).

*Dual targeting CAR approaches*

In order to address the problem of "on target, off tumour" toxicity, CAR T cells have been developed with dual antigen specificty. In the "dual targeting" approach, two complementary CARs are co-expressed in the same T-cell population, each directed to a distant tumour target and engineered to provide complementary signals.

Wlikie et al (2012 J Clin Immunol 32:1059-1070) describe a dual targeting approach in which ErbB2- and MUC1-specific CARs are co-expressed. The ErbB2-specific CAR provided the CD3ζ signal only and the MUC1-specific CAR provided the CD28 co-stimulatory signal only. It was found that complementary signalling occurred in the presence of both antigens, leading to IL-2 production. However, IL-2 production was modest when compared to control CAR-engineered T cells in which signaling is delivered by a fused CD28+CD3ζ endodomain.

A similar approach was described by Kloss et al (2013 Nature Biotechnol. 31:71-75) in which a CD-19 specific CAR was used which provides a CD3ζ-mediated activation signal in combination with a chimeric co-stimulatory receptor specific for PSMA. With this 'co-CAR' design, the CAR T-cell receives an activation signal when it encounters a target cell with one antigen, and a co-stimulatory signal when it encounters a target cell with the other antigen, and only receives both activatory and co-stimulatory signals upon encountering target cells bearing both antigens.

This represents an early attempt at restricting CAR activity to only a target cell bearing two antigens. This approach however is limited: although CAR T-cell activity will be greatest against targets expressing both antigens, CAR T-cells will still kill targets expressing only antigen recognized by the activatory CAR; further, co-stimulation results in prolonged effects on T-cells which last long after release of target cell. Hence, activity against single-antigen positive T-cells equal to that against double-positives might be possible for example in a situation where single-positive tissues are adjacent to, or in a migratory path from double positive tumour.

There is thus a need for improved CAR-based therapeutic approaches with reduced on-target off-tumour toxicity where T-cell activation is wholly restricted to target cells which express both antigens.

DESCRIPTION OF THE FIGURES

Figure 1: (a) Generalized architecture of a CAR: A binding domain recognizes antigen; the spacer elevates the binding domain from the cell surface; the trans-membrane domain anchors the protein to the membrane and the endodomain transmits signals. (b) to (d): Different generations and permutations of CAR endodomains: (b) initial designs transmitted ITAM signals alone through FcεR1-γ or CD3ζ endodomain, while later designs transmitted additional (c) one or (d) two co-stimulatory signals in cis.

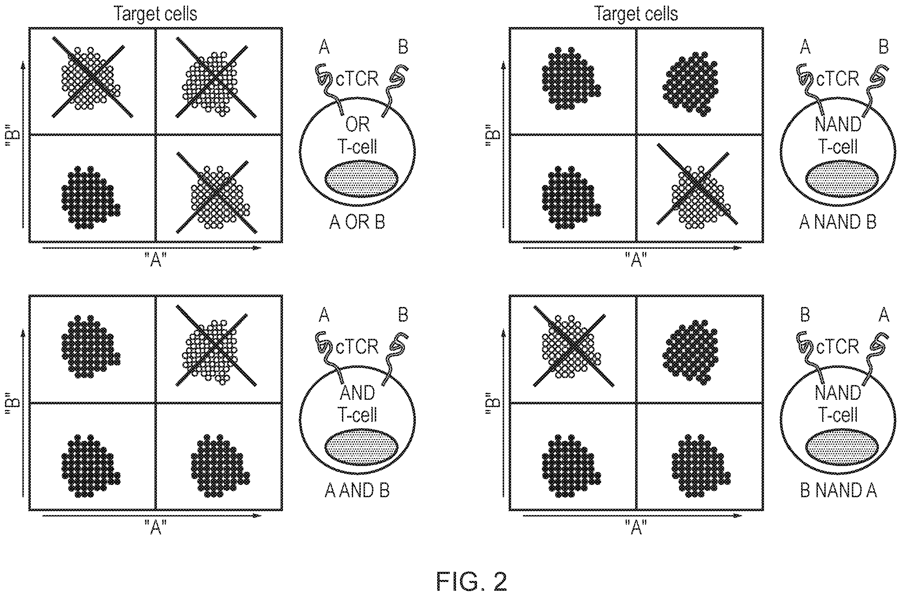

Figure 2: Schematic diagram illustrating Logic Gates
The invention relates to engineering T-cells to respond to logical rules of target cell antigen expression. This is best illustrated with an imaginary FACS scatter-plot. Target cell populations express both, either or neither of antigens "A" and "B". Different target populations (marked in red) are killed by T-cells transduced with a pair of CARs connected by different gates. With OR gated receptors, both single-positive and double-positive cells will be killed. With AND gated receptors, only double-positive target cells are killed. With AND NOT gating, double-positive targets are preserved while single-positive targets will be killed.

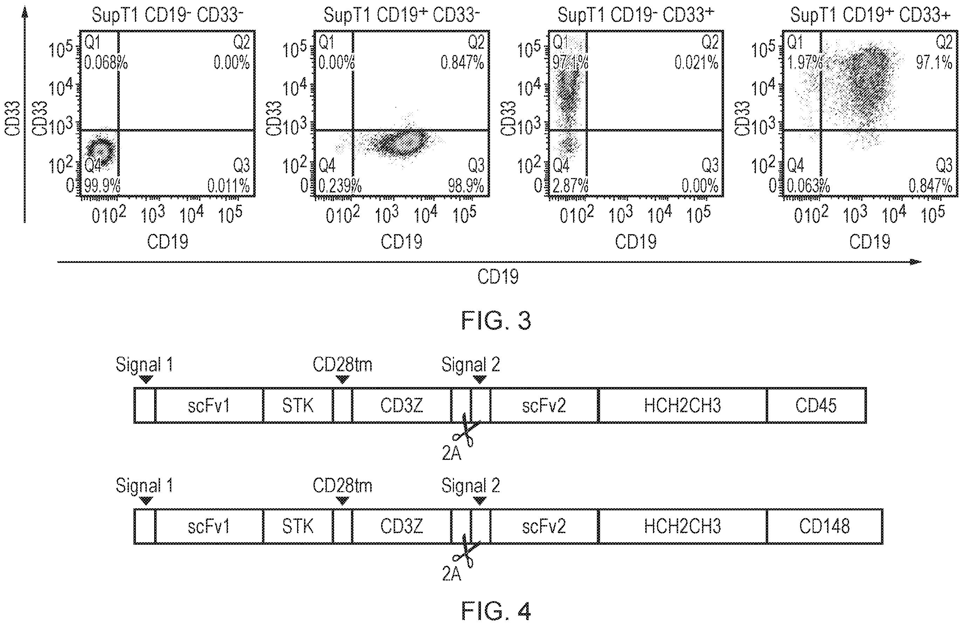

Figure 3: Creation of target cell populations
SupT1 cells were used as target cells. These cells were transduced to express either CD19, CD33 or both CD19 and CD33. Target cells were stained with appropriate antibodies and analysed by flow cytometry.

Figure 4: Cartoon showing both versions of the cassette used to express both AND gates Activating and inhibiting CARs were co-expressed once again using a FMD-2A sequence. Signal1 is a signal peptide derived from IgG1 (but can be any effective signal peptide). scFv1 is the single-chain variable segment which recognizes CD19 (but can be a scFv or peptide loop or ligand or in fact any domain which recognizes any desired arbitrary target). STK is the CD8 stalk but may be any non-bulky extracellular domain. CD28tm is the CD28 trans-membrane domain but can by any stable type I protein transmembrane domain and CD3Z is the CD3 Zeta endodomain but can be any endodomain which contains ITAMs. Signal2 is a signal peptide derived from CD8 but can be any effective signal peptide which is different in DNA sequence from signal 1. scFv recognizes CD33 but as for scFv1 is arbitrary. HC2CH3 is the hinge-CH2-CH3 of human IgG1 but can be any bulky extracellular domain. CD45 and CD148 are the transmembrane and endodomains of CD45 and CD148 respectively but can be derived from any of this class of protein.

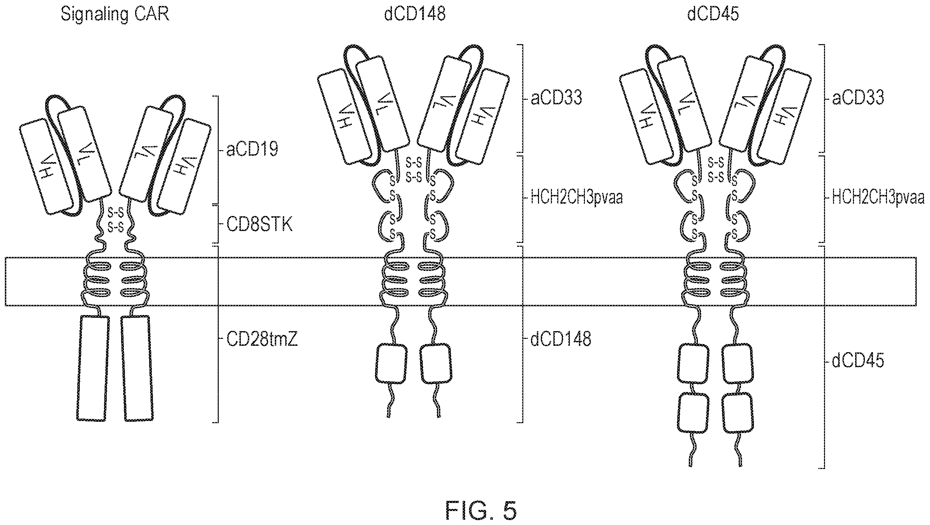

Figure 5: Schematic representation of the protein structure of chimeric antigen receptors (CARs) for the AND gates
The stimulatory CAR consisting of an N-terminal anti-CD19 scFv domain followed by the extracellular stalk region of human CD8, human CD28 transmembrane domain and human CD3 Zeta (CD247) intracellular domain. Two inhibitory CARs were tested. These consist of an N-terminal anti-CD33 scFv domain followed by the extracellular hinge, CH2 and CH3 (containing a pvaa mutation to reduce FcR binding) region of human IgG1 followed by the transmembrane and intracellular domain of either human CD148 or CD45. "S" depicts the presence of disulphide bonds.

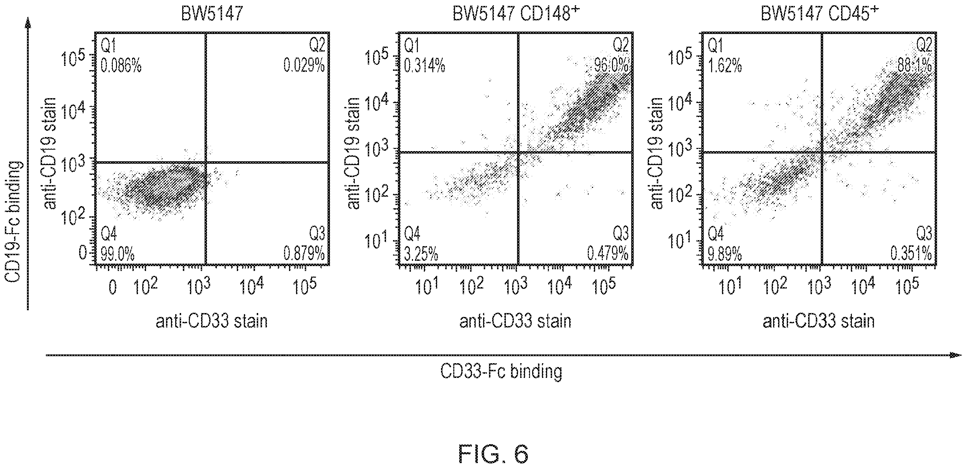

**Figure 6:** Co-expression of activation and inhibitory CARs

BW5147 cells were used as effector cells and were transduced to express both the activation anti-CD19 CAR and one of the inhibitory anti-CD33 CARs. Effector cells were stained with CD19-mouse-Fc and CD33-rabbit-Fc and with appropriate secondary antibodies and analysed by flow cytometry.

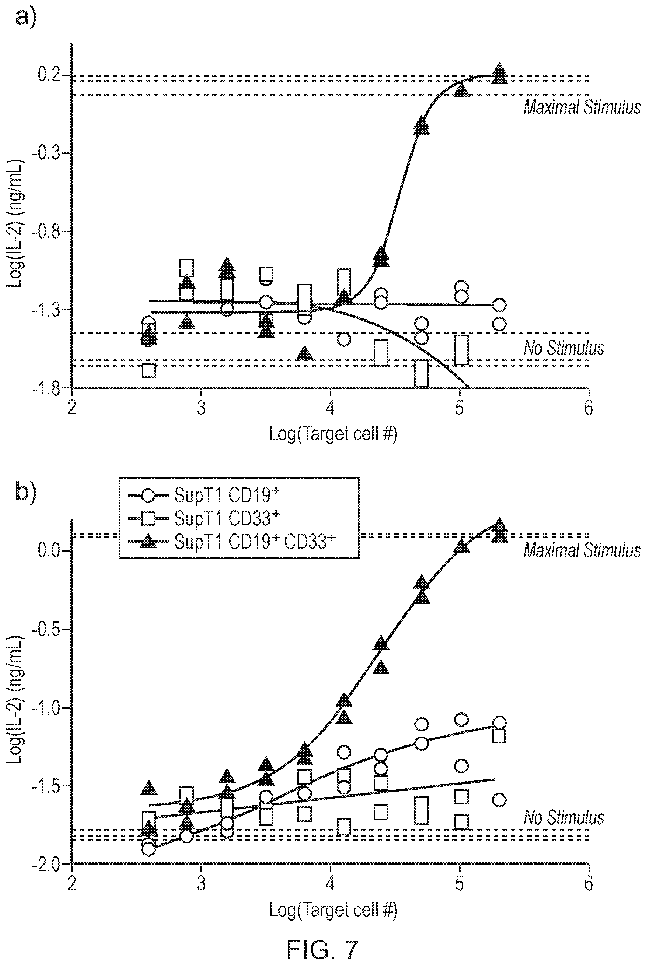

**Figure 7:** Functional analysis of the AND gates

Effector cells (5x10^4 cells) expressing activation anti-CD19 CAR and the inhibitory anti-CD33 CAR with the A) CD148 or B) CXD45 intracellular domain were co- incubated with a varying number of target cells and IL-2 was analysed after 16hours by ELISA. The graph displays the maximum IL-2 secretion from a chemical stimulation (PMA and Ionomycin) of the effector cells alone and the background IL-2 from effector cells without any stimulus from three replicates.

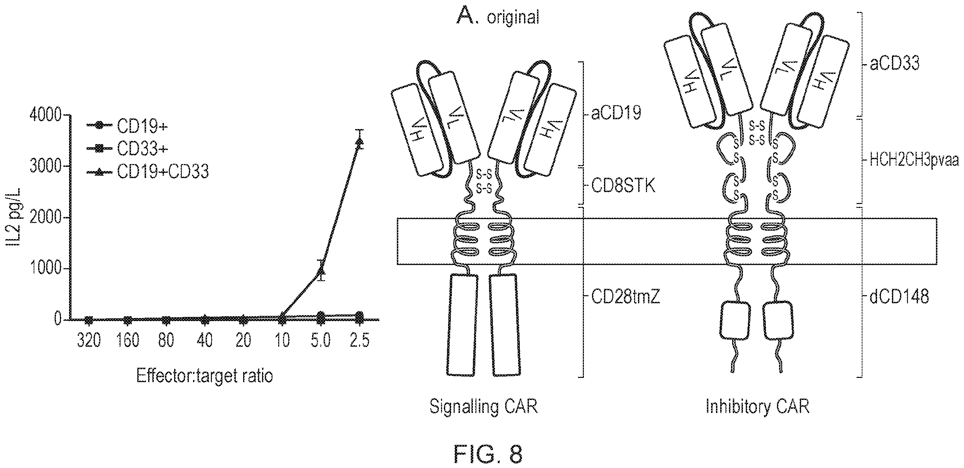

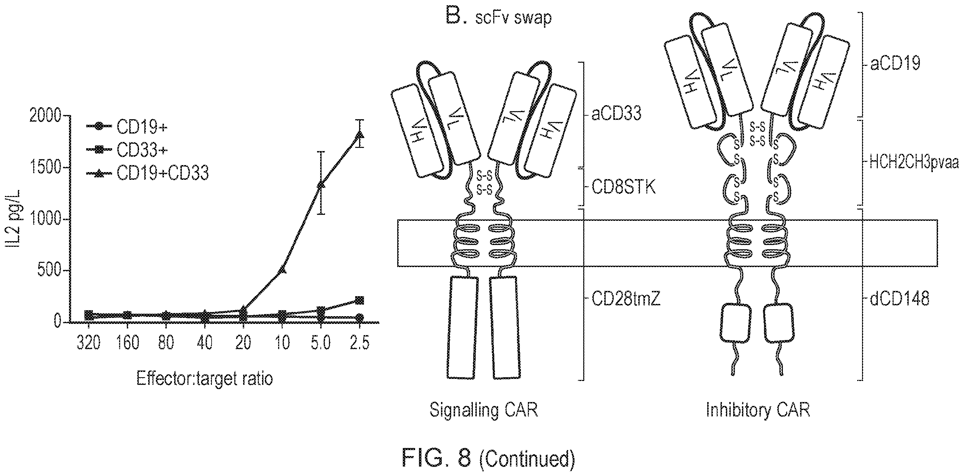

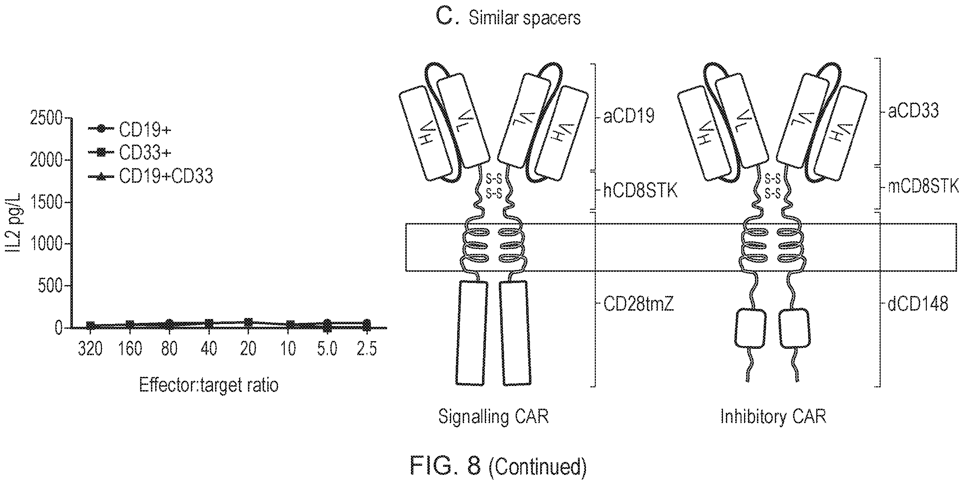

**Figure 8:** Dissection of AND gate function

**A.** The prototype AND gate is illustrated on the right and its function in response to CD19, CD33 single and CD19, CD33 double positive targets is shown on the left. **B.** The scFvs are swapped so the activating endodomain is triggered by CD33 and the inhibitory endodomain is activated by CD19. This AND gate remains functional despite this scFv swap. **C.** The CD8 mouse stalk replaced Fc in the spacer of the inhibitory CAR. With this modification, the gate fails to respond to either CD19 single positive or CD19, CD33 double positive targets.

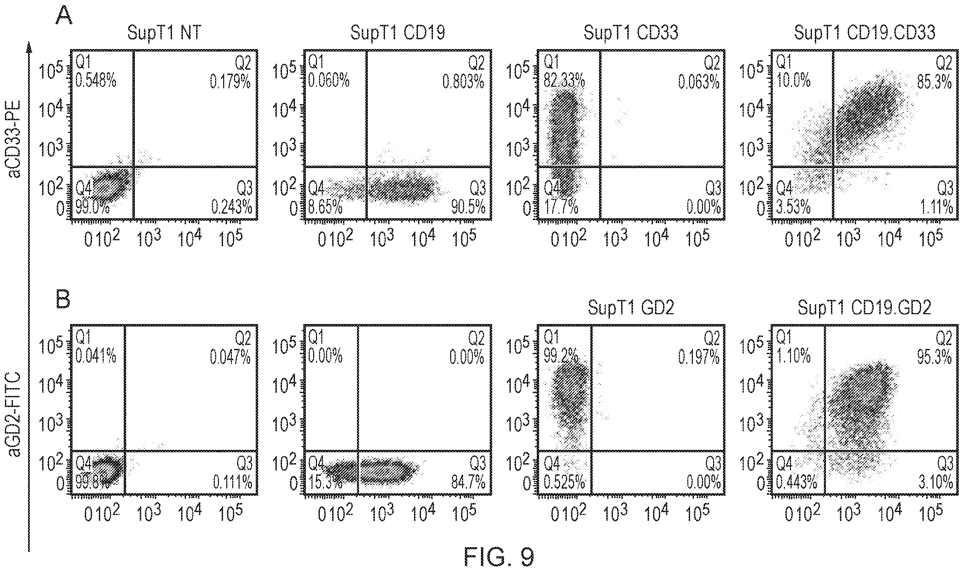

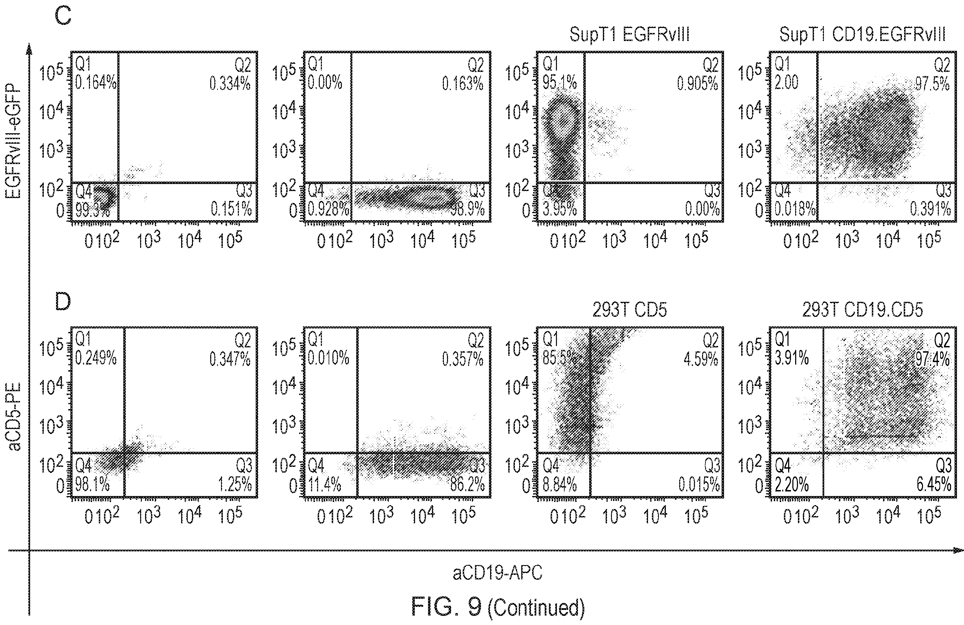

**Figure 9:** Expression of target antigens on artificial target cells

**A.** Shows flow cytometry scatter plots CD19 vs CD33 of the original set of artificial target cells derived from SupT1 cells. From left to right: double negative SupT1 cells, SupT1 cells positive for CD19, positive for CD33 and positive for both CD19 and CD33. **B.** Shows flow cytometry scatter plots CD19 vs GD2 of the artificial target cells generated to test the CD19 AND GD2 gate: From left to right: negative SupT1 cells, SupT1 cells expressing CD19, SupT1 cells transduced with GD2 and GM3 synthase vectors which become GD2 positive and SupT1 cells transduced with CD19 as well as GD2 and GM3 synthase which are positive for both GD2 and CD19. **C**. Shows flow cytometry scatter plots of CD19 vs EGFRvIII of the artificial targets generated to test the CD19 AND EGFRvIII gate. From left to right: negative SupT1 cells, SupT1 cells expressing CD19, SupT1 cells transduced with EGFRvIII and SupT1 cells transduced with both CD19 and EGFRvIII. **D.** Shows flow cytometry scatter plots of CD19 vs CD5 of the artificial targets generated to test the CD19 AND CD5 gate. From left to right: negative 293T cells, 293T cells transduced with CD19, 293T cells transduced with CD5, 293T cells transduced with both CD5 and CD19 vectors.

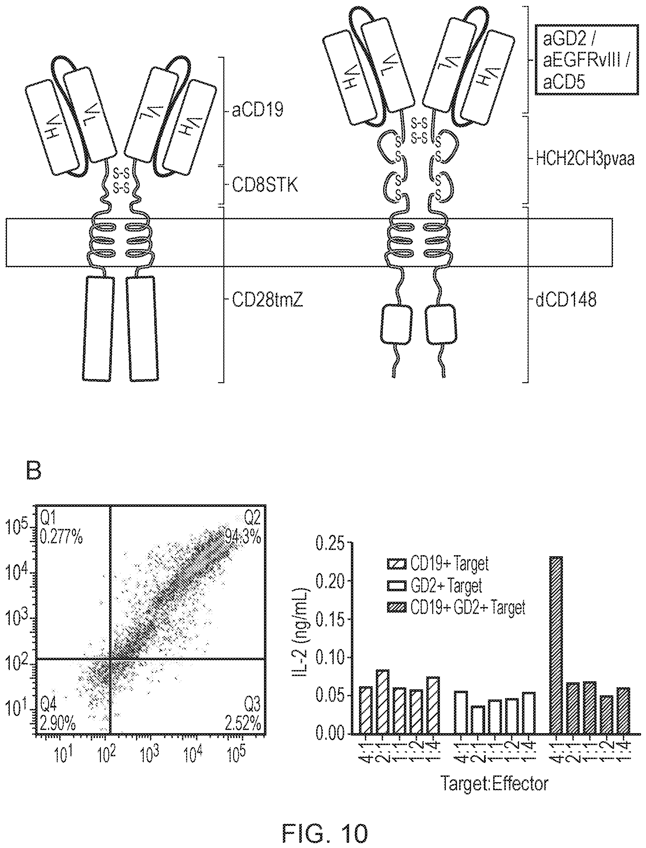

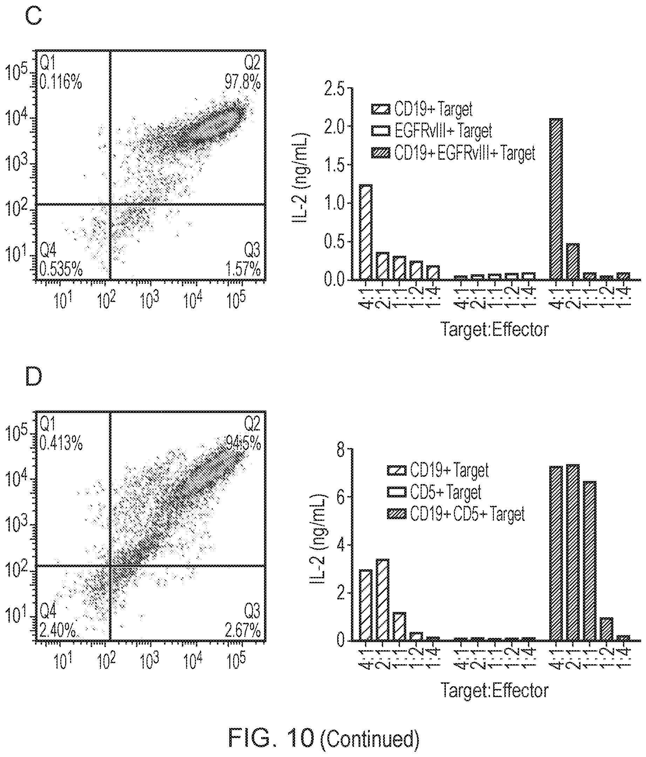

**Figure 10:** Generalizability of the AND gate

**A.** Cartoon of AND gate modified so the second CAR's specificity is changed from the original specificity of CD33, to generate 3 new CARs: CD19 AND GD2, CD19 AND EGFRvIII, CD19 AND CD5. **B.** CD19 AND GD2 AND gate: Left: expression of AND gate is shown recombinant CD19-Fc staining (x-axis) for the CD19 CAR, versus anti-human-Fc staining (Y-axis) for the GD2 CAR. Right: function in response to single positive and double positive targets. **C.** CD19 AND EGFRvIII AND gate: Left: expression of AND gate is shown recombinant CD19-Fc staining (x-axis) for the CD19 CAR, versus anti-human-Fc staining (Y-axis) for the EGFRvIII CAR. Right: function in response to single positive and double positive targets. **D.** CD19 AND CD5 AND gate: Left: expression of AND gate is shown recombinant CD19-Fc staining (x-axis) for the CD19 CAR, versus anti-human-Fc staining (Y-axis) for the CD5 CAR. Right: function in response to single positive and double positive targets.

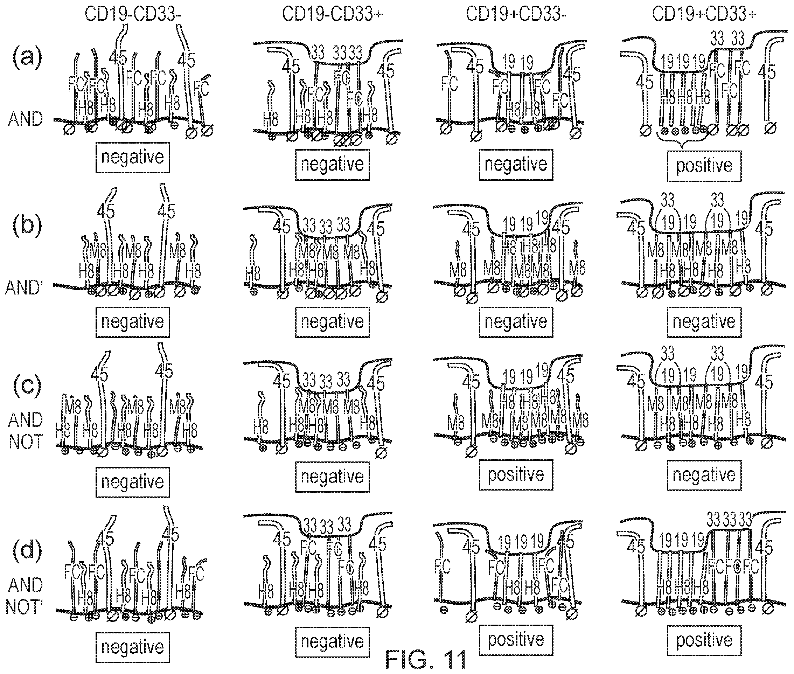

**Figure 11:** Kinetic segregation model of CAR logic gates

Model of kinetic segregation and behaviour of AND gate, NOT AND gate and controls. CARs recognize either CD19 or CD33. The immunological synapse can be imagined between the blue line, which represents the target cell membrane and the red line, which represents the T-cell membrane. '45' is the native CD45 protein present on T-cells. 'H8' is a CAR ectodomain with human CD8 stalk as the spacer. 'Fc' is a CAR ectodomain with human HCH2CH3 as the spacer. 'M8' is a CAR ectodomain with murine CD8 stalk as the spacer. '19' represents CD19 on the target cell surface. '33' represents CD33 on the target cell surface. The symbol '⊕' represents an activating endodomain containing ITAMS. The symbol '⊖' represents a phosphatase with slow kinetics - a 'ligation on' endodomain such as one comprising of the catalytic domain of PTPN6 or an ITIM. The symbol '∅' represents a phosphatase with fast kinetics - a 'ligation off' endodomain such as the endodomain of CD45 or CD148. This symbol is enlarged in the figure to emphasize its potent activity.

(a) Shows the postulated behaviour of the functional AND gate which comprises of a pair of CARs whereby the first CAR recognizes CD19, has a human CD8 stalk spacer and an activating endodomain; and the second CAR recognizes CD33, has an Fc spacer and a CD148 endodomain;

(b) Shows the postulated behaviour of the control AND gate. Here, the first CAR recognizes CD19, has a human CD8 stalk spacer and an activating endodomain; and the second CAR recognizes CD33, but has a mouse CD8 stalk spacer and a CD148 endodomain;

(c) Shows the behaviour of a functional AND NOT gate which comprises of a pair of CARs whereby the first CAR recognizes CD19, has a human CD8 stalk spacer and an activating endodomain; and the second CAR recognizes CD33, has a mouse CD8 stalk spacer and a PTPN6 endodomain;

(d) Shows the postulated behaviour of the control AND NOT gate which comprises of a pair of CARs whereby the first CAR recognizes CD19, has a human CD8 stalk spacer and an activating endodomain; and the second CAR recognizes CD33, but has an Fc spacer and a PTPN6 endodomain;

In the first column, target cells are both CD19 and CD33 negative. In the second column, targets are CD19 negative and CD33 positive. In the third column, target cells are CD19 positive and CD33 negative. In the fourth column, target cells are positive for both CD19 and CD33.

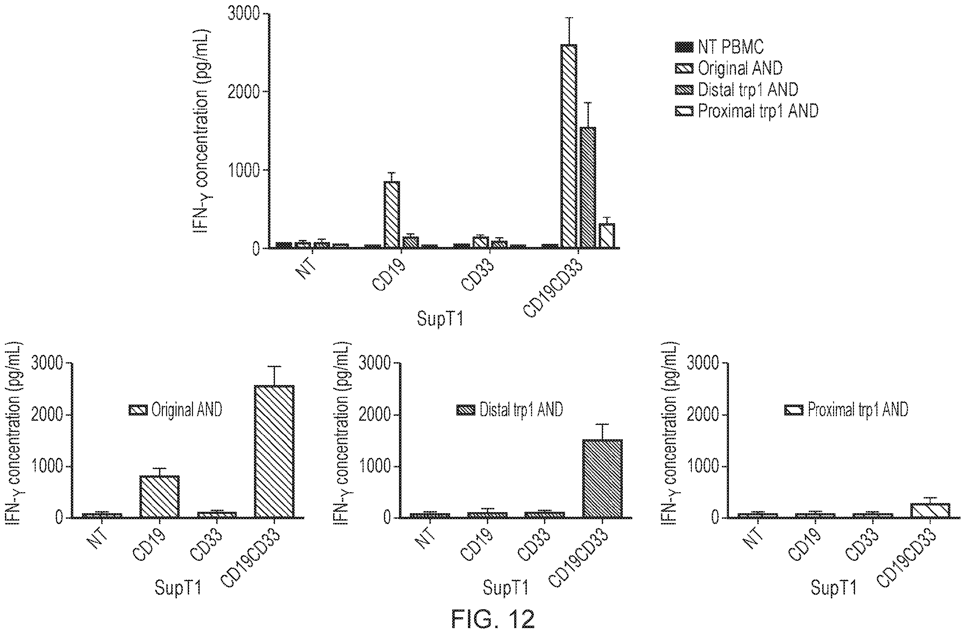

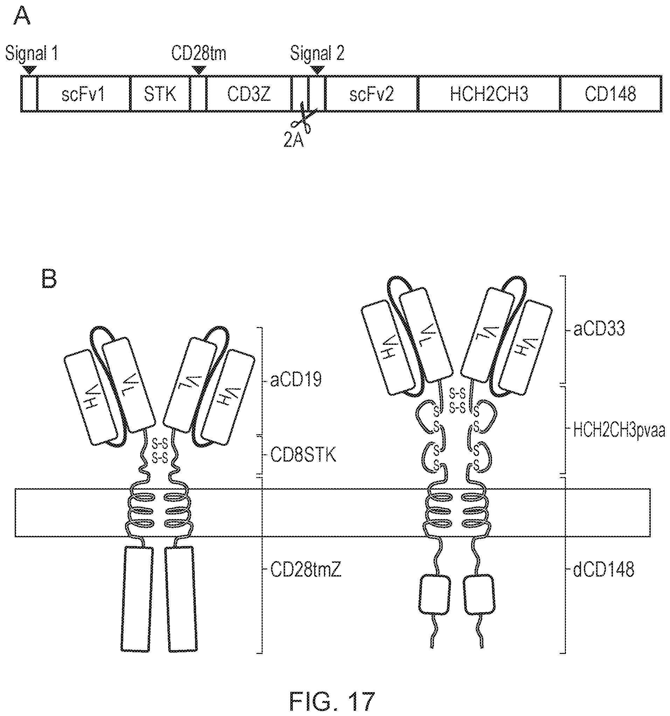

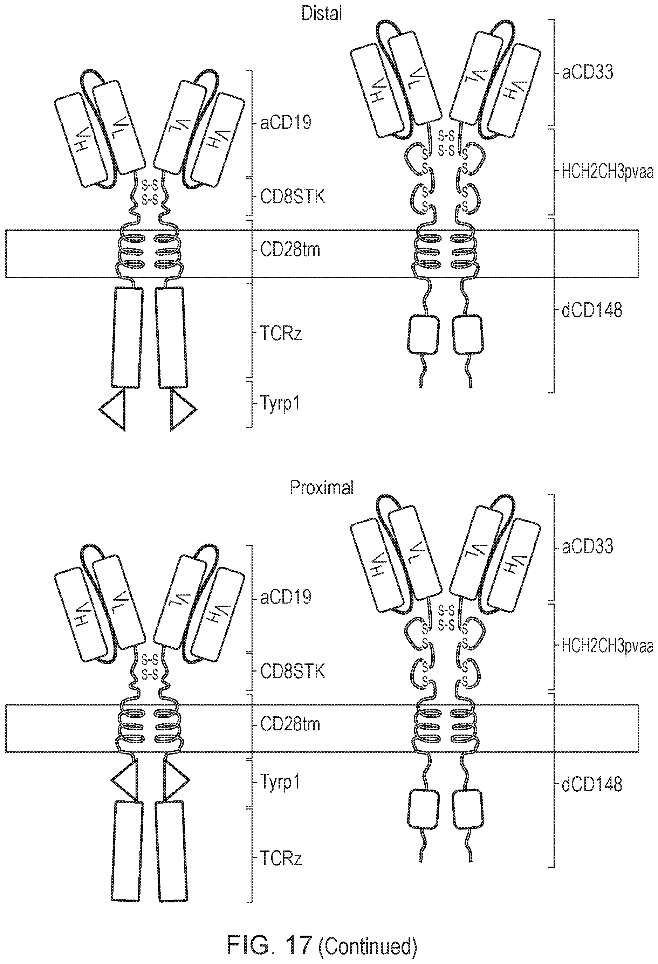

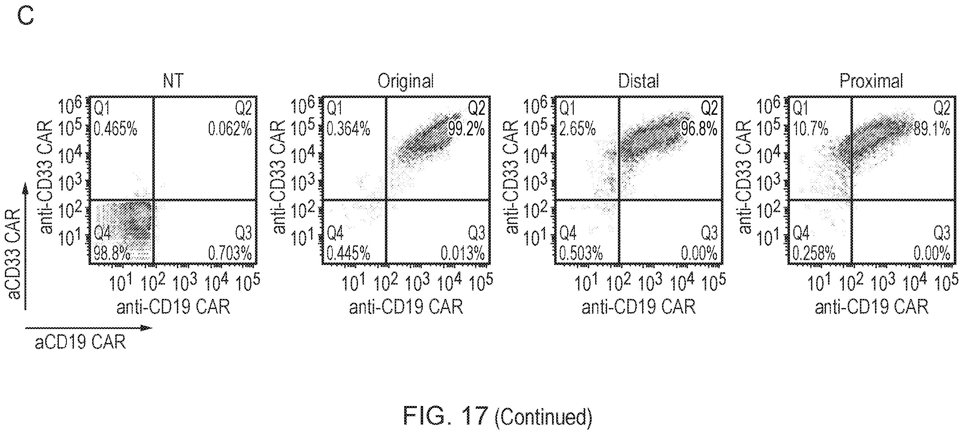

**Figure 12:** AND gate functionality in primary cells
PBMCs were isolated from blood and stimulated using PHA and IL-2. Two days later the cells were transduced on retronectin coated plates with retro virus containing the CD19:CD33 AND gate construct with the Tyrp1 retention signal placed either distal or proximal to the endodomain (see legend for Figure 17). On day 5 the expression level of the two CARs translated by the AND gate construct was evaluated via flow cytometry and the cells were depleted of CD56+ cells (predominantly NK cells). On day 6 the PBMCs were placed in a co-culture with target cells at a 1:2 effector to target cell ratio. On day 8 the supernatant was collected and analysed for IFN-gamma secretion via ELISA. The results are aggregated from four different donors.

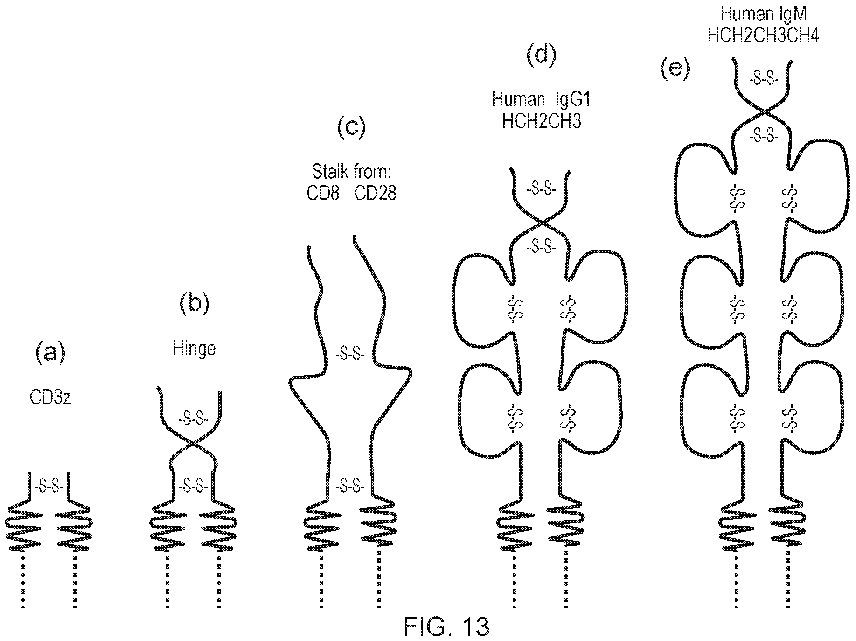

**Figure 13:** A selection / hierarchy of possible spacer domains of increasing size is shown. The ectodomain of CD3-Zeta is suggested as the shortest possible spacer, followed by the (b) the IgG1 hinge. (c) murine or human CD8 stalk and the CD28 ectodomains are considered intermediate in size and co-segregate. (d) The hinge, CH2 and CH3 domain of IgG1 is bigger and bulkier, and (e) the hinge, CH2, CH3 and CH4 domain of IgM is bigger still. Given the properties of the target molecules, and the epitope of the binding domains on said target molecules, it is possible to use this hierarchy of spacers to create a CAR signaling system which either co-segregates or segregates apart upon synapse formation.

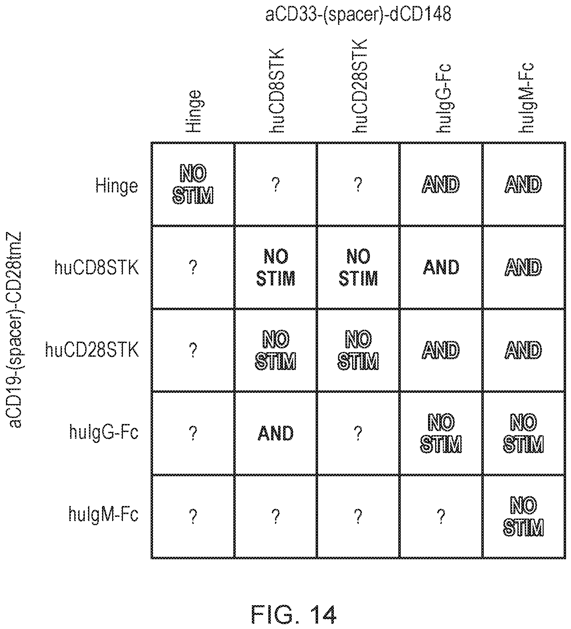

**Figure 14:** Matrix for the AND gate platform.
Multiple AND gates were produced and tested with various combinations of the spacers: hinge; huCD8STK; huCD28ecto; huIgG (HCH2CH3pvaa); and huIgM (CH2HCH3CH4) as described in the legend for Figure 13. They were tested in the AND gate platform as previously described with a CD19 signalling CAR and a CD33 inhibitory CAR with a CD148 ligation-off inhibitory endodomain. Blue = predicted; Red= proven; NO STIM = no stimulation with any cognate targets; AND = functions as AND gate.

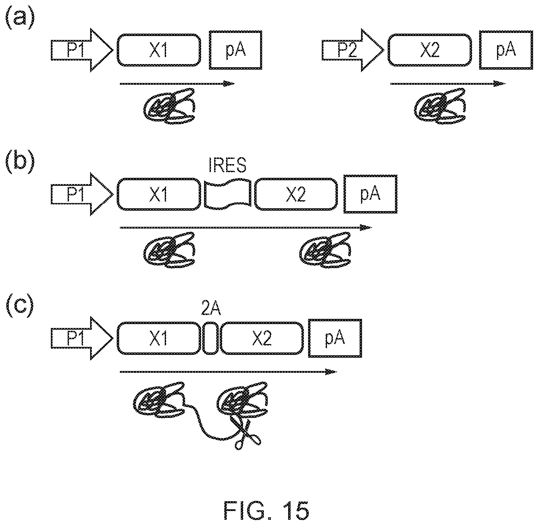

**Figure 15:** Methods utilised to express different proteins from the same vector (a) Two different promoters within the same cassette result in two different transcripts which each give rise to separate proteins. (b) Use of an Internal Ribosome Entry sequence (IRES) leads to a single transcript which is translated into two separate proteins. (c) Use of the FMDV 2A peptide results in a single transcript, and a single polyprotein which rapidly cleaves into two separate proteins.

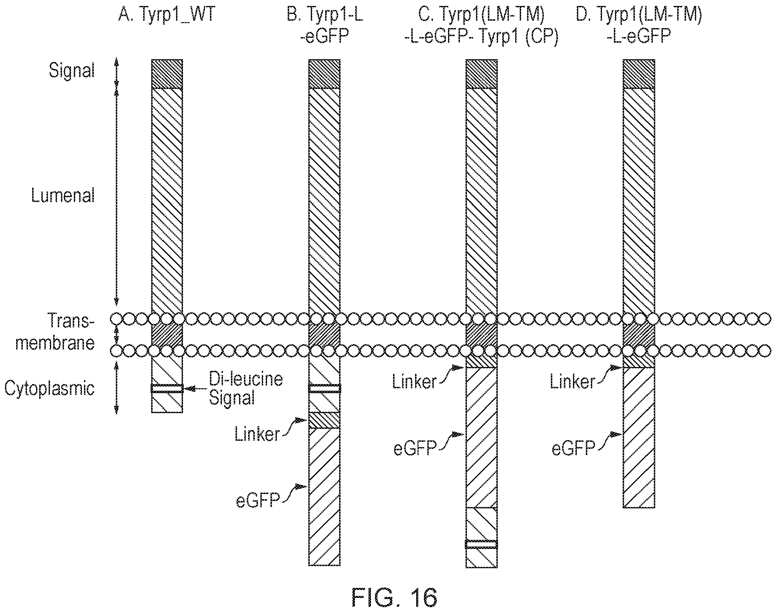

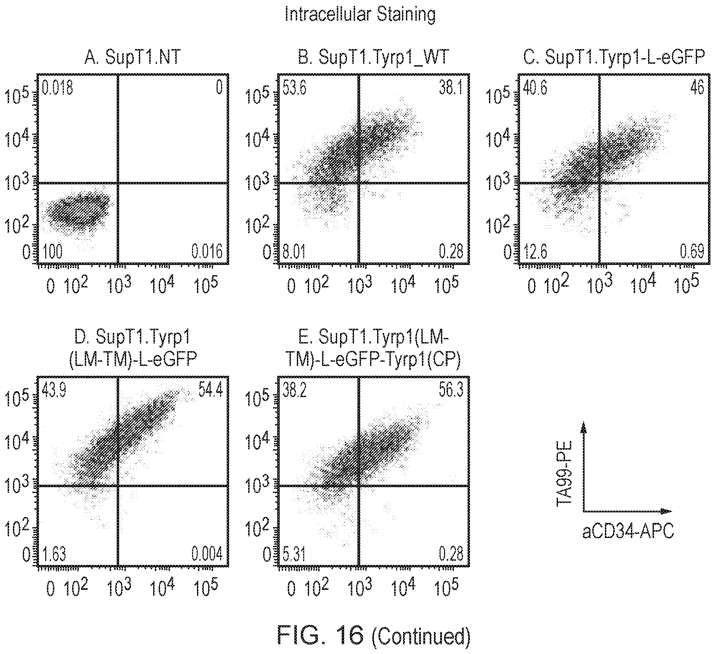

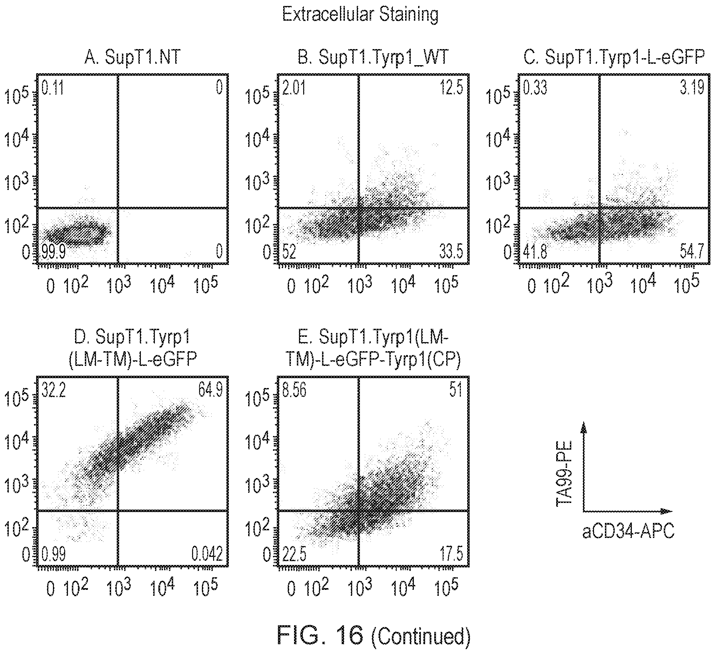

**Figure 16:** TYRP1 endodomain is able to direct the retention of a transmembrane protein with a complex endodomain
Tyrp1 is a type I transmembrane protein, 537aa long. The di-leucine motif, which retains the protein in the intracellular compartment, is indicated as a black rectangle on the cytoplasmic domain. (A) Tyrp1 (wt). Wild type Tyrp1 consists of a peptide signal, a luminal domain, a transmembrane domain, and a cytoplasmic domain. The cytoplasmic domain contains the di- leucine retention signal. (B) Tyrp1 (wt)-SG Linker- eGFP. This construct contains the wild type Tyrp1 simply fused to eGFP via a serine-glycine-glycine-glycine-serine linker. The Tyrp1-L-eGFP represents the cytoplasmic- proximal Tyrp1. (C) Tyrp1 Lumenal (LM)-Transmembrane (TM)- SG Linker- eGFP- Tyrp1 Cytoplasmic (CP). This construct constitutes the cytoplasmic- distal Tyrp1, since SG linker- eGFP interposes between the transmembrane and cytoplasmic domains. D: Tyrp1 Lumenal (LM)- Transmembrane (TM)-SG Linker- eGFP. This construct serves as the positive control, as the cytoplasmic domain containing the retention signal has been excluded. All constructs are co-expressed with IRES.CD34. Staining of transduced SupT1 cells is shown with intracellular and surface staining bottom left / right respectively.

**Figure 17:** Functionality of the TYRP1 retention signal in primary cells

A construct was generated which co-express an anti-CD19 and an anti-CD33 CAR using a FMD-2A like peptide. Two variants of this construct were also generated: in the first variant,the di-leucine motif from TYRP1 was inserted into the anti-CD19 CAR endodomain just proximal to the TM domain; In the second variant the same TYRP1 di-lecuine motif was attached to the carboxy-terminus of the anti-CD19 CAR endodomain. PBMCs were isolated from blood and stimulated using PHA and IL-2. Two days later the cells were transduced on retronectin coated plates with retro virus containing the different CD19:CD33 CAR constructs. On day 5 the expression level of the two CARs translated by the construct was evaluated via flow cytometry using recombinant CD19-Fc and CD33-Fc fusions. A. Shows cartoon of the synthetic gene constructed to allow co-expression; B. Shows a cartoon of the subsequent pairs of proteins generated by the three construcs; C. Shows expression of the two receptors by flow-cytometry. In the original construct, both CARs are equally expressed. With incorporation of the di-leucine motif distally in the endodomain of the anti-CD19 CAR, the CD33 CAR expression remains constant but the CD19 expression drops to intermediate levels. With incorporation of the di-leucine motif proximally in the endodomain of the anti-CD19 CAR, the CD33 CAR expression remains constant, but the CD19 expression drops to low levels.

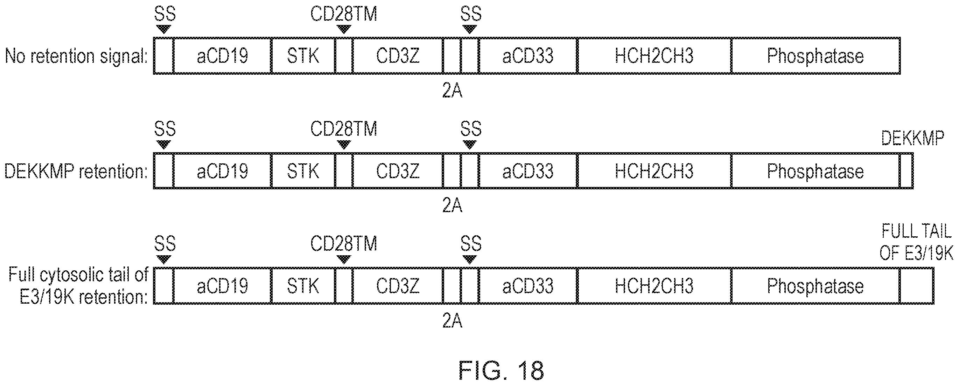

**Figure 18:** Retention signal from cytosolic tail of E3/19K

A construct was generated which co-expresses an anti-CD19 and an anti-CD33 CAR using a FMD-2A like peptide. Two variants of this construct were also generated: in the first variant, the last 6aa from E3/19K (DEKKMP), which were found to be critical for its Golgi/ER retention ability, were attached to the carboxy-terminus of the anti-CD33 CAR endodomain; in the second variant, the entire cytosolic tail of adenovirus E3/19K protein was attached to the carboxy-terminus of the anti-CD33 CAR endodomain

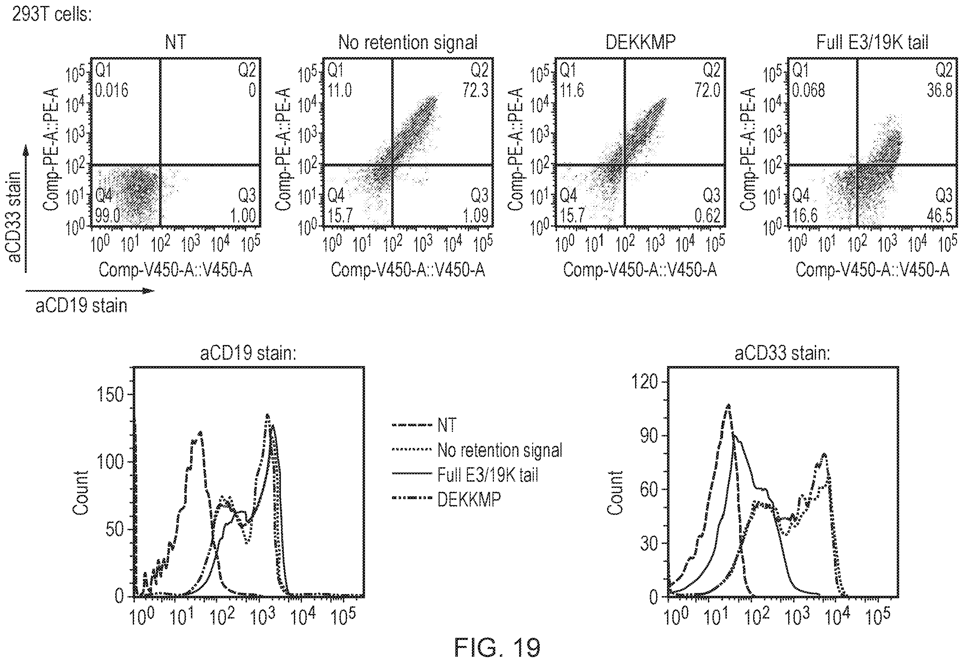

**Figure 19**: Functionality of E3/19K retention signal

The constructs shown in Figure 4 were transfected into 293T cells and the expression level of the two CARs translated by the construct was evaluated via flow cytometry using recombinant CD19-Fc and CD33-Fc fusions. A clear retention was observed when the full length adenovirus E3/19K protein, or the DEKKMP motif was placed on the anti-CD33 receptor. The anti-CD19 receptor expression levels were unaffected.

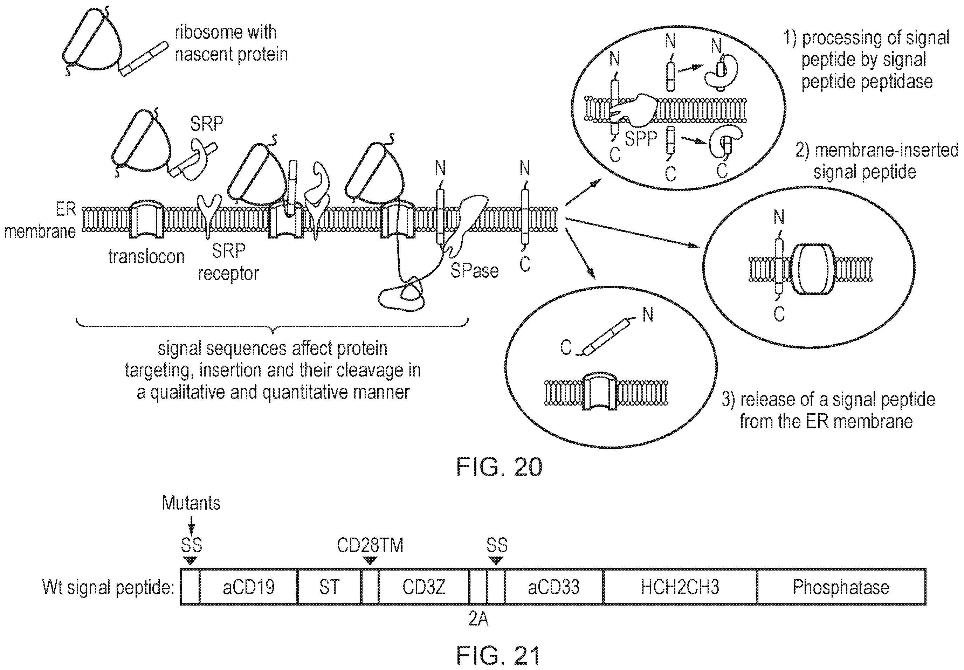

**Figure 20:** Schematic diagram illustrating the function of signal sequences in protein targeting

**Figure 21:** Schematic diagram of nucleic acid construct encoding two CARs

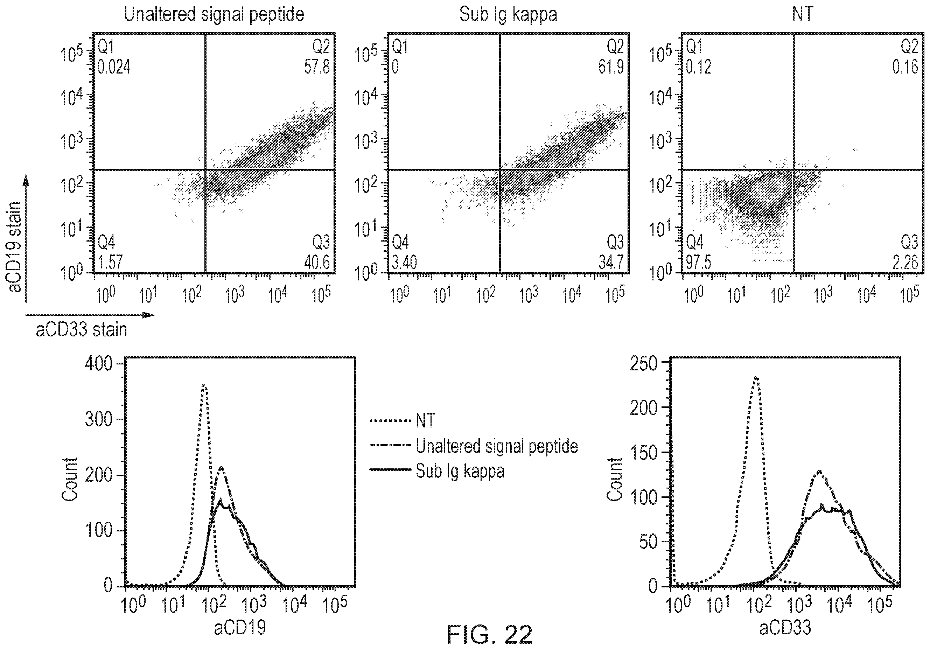

**Figure 22**: Verifying the function of a substituted signal sequence.

PCT/GB2014/053452 describes vector system encoding two chimeric antigen receptors (CARs), one against CD19 and one against CD33. The signal peptide used for the CARs in that study was the signal peptide from the human CD8a signal sequence. For the purposes of this study, this was substituted with the signal peptide from the murine Ig kappa chain V-III region, which has the sequence: METD**TLILWVLLLLV**PGSTG (hydrophobic residues hightlited in bold). In order to establish that the murine Ig kappa chain V-III signal sequence functioned as well as the signal sequence from human CD8a, a comparative study was performed. For both signal sequences, functional expression of the anti-CD33 CAR and the anti-CD19 CAR was observed.

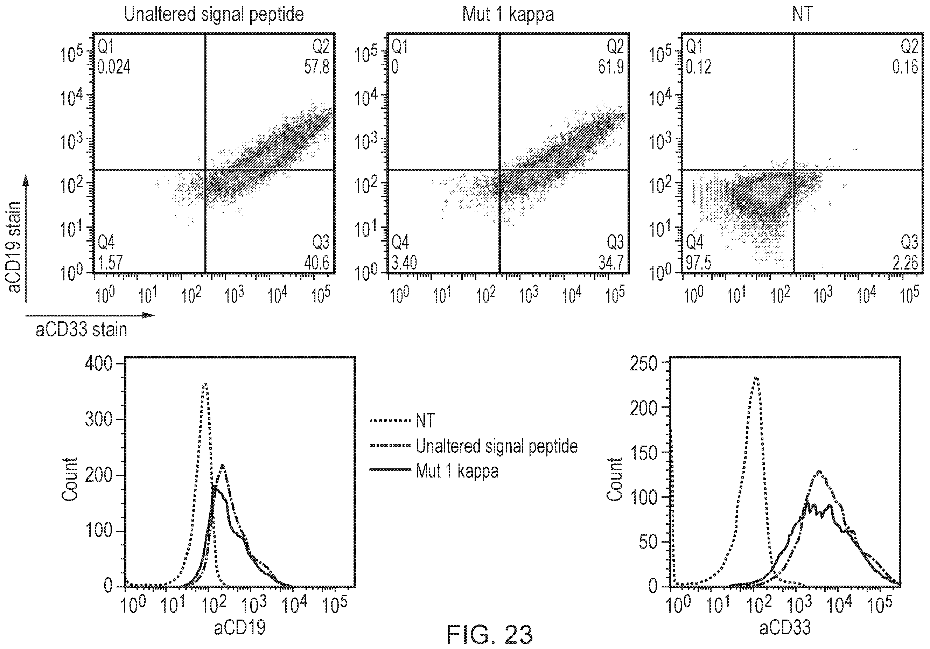

**Figure 23:** Testing the effect of one amino acid deletion in the murine Ig kappa chain V-III. Mutant 1 kappa chain was created with the following deletion (shown in grey) in the h-region METD**TLILWVLLLLVP**GSTG and the relative expression on the anti-CD33 CAR and the anti-CD19 CAR was observed.

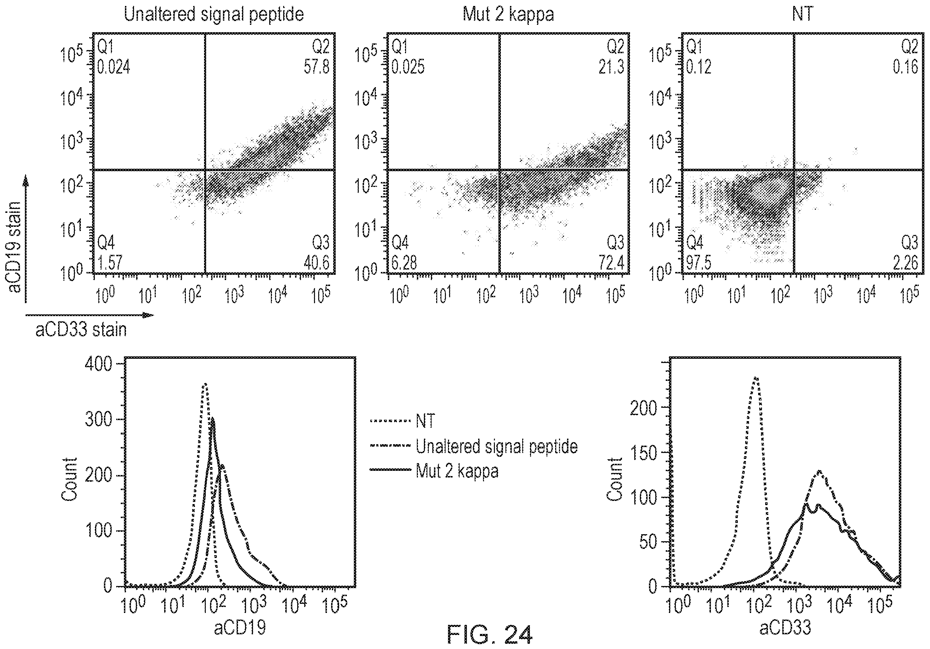

**Figure 24:** Testing the effect of two amino acid deletions in the murine Ig kappa chain V-III. Mutant 2 kappa chain was created with the following deletions (shown in grey) in the h-region METDTL**ILWVLLL**LVPGSTG and the relative expression on the anti-CD33 CAR and the anti-CD19 CAR was observed.

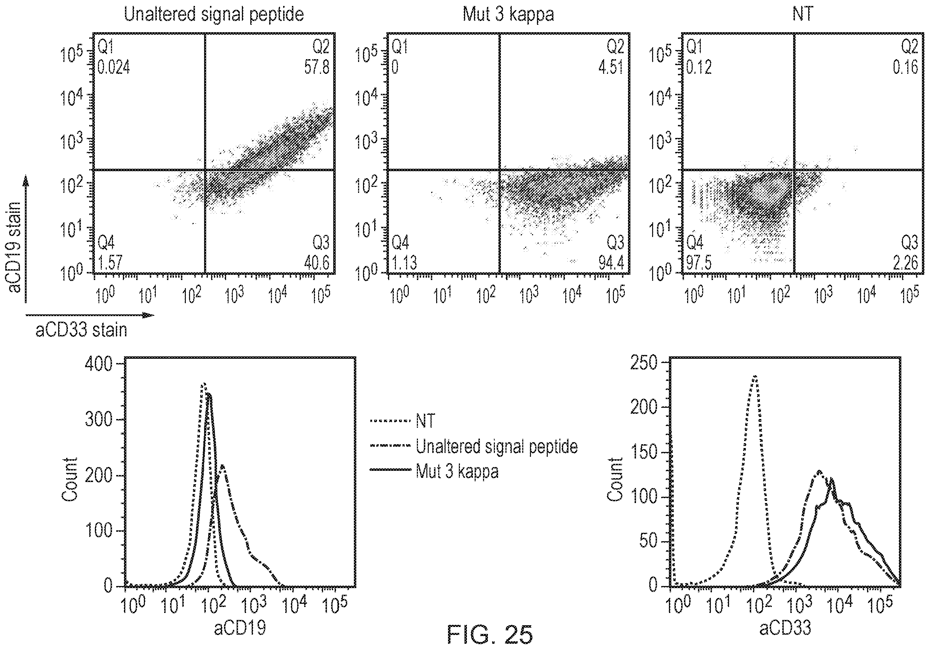

**Figure 25:** Testing the effect of three amino acid deletions in the murine Ig kappa chain V-III. Mutant 2 kappa chain was created with the following deletions (shown in grey) in the h-region METD**TL**ILW**VLLL**LVPGSTG and the relative expression on the anti-CD33 CAR and the anti-CD19 CAR was observed.

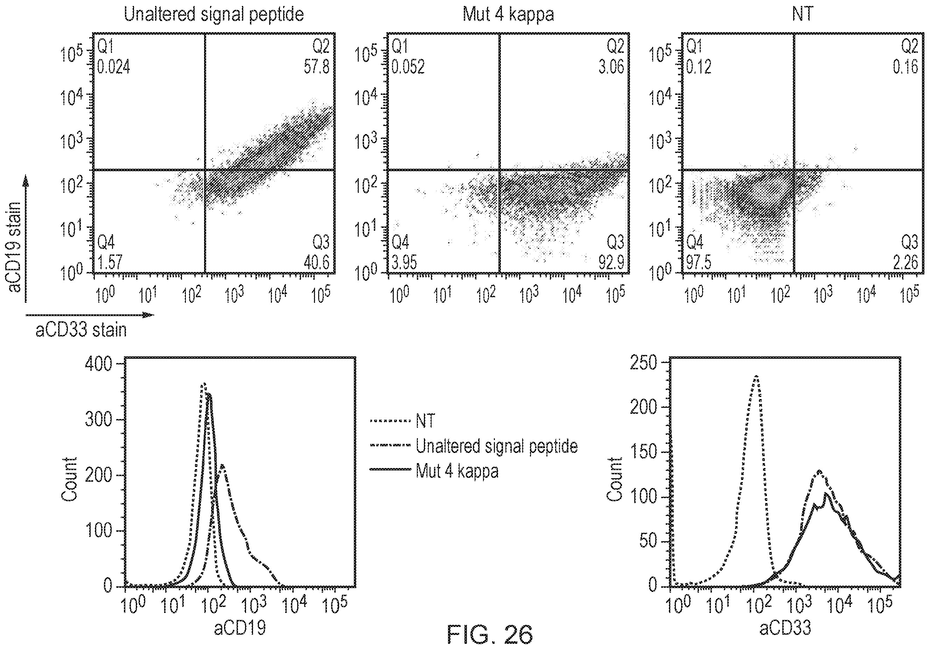

**Figure 26:** Testing the effect of five amino acid deletions in the murine Ig kappa chain V-III. Mutant 2 kappa chain was created with the following deletions (shown in grey) in the h-region METD**TL**ILW**VLLL**LVPGSTG and the relative expression on the anti-CD33 CAR and the anti-CD19 CAR was observed.

SUMMARY OF ASPECTS OF THE INVENTION

The present inventors have developed a panel of "logic-gated" chimeric antigen receptor pairs which, when expressed by a cell, such as a T cell, are capable of detecting a particular pattern of expression of at least two target antigens. If the at least two target antigens are arbitrarily denoted as antigen A and antigen B, the three possible options are as follows:

"OR GATE" - T cell triggers when either antigen A or antigen B is present on the target cell
"AND GATE" - T cell triggers only when both antigens A and B are present on the target cell
"AND NOT GATE" - T cell triggers if antigen A is present alone on the target cell, but not if both antigens A and B are present on the target cell

Engineered T cells expressing these CAR combinations can be tailored to be exquisitely specific for cancer cells, based on their particular expression (or lack of expression) of two or more markers.
The present inventors have also found that, when a CAR is co-expressed with a second CAR as a polyprotein which after translation is subsequently cleaved to separate the two CARs, it is possible to modulate the relative cell surface expression of the two CARs by reducing trafficking to the cell surface of one or both CAR(s) and/or by reducing its half-life at the cell surface. This need not be limited to a pair of CARs, but may be used to allow control of the relative expression of multiple proteins initially translated as a polyprotein.
As used herein, 'polyprotein' refers to a polypeptide sequence translated from a single nucleic acid construct as a single entity, but which comprises polypeptide sequences which are subsequently separated and which function as discrete entities (e.g. separate CARs).
The present invention relates to an AND logic gate, in which the relative expression of the two CARs is modulated.
Thus in a first aspect, the present invention provides a nucleic acid construct comprising the following structure:

A-X-B

in which

X is a nucleic acid sequence which encodes a cleavage site; and
A and B are nucleic acid sequences encoding a first and a second chimeric antigen receptor (CAR), each CAR comprising:

(i) an antigen-binding domain;
(ii) a spacer
(iii) a trans-membrane domain; and
(iv) an endodomain

wherein the antigen binding domains of the first and second CARs bind to different antigens, wherein the spacer of the first CAR has a different size to the spacer of the second CAR such that when the first CAR and the second CAR bind their respective target antigens at the cell surface, the first and the second CAR become specially separated on the cell membrane; wherein one of the first or second CARs is an activating CAR comprising an activating endodomain and the other CAR is an inhibitory CAR comprising a ligation-off inhibitory endodomain which inhibitory CAR inhibits T-cell activation by the activating CAR in the absence of inhibitory CAR ligation, but does not significantly inhibit T-cell activation by the activating CAR when the inhibitory CAR is ligated;
and wherein:

(a) the first and/or second CAR comprises an intracellular retention signal; and/or
(b) the signal peptide of the first or second CAR comprises one or more mutation(s) such that it has fewer hydrophobic amino acids.

For (b) the mutated signal peptide may have fewer hydrophobic amino acids than the "wild-type" signal peptide sequence from which it is derived. The mutated signal peptide may have fewer hydrophobic amino acids than the signal peptide of the other CAR.
The spacer of the first CAR may have a different length and/or charge and/or shape and/or configuration and/or glycosylation to the spacer of the second CAR, such that when the first CAR and the second CAR bind their respective target antigens, the first CAR and second CAR become spatially separated on the T cell. Ligation of the first and second CARs to their respective antigens causes them to be compartmentalized together or separately in the immunological synapse resulting in control of activation. This may be understood when one considers the kinetic separation model of T-cell activation (see below).

The first spacer or the second spacer may comprise a CD8 stalk and the other spacer may comprise the hinge, CH2 and CH3 domain of an IgG1.

In the present invention, which relates to the "AND" gate, one of the first or second CARs is an activating CAR comprising an activating endodomain, and the other CAR is a "ligation-off" inhibitory CAR comprising an inhibitory endodomain. The ligation-off inhibitory CAR inhibits T-cell activation by the activating CAR in the absence of inhibitory CAR ligation, but does not significantly inhibit T-cell activation by the activating CAR when the inhibitory CAR is ligated. Since the spacer of the first CAR has a different length and/or charge and/or shape and/or configuration and/or glycosylation from the spacer of the second CAR, when both CARs are ligated they segregate. This causes the inhibitory CAR to be spatially separated from the activating CAR, so that T cell activation can occur. T cell activation therefore only occurs in response to a target cell bearing both cognate antigens.

The inhibitory endodomain may comprise all or part of the endodomain from a receptor-like tyrosine phosphatase, such as CD148 or CD45.

The antigen-binding domain of the first CAR may bind CD5 and the antigen-binding domain of the second CAR may bind CD19. This is of use in targeting chronic lymphocytic leukaemia (CLL). This disease can be treated by targeting CD19 alone, but at the cost of depleting the entire B-cell compartment. CLL cells are unusual in that they co-express CD5 and CD19. Targeting this pair of antigens with an AND gate will increase specificity and reduce toxicity.

Described herein is a method for modulating the relative expression of two (or more) CARs in an AND logic gate, by one or both of the following approaches (a) by incorporation of an intracellular retention signal in one or both CAR(s) and (b) by altering the signal peptide of one CAR in order to remove or replace hydrophobic amino acids.

In particular, the relative expression level of the activating CAR, in relation to the inhibitory CAR, may be reduced by one of the above-mentioned approaches.

For approach (a), the endodomain of the CAR may comprise the intracellular retention signal.

The intracellular retention signal may direct the CAR away from the secretory pathway and/or to a membrane-bound intracellular compartment such as a lysozomal, endosomal or Golgi compartment.

The intracellular retention signal may, for example, be a tyrosine-based sorting signal, a dileucine-based sorting signal, an acidic cluster signal, a lysosomal avoidance signal, an NPFX'(1,2)D-Type signal, a KDEL, a KKX'X' or a KX'KX'X' signal (wherein X' is any amino acid).

The intracellular retention signal may comprise a sequence selected from the group of: NPX'Y, YX'X'Z', [DE]X'X'X'L[LI], DX'X'LL, DP[FW], FX'DX'F, NPF, LZX'Z[DE], LLDLL, PWDLW, KDEL, KKX'X' or KX'KX'X';
wherein X' is any amino acid and Z' is an amino acid with a bulky hydrophobic side chain.

The intracellular retention signal may comprise any of the sequences shown in Tables 1 to 5.

The intracellular retention signal may comprise the Tyrosinase-related protein (TYRP)-1 intracellular retention signal. The intracellular retention signal may comprise the TYRP-1 intracellular domain. The intracellular retention signal may comprise the sequence NQPLLTD (SEQ ID No. 1).

The intracellular retention signal may comprise the Adenoviral E3/19K intracellular retention signal. The intracellular retention signal may comprise the E3/19K cytosolic domain. The intracellular retention signal may comprise the sequence KYKSRRSFIDEKKMP (SEQ ID No. 2); or DEKKMP (SEQ ID No. 3).

The intracellular retention signal may be proximal or distal to a transmembrane domain of the CAR.

For approach (b), the signal peptides of the first and second CAR are different. They may differ in their number of hydrophobic amino acids. One signal peptide may comprise one or more mutation(s) such that it has fewer hydrophobic amino acids either a) than the wild-type sequence from which it was derived; or b) than the other signal peptide.

In particular the signal peptide of the activating CAR may be altered to remove or replace hydrophobic amino acids, such that the relative expression of the activating CAR, relative to the inhibitory CAR, is reduced at the cell surface.

The signal peptides may be different and the sequence of one signal peptide may be altered such that the hydrophobic amino acids are removed or replaced. Alternatively, the first signal peptide and the second signal peptide may be derivable from the same sequence, but one signal peptide may comprise one or more amino acid deletions/substitutions to remove/replace one or more hydrophobic amino acids compared to the other signal peptide.

The hydrophobic amino acid(s) removed or replaced may be selected from the group: Alanine (A); Valine (V); Isoleucine (I); Leucine (L); Methionine (M); Phenylalanine (P); Tyrosine (Y); Tryptophan (W) or the group Valine (V); Isoleucine (I); Leucine (L); and Tryptophan (W).

For approach (b) one signal peptide may comprise one, two, three, four or five mutations, such that it has one, two, three, four or five fewer hydrophobic amino acids than: the wild-type signal sequence from which it is derived and/or the other signal peptide.

In the nucleic acid construct of the present invention, the nucleic acid sequence of X may be a nucleic acid sequence encoding a self-cleaving peptide, a furin cleavage site or a Tobacco Etch Virus cleavage site.

The nucleic acid sequence of X may be a nucleic acid sequence encoding a 2A self-cleaving peptide from an aphtho- or a cardiovirus or a 2A-like peptide.

The nucleic acid construct may comprise a third nucleic acid sequence encoding a protein of interest (POI). The POI may be a transmembrane protein.

The POI may be selected from a list of: excitatory receptors such as 41BB, OX40, CD27, CD28 and related molecules; or inhibitory receptors such as PD1, CTLA4, LAIR1, CD22 and related molecules; or cytokine receptor molecules such as IL1R, IL2R, IL7R, IL15R and related molcules; or homing molecules such as N-CAM, V-CAM, L1-CAM, LFA-1, CDH1-3, Selectins or Integrins. The POI may be a third CAR.

The POI may be a synthetic protein such as a suicide gene or a marker gene.

The amount of a CAR which comprises an intracellular retention signal and/or which has an altered signal peptide which is expressed at the cell surface may be, for example, less than 90%, 70%, 50% or 30% compared to a CAR expressed from the same nucleic acid construct which does not comprise an intracellular retention signal and which has an unaltered signal peptide.

The nucleic acid construct may also encode a further polypeptide, for example a polypeptide which enables selection of transduced cells and/or enables cells expressing the polypeptide to be deleted.

Alternative codons may be used in regions of sequence encoding the same or similar amino acid sequences, in order to avoid homologous recombination.

In a second aspect the present invention provides a vector comprising a nucleic acid construct according to the first aspect of the invention.

The vector may be a retroviral vector or a lentiviral vector or a transposon.

In a third aspect the present invention provides a cell comprising a nucleic acid construct according to the first aspect of the invention or a vector according to the second aspect of the invention.

The cell may be an immune cell such as a T cell or a natural killer (NK) cell.

In a fourth aspect, the present invention provides a method for making a cell according to the third aspect of the invention, which comprises the step of introducing: a nucleic acid construct according to the first aspect of the invention or a vector according to the second aspect of the invention, into a cell ex-vivo.

The cell may be part of or derived from a sample isolated from a subject.

The cell used in the method of the fourth aspect of the invention may be from a sample isolated from a patient, a related or unrelated haematopoietic transplant donor, a completely unconnected donor, from cord blood, differentiated from an embryonic cell line, differentiated from an inducible progenitor cell line, or derived from a transformed cell line.

In a fifth aspect the present invention provides a pharmaceutical composition comprising a plurality of cells according to the fourth aspect of the invention. The composition may be an autologous T and/or NK cell composition.

The present disclosure relates to a method for treating and/or preventing a disease, which comprises the step of administering a pharmaceutical composition according to the fifth aspect of the invention to a subject.

The method may comprise the following steps:

> (i) isolation of a T and/or NK cell-containing sample from a subject;
> (ii) transduction or transfection of the T and/or NK cells with: a nucleic acid construct according to the first aspect of the invention or a vector according to the second aspect of the invention; and
> (iii) administering the T and/or NK cells from (ii) to a the subject.

There is also provided a pharmaceutical composition according to the fifth aspect of the invention for use in treating and/or preventing a disease.

Described herein is the use of a cell according to the third aspect of the invention in the manufacture of a medicament for treating and/or preventing a disease.

Also described herein is a method for modulating the relative cell surface expression of an activating CAR expressed from a single nucleic acid construct with a ligation-off inhibitory CAR by (a) including an intracellular retention signal in the nucleic acid sequence which encodes the activating CAR(s) and/or (b) altering the nucleic acid sequence which encodes the signal peptide of the activating CAR in order to remove or replace one or more hydrophobic amino acids in comparison with the signal peptide of the ligation-off inhibitory CAR. The nucleic acid construct may be as defined in the first aspect of the invention.

The kinetic-segregation based AND gate of the present invention offers a significant technical advantage to the previously described "co-CAR", i.e. the dual targeting approach in which two antigens are recognized by two CARs which supply either an activating or a co-stimulating signal to the T-cell.

With the co-CAR approach, although greatest activity might be expected against target cells bearing both antigens, considerable activity against tissues bearing only antigen recognized by the activating CAR can be expected. This activity can be expected to be at least that of a first-generation CAR. First generation CARs have resulted in considerable toxicity: for instance biliary toxicity was observed in clinical testing of a first generation CAR recognizing Carbonic anhydrase IX which was unexpectedly expressed on biliary epithelium (Rotterdam ref). Notably, terminally differentiated effectors do not require or respond to co-stimulatory signals, so any terminally differentiated CAR T-cells would act maximally despite the absence of a co-stimulatory CAR signal.

Further, co-stimulatory signals lead to long-lasting effects on the T-cell population. These effects long outlast the T-cell / target synapse interaction. Consequently, CAR T-cells which become fully activated within the tumour and migrate could have maximally potent activity against single-antigen bearing normal tissues. This "spill-over" effect may be most pronounced in tissues within, near or which drain from the tumour. In fact, strategies based on the concept of the activity of a first generation CAR being enhanced by co-stimulatory signals engaged not CAR activation but through a distinct receptor, have been proposed and tested (Rossig, Blood. 2002 Mar 15;99(6):2009-16.).

The co-CAR approach hence can be expected to result at best to a reduction but not abolition of toxicity towards single antigen expressing normal tissue. The present invention uses kinetic segregation at the immunological synapse formed between the T-cell / target cell to regulate T-cell triggering itself. Consequently tight absolute control of triggering in the absence of the second antigen is achieved. Hence the totality of T-cell activation is restricted to target cells expressing both antigens, the AND gate should function irrespective of the effector cell type or differentiation state, and no "spill-over" effect AND gate T-cell activation is possible.

Moreover the capacity to modulate the relative expression of the two CARs, provided by the present invention, brings further advantages to the AND gate. When an AND gate system is working correctly, the ligation-off inhibitory CAR inhibits T-cell activation by the activating CAR in the absence of inhibitory CAR ligation, but does not significantly inhibit T-cell activation by the activating CAR when the inhibitory CAR is ligated. However, in some systems the activating CAR is "overactive" leading to a high level of background i.e. activation by the activating CAR in the absence of inhibitory CAR ligation. The present inventors have found that, by down regulating the relative expression of the activating CAR in comparison to the inhibitory CAR, it is possible to "tighten up" the system and reduce the level of background activation in the absence of the second antigen.

The inclusion of an intracellular retention signal in a CAR, or the alteration of the signal peptide of the CAR to reduce the number of hydrophobic amino acids, reduces the amount of the CAR expressed on the cell surface. As such, the relative expression level of two CARs expressed from a single construct can be modulated. As a CAR is only active at the cell surface, reducing the relative cell surface expression of the CAR also reduces its relative activity.

Thus the present invention provides a nucleic acid construct encoding an AND gate, in which the relative level of expression of the two or more CARs may be finely tuned, either to reduce the expression of the activating CAR (as described above) or to mirror the relative level of expression of the respective antigens on the target cell.

This invention can be extended to modulate the relative expression of three or more proteins expressed as a concatenated polypeptide, separated by cleavage sites and relative surface expression dictated by retention signals or signal peptides of differing activity.

DETAILED DESCRIPTION

CHIMERIC ANTIGEN RECEPTORS (CARs)

The present invention provides a nucleic acid construct which expresses two or more chimeric antigen receptors (CARs).

CARs, which are shown schematically in Figure 1, are chimeric type I trans-membrane proteins which connect an extracellular antigen-recognizing domain (binder) to an intracellular signalling domain (endodomain). The binder is typically a single-chain variable fragment (scFv) derived from a monoclonal antibody (mAb), but it can be based on other formats which comprise an antibody-like antigen binding site. A spacer domain is usually necessary to isolate the binder from the membrane and to allow it a suitable orientation. A common spacer domain used is the Fc of IgG1. More compact spacers can suffice e.g. the stalk from CD8$\alpha$ and even just the IgG1 hinge alone, depending on the antigen. A trans-membrane domain anchors the protein in the cell membrane and connects the spacer to the endodomain.

Early CAR designs had endodomains derived from the intracellular parts of either the $\gamma$ chain of the Fc$\varepsilon$R1 or CD3$\zeta$. Consequently, these first generation receptors transmitted immunological signal 1, which was sufficient to trigger T-cell killing of cognate target cells but failed to fully activate the T-cell to proliferate and survive. To overcome this limitation, compound endodomains have been constructed: fusion of the intracellular part of a T-cell co-stimulatory molecule to that of CD3$\zeta$ results in second generation receptors which can transmit an activating and co-stimulatory signal simultaneously after antigen recognition. The co-stimulatory domain most commonly used is that of CD28. This supplies the most potent co-stimulatory signal - namely immunological signal 2, which triggers T-cell proliferation. Some receptors have also been described which include TNF receptor family endodomains, such as the closely related OX40 and 41BB which transmit survival signals. Even more potent third generation CARs have now been described which have endodomains capable of transmitting activation, proliferation and survival signals.

CAR-encoding nucleic acids may be transferred to T cells using, for example, retroviral vectors. Lentiviral vectors may be employed. In this way, a large number of cancer-specific T cells can be generated for adoptive cell transfer. When the CAR binds the target-antigen, this results in the transmission of an activating signal to the T-cell it is expressed on. Thus the CAR directs the specificity and cytotoxicity of the T cell towards tumour cells expressing the targeted antigen.

The inventors have defined three distinct categories of cells which co-express a first CAR and a second CAR such that the cell can recognize a desired pattern of expression on target cells in the manner of a logic gate as detailed in the truth tables: Table 1, 2 and 3.

Both the first and second (and optionally subsequent) CARs comprise:

(i) an antigen-binding domain;
(ii) a spacer;
(iii) a transmembrane domain; and
(iii) an intracellular domain, which may comprise or associate with a T-cell signalling endodomain.

Table 1: Truth Table for CAR OR GATE

| Antigen A | Antigen B | Response |
|-----------|-----------|---------------|
| Absent | Absent | No activation |
| Absent | Present | Activation |
| Present | Absent | Activation |
| Present | Present | Activation |

Table 2: Truth Table for CAR AND GATE

| Antigen A | Antigen B | Response |
|-----------|-----------|---------------|
| Absent | Absent | No activation |
| Absent | Present | No Activation |
| Present | Absent | No Activation |
| Present | Present | Activation |

Table 3: Truth Table for CAR AND NOT GATE

| Antigen A | Antigen B | Response |
|-----------|-----------|---------------|
| Absent | Absent | No activation |
| Absent | Present | No Activation |
| Present | Absent | Activation |
| Present | Present | No Activation |

Using the nucleic acid construct of the present invention, the first and second CARs are produced as a polypeptide comprising both CARs, together with a cleavage site.

SEQ ID No. 4 and 5 are examples of AND gates, which comprise two CARs. The nucleic acid construct of the invention may comprise one or other part of the following amino acid sequences, which corresponds to a single CAR. One or both CAR sequences may be modified to include one or more intracellular retention signals, and/or to alter their signal peptides, as defined below.

SEQ ID No 4 is a CAR AND gate which recognizes CD19 AND CD33 using a CD148 phosphatase
SEQ ID No 5 is an alternative implementation of the CAR AND GATE which recognizes CD19 AND CD33 which uses a CD45 phosphatase.

SEQ ID No. 4

MSLPVTALLLPLALLLHAARPDIQMTQTTSSLSASLGDRVTISCRASQDISKYLNWYQQKPD
GTVKLLIYHTSRLHSGVPSRFSGSGSGTDYSLTISNLEQEDIATYFCQQGNTLPYTFGGGTK
LEITKAGGGGSGGGGSGGGGSGGGGSEVKLQESGPGLVAPSQSLSVTCTVSGVSLPDYG
VSWIRQPPRKGLEWLGVIWGSETTYYNSALKSRLTIIKDNSKSQVFLKMNSLQTDDTAIYYC
AKHYYYGGSYAMDYWGQGTSVTVSSDPTTTPAPRPPTPAPTIASQPLSLRPEACRPAAGG
AVHTRGLDFACDIFWVLVVVGGVLACYSLLVTVAFIIFWVRRVKFSRSADAPAYQQGQNQL
YNELNLGRREEYDVLDKRRGRDPEMGGKPRRKNPQEGLYNELQKDKMAEAYSEIGMKGE
RRRGKGHDGLYQGLSTATKDTYDALHMQALPPRRAEGRGSLLTCGDVEENPGPMAVPTQ
VLGLLLLWLTDARCDIQMTQSPSSLSASVGDRVTITCRASEDIYFNLVWYQQKPGKAPKLLI
YDTNRLADGVPSRFSGSGSGTQYTLTISSLQPEDFATYYCQHYKNYPLTFGQGTKLEIKRS
GGGGSGGGGSGGGGSGGGGSRSEVQLVESGGGLVQPGGSLRLSCAASGFTLSNYGMH
WIRQAPGKGLEWVSSISLNGGSTYYRDSVKGRFTISRDNAKSTLYLQMNSLRAEDTAVYYC
AAQDAYTGGYFDYWGQGTLVTVSSMDPAEPKSPDKTHTCPPCPAPPVAGPSVFLFPPKPK
DTLMIARTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHE
ALHNHYTQKSLSLSPGKKDPKAVFGCIFGALVIVTVGGFIFWRKKRKDAKNNEVSFSQIKPK
KSKLIRVENFEAYFKKQQADSNCGFAEEYEDLKLVGISQPKYAAELAENRGKNRYNNVLPY
DISRVKLSVQTHSTDDYINANYMPGYHSKKDFIATQGPLPNTLKDFWRMVWEKNVYAIIMLT
KCVEQGRTKCEEYWPSKQAQDYGDITVAMTSEIVLPEWTIRDFTVKNIQTSESHPLRQFHF
TSWPDHGVPDTTDLLINFRYLVRDYMKQSPPESPILVHCSAGVGRTGTFIAIDRLIYQIENEN
TVDVYGIVYDLRMHRPLMVQTEDQYVFLNQCVLDIVRSQKDSKVDLIYQNTTAMTIYENLAP
VTTFGKTNGYIA

SEQ ID No. 4 breaks down as follows:

**Signal peptide derived from Human CD8a:**
MSLPVTALLLPLALLLHAARP
**scFv aCD19:**

DIQMTQTTSSLSASLGDRVTISCRASQDISKYLNWYQQKPDGTVKLLIYHTSRLHSGVPSRF
SGSGSGTDYSLTISNLEQEDIATYFCQQGNTLPYTFGGGTKLEITKAGGGGSGGGGSGGG

GSGGGGSEVKLQESGPGLVAPSQSLSVTCTVSGVSLPDYGVSWIRQPPRKGLEWLGVIW
GSETTYYNSALKSRLTIIKDNSKSQVFLKMNSLQTDDTAIYYCAKHYYYGGSYAMDYWGQG

TSVTVS

**Linker:**
SD

**Human CD8aSTK:**
PTTTPAPRPPTPAPTIASQPLSLRPEACRPAAGGAVHTRGLDFACDI
**Human CD28TM:**
FVWLVWGGVLACYSLLVTVAFIIFWV
**Human CD3zeta intracellular domain:**

RRVKFSRSADAPAYQQGQNQLYNELNLGRREEYDVLDKRRGRDPEMGGKPRRKNPQEG

LYNELQKDKMAEAYSEIGMKGERRRGKGHDGLYQGLSTATKDTYDALHMQALPPR

**2A peptide:**
RAEGRGSLLTCGDVEENPGP
**Signal peptide derived from mouse Ig kappa:**
MAVPTQVLGLLLLWLTDA
**scFv aCD33:**

RCDIQMTQSPSSLSASVGDRVTITCRASEDIYFNLVWYQQKPGKAPKLLIYDTNRLADGVPS

RFSGSGSGTQYTLTISSLQPEDFATYYCQHYKNYPLTFGQGTKLEIKRSGGGGSGGGGSG

GGGSGGGGSRSEVQLVESGGGLVQPGGSLRLSCAASGFTLSNYGMHWIRQAPGKGLEW

VSSISLNGGSTYYRDSVKGRFTISRDNAKSTLYLQMNSLRAEDTAVYYCAAQDAYTGGYFD

YWGQGTLVTVSSM

**Linker:**
DPA
**Hinge and Fc derived from human IgG1 with mutations to prevent FcRg association (HCH2CH3pvaa):**

EPKSPDKTHTCPPCPAPPVAGPSVFLFPPKPKDTLMIARTPEVTCVVVDVSHEDPEVKFNW

YVDGVEVHNAKTKPREEQYNSTYRVVSVLTVLHQDWLNGKEYKCKVSNKALPAPIEKTISK

AKGQPREPQVYTLPPSRDELTKNQVSLTCLVKGFYPSDIAVEWESNGQPENNYKTTPPVLD

SDGSFFLYSKLTVDKSRWQQGNVFSCSVMHEALHNHYTQKSLSLSPGK

**Linker:**
KDPK
**Human CD148TM:**
AVFGCIFGALVIVTVGGFIFW
**Human CD148 intracellular domain:**

RKKRKDAKNNEVSFSQIKPKKSKLIRVENFEAYFKKQQADSNCGFAEEYEDLKLVGISQPKY

AAELAENRGKNRYNNVLPYDISRVKLSVQTHSTDDYINANYMPGYHSKKDFIATQGPLPNTL

KDFWRMVWEKNVYAIIMLTKCVEQGRTKCEEYWPSKQAQDYGDITVAMTSEIVLPEWTIRD

FTVKNIQTSESHPLRQFHFTSWPDHGVPDTTDLLINFRYLVRDYMKQSPPESPILVHCSAGV

GRTGTFIAIDRLIYQIENENTVDVYGIVYDLRMHRPLMVQTEDQYVFLNQCVLDIVRSQKDSK

VDLIYQNTTAMTIYENLAPVTTFGKTNGYIA

SEQ ID No. 5

MSLPVTALLLPLALLLHAARPDIQMTQTTSSLSASLGDRVTISCRASQDISKYLNWYQQKPD
GTVKLLIYHTSRLHSGVPSRFSGSGSGTDYSLTISNLEQEDIATYFCQQGNTLPYTFGGGTK
LEITKAGGGGSGGGGSGGGGSGGGGSEVKLQESGPGLVAPSQSLSVTCTVSGVSLPDYG
VSWIRQPPRKGLEWLGVIWGSETTYYNSALKSRLTIIKDNSKSQVFLKMNSLQTDDTAIYYC
AKHYYYGGSYAMDYWGQGTSVTVSSDPTTTPAPRPPTPAPTIASQPLSLRPEACRPAAGG
AVHTRGLDFACDIFWVLVVVGGVLACYSLLVTVAFIIFWVRRVKFSRSADAPAYQQGQNQL
YNELNLGRREEYDVLDKRRGRDPEMGGKPRRKNPQEGLYNELQKDKMAEAYSEIGMKGE
RRRGKGHDGLYQGLSTATKDTYDALHMQALPPRRAEGRGSLLTCGDVEENPGPMAVPTQ
VLGLLLLWLTDARCDIQMTQSPSSLSASVGDRVTITCRASEDIYFNLVWYQQKPGKAPKLLI
YDTNRLADGVPSRFSGSGSGTQYTLTISSLQPEDFATYYCQHYKNYPLTFGQGTKLEIKRS
GGGGSGGGGSGGGGSGGGGSRSEVQLVESGGGLVQPGGSLRLSCAASGFTLSNYGMH
WIRQAPGKGLEWVSSISLNGGSTYYRDSVKGRFTISRDNAKSTLYLQMNSLRAEDTAVYYC
AAQDAYTGGYFDYWGQGTLVTVSSMDPAEPKSPDKTHTCPPCPAPPVAGPSVFLFPPKPK
DTLMIARTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHE
ALHNHYTQKSLSLSPGKKDPKALIAFLAFLIIVTSIALLVVLYKIYDLHKKRSCNLDEQQELVER
DDEKQLMNVEPIHADILLETYKRKIADEGRLFLAEFQSIPRVFSKFPIKEARKPFNQNKNRYV
DILPYDYNRVELSEINGDAGSNYINASYIDGFKEPRKYIAAQGPRDETVDDFWRMIWEQKAT
VIVMVTRCEEGNRNKCAEYWPSMEEGTRAFGDVVVKINQHKRCPDYIIQKLNIVNKKEKAT
GREVTHIQFTSWPDHGVPEDPHLLLKLRRRVNAFSNFFSGPIVVHCSAGVGRTGTYIGIDA
MLEGLEAENKVDVYGYVVKLRRQRCLMVQVEAQYILIHQALVEYNQFGETEVNLSELHPYL
HNMKKRDPPSEPSPLEAEFQRLPSYRSWRTQHIGNQEENKSKNRNSNVIPYDYNRVPLKH
ELEMSKESEHDSDESSDDDSDSEEPSKYINASFIMSYWKPEVMIAAQGPLKETIGDFWQMI
FQRKVKVIVMLTELKHGDQEICAQYWGEGKQTYGDIEVDLKDTDKSSTYTLRVFELRHSKR
KDSRTVYQYQYTNWSVEQLPAEPKELISMIQVVKQKLPQKNSSEGNKHHKSTPLLIHCRDG
SQQTGIFCALLNLLESAETEEVVDIFQVVKALRKARPGMVSTFEQYQFLYDVIASTYPAQNG
QVKKNNHQEDKIEFDNEVDKVKQDANCVNPLGAPEKLPEAKEQAEGSEPTSGTEGPEHSV
NGPASPALNQGS

SEQ ID No. 5 breaks down as follows:

**Signal peptide derived from Human CD8a:**
MSLPVTALLLPLALLLHAARP
**scFv aCD19:**

DIQMTQTTSSLSASLGDRVTISCRASQDISKYLNWYQQKPDGTVKLLIYHTSRLHSGVPSRF
SGSGSGTDYSLTISNLEQEDIATYFCQQGNTLPYTFGGGTKLEITKAGGGGSGGGGSGGGG
GSGGGGSEVKLQESGPGLVAPSQSLSVTCTVSGVSLPDYGVSWIRQPPRKGLEWLGVIW
GSETTYYNSALKSRLTIIKDNSKSQVFLKMNSLQTDDTAIYYCAKHYYYGGSYAMDYWGQG
TSVTVS

**Linker:**
SD
**Human CD8aSTK:**
PTTTPAPRPPTPAPTIASQPLSLRPEACRPAAGGAVHTRGLDFACDI
**Human CD28TM:**
FWVLVVVGGVLACYSLLVTVAFIIFWV
**Human CD3zeta intracellular domain:**

RRVKFSRSADAPAYQQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPR

**2A peptide:**
RAEGRGSLLTCGDVEENPGP
**Signal peptide derived from mouse Ig kappa:**
MAVPTQVLGLLLLWLTDA
**scFv aCD33:**

RCDIQMTQSPSSLSASVGDRVTITCRASEDIYFNLVWYQQKPGKAPKLLIYDTNRLADGVPS
RFSGSGSGTQYTLTISSLQPEDFATYYCQHYKNYPLTFGQGTKLEIKRSGGGGSGGGGSG
GGGSGGGGSRSEVQLVESGGGLVQPGGSLRLSCAASGFTLSNYGMHWIRQAPGKGLEW
VSSISLNGGSTYYRDSVKGRFTISRDNAKSTLYLQMNSLRAEDTAVYYCAAQDAYTGGYFD
YWGQGTLVTVSSM

**Linker:**
DPA
**Hinge and Fc derived from human IgG1 with mutations to prevent FcRg association (HCH2CH3pvaa):**

EPKSPDKTHTCPPCPAPPVAGPSVFLFPPKPKDTLMIARTPEVTCVVVDVSHEDPEVKFNW
YVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK

**Linker:**
KDPK
**Human CD45TM:**
ALIAFLAFLIIVTSIALLVVLY
**Human CD45 intracellular domain:**

KIYDLHKKRSCNLDEQQELVERDDEKQLMNVEPIHADILLETYKRKIADEGRLFLAEFQSIPR

VFSKFPIKEARKPFNQNKNRYVDILPYDYNRVELSEINGDAGSNYINASYIDGFKEPRKYIAA

QGPRDETVDDFWRMIWEQKATVIVMVTRCEEGNRNKCAEYWPSMEEGTRAFGDVVVKIN

QHKRCPDYIIQKLNIVNKKEKATGREVTHIQFTSWPDHGVPEDPHLLLKLRRRVNAFSNFFS

GPIVVHCSAGVGRTGTYIGIDAMLEGLEAENKVDVYGYVVKLRRQRCLMVQVEAQYILIHQA

LVEYNQFGETEVNLSELHPYLHNMKKRDPPSEPSPLEAEFQRLPSYRSWRTQHIGNQEEN

KSKNRNSNVIPYDYNRVPLKHELEMSKESEHDSDESSDDDSDSEEPSKYINASFIMSYWKP

EVMIAAQGPLKETIGDFWQMIFQRKVKVIVMLTELKHGDQEICAQYWGEGKQTYGDIEVDL

KDTDKSSTYTLRVFELRHSKRKDSRTVYQYQYTNWSVEQLPAEPKELISMIQVVKQKLPQK

NSSEGNKHHKSTPLLIHCRDGSQQTGIFCALLNLLESAETEEVVDIFQVVKALRKARPGMVS

TFEQYQFLYDVIASTYPAQNGQVKKNNHQEDKIEFDNEVDKVKQDANCVNPLGAPEKLPEA

KEQAEGSEPTSGTEGPEHSVNGPASPALNQGS

The present invention relates to the modulation of the relative expression of the two or more CARs in an AND gate. This may be done, for example, by including an intracellular retention signal in one or other CAR.

In relation to SEQ ID NO. 4 and 5 given above, a suitable position for the intracellular retention signal may be readily determined based on the position of the retention signal, or signals of a similar type, in its native protein. The modulatory effect of the retention signal may also be fine tuned by choosing a certain position for the retention signal in the molecule.

For example, the nucleic acid construct may comprise a tyrp-1 retention protein sequence, such as:

RARRSMDEANQPLLTDQYQCYAEEYEKLQNPNQSVV (SEQ ID No. 6)

Such a sequence may be included, for example, in the CD19 CAR sequence in order for the relative expression level of CD19 CAR to be reduced with respect to CD33 CAR.

The position of the tyrp-1 retention signal in the aCD19 receptor will alter the amount of reduction: for low expression levels, the tyrp-1 retention signal may be placed between "Human CD28TM" and "Human CD3zeta intracellular domain"; for medium expression levels the tyrp-1 retention signal may be placed between "Human CD3zeta intracellular domain" and "2A peptide" in SEQ ID NO. 4 or 5.

Alternatively, the nucleic acid construct may comprise the Adenoviral E3/19K intracellular retention signal. The intracellular retention signal may comprise the E3/19K cytosolic domain KYKSRRSFIDEKKMP (SEQ ID No. 2) or a portion thereof, such as the sequence DEKKMP (SEQ ID No. 3).

The E3/19K retention signal may be positioned at the C-terminus of the CAR whose expression is to be reduced.

The nucleic acid construct of the invention may encode SEQ ID No. 4 or 5, or any of their components parts, such as the CD19 CAR or the CD33 CAR.

The nucleic acid construct of the invention may encode a variant of the CAR -encoding part of the sequence shown as SEQ ID No. 4 or 5 having at least 80, 85, 90, 95, 98 or 99% sequence identity, provided that the variant sequence is a CAR having the required properties.

Methods of sequence alignment are well known in the art and are accomplished using suitable alignment programs. The % sequence identity refers to the percentage of amino acid or nucleotide residues that are identical in the two sequences when they are optimally aligned. Nucleotide and protein sequence homology or identity may be determined using standard algorithms such as a BLAST program (Basic Local Alignment Search Tool at the National Center for Biotechnology Information) using default parameters, which is publicly available at http://blast.ncbi.nlm.nih.gov. Other algorithms for determining sequence identity or homology include: LALIGN (http://www.ebi.ac.uk/Tools/psa/lalign/ and http://www.ebi.ac.uk/Tools/psa/lalign/nucleotide.html), AMAS (Analysis of Multiply Aligned Sequences, at http://www.compbio.dundee.ac.uk/Software/Amas/amas.html), FASTA (http://www.ebi.ac.uk/Tools/sss/fasta/), Clustal Omega (http://www.ebi.ac.uk/Tools/msa/clustalo/), SIM (http://web.expasy.org/sim/), and EMBOSS Needle (http://www.ebi.ac.uk/Tools/psa/emboss needle/nucleotide.html).

KINETIC SEGREGATION MODEL

Subsequent pairing of CARs to generate the AND gate and the AND NOT gate are based on the kinetic segregation model (KS) of T-cell activation. This is a functional model, backed by experimental data, which explains how antigen recognition by a T-cell receptor is converted into down-stream activation signals. Briefly: at the ground state, the signalling components on the T-cell membrane are in dynamic homeostasis whereby dephosphorylated ITAMs are favoured over phosphorylated ITAMs. This is due to greater activity of the transmembrane CD45/CD148 phosphatases over membrane-tethered kinases such as lck. When a T-cell engages a target cell through a T-cell receptor (or CAR) recognition of cognate antigen, tight immunological synapses form. This close juxtapositioning of the T-cell and target membranes excludes CD45/CD148 due to their large ectodomains which cannot fit into the synapse. Segregation of a high concentration of T-cell receptor associated ITAMs and kinases in the synapse, in the absence of phosphatases, leads to a state whereby phosphorylated ITAMs are favoured. ZAP70 recognizes a threshold of phosphorylated ITAMs and propagates a T-cell activation signal. This advanced understanding of T-cell activation is exploited by the present invention. In particular, the invention is based on this understanding of how ectodomains of different length and/or bulk and/or charge and/or configuration and/or glycosylation result in differential segregation upon synapse formation.

THE CAR LOGICAL AND GATE

In the AND gate, one CAR comprises an activating endodomain and one CAR comprises an inhibitory endodomain whereby the inhibitory CAR constitutively inhibits the first activating CAR, but upon recognition of its cognate antigen releases its inhibition of the activating CAR. In this manner, a T-cell can be engineered to trigger only if a target cell expresses both cognate antigens. This behaviour is achieved by the activating CAR comprising an activating endodomain containing ITAM domains for example the endodomain of CD3 Zeta, and the inhibitory CAR comprising the endodomain from a phosphatase able to dephosphorylate an ITAM (e.g. CD45 or CD148). Crucially, the spacer domains of both CARs are significantly different in size. When only the activating CAR is ligated, the inhibitory CAR is in solution on the T-cell surface and can diffuse in and out of the synapse inhibiting the activating CAR. When both CARs are ligated, due to differences in spacer properties, the activating and inhibiting CAR are differentially segregated allowing the activating CAR to trigger T-cell activation unhindered by the inhibiting CAR.

This is of considerable utility in the field of cancer therapy. Currently, immunotherapies typically target a single antigen. Most cancers cannot be differentiated from normal tissues on the basis of a single antigen. Hence, considerable "on-target off-tumour" toxicity occurs whereby normal tissues are damaged by the therapy. For instance, whilst targeting CD20 to treat B-cell lymphomas with Rituximab, the entire normal B-cell compartment is depleted. For instance, whilst targeting CD52 to treat chronic lymphocytic leukaemia, the entire lymphoid compartment is depleted. For instance, whilst targeting CD33 to treat acute myeloid leukaemia, the entire myeloid compartment is damaged etc. By restricting activity to a pair of antigens, much more refined targeting, and hence less toxic therapy can be developed. A practical example is targeting of CLL which expresses both CD5 and CD19. Only a small proportion of normal B-cells express both antigens, so the off-target toxicity of targeting both antigens with a logical AND gate is substantially less than targeting each antigen individually.

The design of the present invention is a considerable improvement on previous implementation as described by Wilkie et al. ((2012). J. Clin. Immunol. 32, 1059-1070) and then tested *in vivo* (Kloss et al (2013) Nat. Biotechnol. 31, 71-75). In this implementation, the first CAR comprises of an activating endodomain, and the second a co-stimulatory domain. This way, a T-cell only receives an activating and co-stimulatory signal when both antigens are present. However, the T-cell still will activate in the sole presence of the first antigen resulting in the potential for off-target toxicity. Further, the implementation of the present invention allows for multiple compound linked gates whereby a cell can interpret a complex pattern of antigens.

**TABLE 4**

| Cancer Type | Antigens |
| --- | --- |
| Chronic Lymphocytic Leukaemia | CD5, CD19 |
| Neuroblastoma | ALK, GD2 |
| Glioma | EGFR, Vimentin |
| Multiple myeloma | BCMA, CD138 |
| Renal Cell Carcinoma | Carbonic anhydrase IX, G250 |

(continued)

| Cancer Type | Antigens |
|---|---|
| T-ALL | CD2, N-Cadherin |
| Prostate Cancer | PSMA, hepsin (or others) |

DIFFERENTIAL EXPRESSION OF TARGET ANTIGENS

The AND gate of the present invention triggers in the presence of at least two antigens, for example in the presence of one of the antigen pairs listed in Table 4. It is known that for some antigen pairs, the relative levels of expression of the antigens on the cancer cell can be very different. In these circumstances, the present invention enables the relative levels of expression of each antigen-specific CAR to be modulated in order to reflect the relative level of expression of the target antigen.

For example, Cabezudo et al (1999 Haematologica 84:413) investigated the expression of CD5 and CD19 in cells from a variety of B-cell disorders, including chronic lymphocytic leukemia (CLL), B-cell prolymphocytic leukemia (PLL), splenic lymphoma with villous lymphocytes (SLVL) and mantle-cell (Mc) lymphoma in leukemic phase. It was found that for all the B-cell disorders investigated, the levels of expression of CD5 was 2-8 fold greater than the level of expression of CD19.

In the AND gate of the present invention, the relative expression of the CD19 CAR and CD5 CAR may be modified in order to reflect the relative levels of CD19 and CD5 on the cancer cells. For example, the level of expression of the CD19 CAR may be reduced by incorporation of an intracellular retention signal or by altering the signal peptide such that it has fewer hydrophobic amino acids than the signal peptide of the CD5 CAR.

COMPOUND GATES

The kinetic segregation model with the above components allows compound gates to be made e.g. a T-cell which triggers in response to patterns of more than two target antigens. For example, it is possible to make a T cell which only triggers when three antigens are present (A AND B AND C). Here, a cell expresses three CARs, each recognizing antigens A, B and C. One CAR is excitatory and two are inhibitory, which each CAR having spacer domains which result in differential segregation. Only when all three are ligated, will the T-cell activate. A further example: (A OR B) AND C: here, CARs recognizing antigens A and B are activating and have spacers which co-localise, while CAR recognizing antigen C is inhibitory and has a spacer which results in different co-segregation.

SIGNAL PEPTIDE

The polypeptides A and B (and optionally others, C, D etc) encoded by the nucleic acid construct of the invention each comprise may a signal sequence so that when the polypeptide is expressed inside a cell the nascent protein is directed to the endoplasmic reticulum (ER) (see Figure 20).

The term "signal peptide" is synonymous with "signal sequence".

A signal peptide is a short peptide, commonly 5-30 amino acids long, present at the N-terminus of the majority of newly synthesized proteins that are destined towards the secretory pathway. These proteins include those that reside either inside certain organelles (for example, the endoplasmic reticulum, golgi or endosomes), are secreted from the cell, and transmembrane proteins.

Signal peptides commonly contain a core sequence which is a long stretch of hydrophobic amino acids that has a tendency to form a single alpha-helix. The signal peptide may begin with a short positively charged stretch of amino acids, which helps to enforce proper topology of the polypeptide during translocation. At the end of the signal peptide there is typically a stretch of amino acids that is recognized and cleaved by signal peptidase. Signal peptidase may cleave either during or after completion of translocation to generate a free signal peptide and a mature protein. The free signal peptides are then digested by specific proteases.

The signal peptide is commonly positioned at the amino terminus of the molecule, although some carboxy-terminal signal peptides are known.

As mentioned above, signal sequences have a tripartite structure, consisting of a hydrophobic core region (h-region) flanked by an n- and c-region. The latter contains the signal peptidase (SPase) consensus cleavage site. Usually, signal sequences are cleaved off co-translationally, the resulting cleaved signal sequences are termed signal peptides.

In the signal peptide from the murine Ig kappa chain V-III region, which has the sequence: METD**TLILWVLLLLV**PGSTG: the n-region has the sequence METD; the h-region (shown in bold) has the sequence TLILWVLLLLV; and the c-region has the sequence PGSTG.

In the nucleic acid construct of the present invention, the signal peptides of the two CARs may differ in the number of hydrophobic amino acids, to modulate the relative levels of expression of the CARs at the cell surface.

In the nucleic acid construct of the present invention the signal sequence of the two (or more) polypeptides therefore may differ in their h-regions. One polypeptide (which has higher relative expression) may have a greater number of hydrophobic amino acids in the h-region that the other polypeptide (which has lower relative expression). The signal peptide of the polypeptide with lower relative expression may comprise one or more amino acid mutations, such as substitutions or deletions, of hydrophobic amino acids in the h-region than the signal peptide of the polypeptide with lower relative expression.

The first signal peptide and the second signal peptide may have substantially the same n- and c- regions, but differ in the h-region as explained above. "Substantially the same" indicates that the n- and c- regions may be identical between the first and second signal peptide or may differ by one, two or three amino acids in the n- or c-chain, without affecting the function of the signal peptide.

The hydrophobic amino acids in the core may, for example be: Alanine (A); Valine (V); Isoleucine (I); Leucine (L); Methionine (M); Phenylalanine (P); Tyrosine (Y); or Tryptophan (W).

The hydrophobic acids mutated in order to alter signal peptide efficiency may be any from the above list, in particular: Valine (V); Isoleucine (I); Leucine (L); and Tryptophan (W).

Of the residues in the h-region, one signal peptide (for example, the altered signal peptide) may comprise at least 10%, 20%, 30%, 40% or 50% fewer hydrophobic amino acids than the other signal peptide (for example, the unaltered signal peptide).

Where the h-region comprises 5-15 amino acids, one signal peptide may comprise 1, 2, 3, 4 or 5 more hydrophobic amino acids than the other signal peptide.

The altered signal peptide may comprise 1, 2, 3, 4 or 5 amino acid deletions or substitutions of hydrophobic amino acids. Hydrophobic amino acids may be replaced with non-hydrophobic amino acids, such as hydrophilic or neutral amino acids.

Signal sequences can be detected or predicted using software techniques (see for example, http://www.predisi.de/).

A very large number of signal sequences are known, and are available in databases. For example, http://www.signalpeptide.de lists 2109 confirmed mammalian signal peptides in its database.

Table 5 provides a list of signal sequences purely for illustrative purposes. The hydrophobic core is highlighted in bold. This includes examples of amino acids which may be substituted or removed for the purposes of the present invention.

Table 5

| Accession Number | Entry Name | Protein Name | Length | Signal Sequence (hydrophobic core) |
|---|---|---|---|---|
| P01730 | CD4_HUMAN | T-cell surface glycoprotein CD4 | 25 | MNRGVPFRH**LLLVL**Q**LALL**PAATQG |
| P08575 | CD45_HUMAN | Leukocyte common antigen | 23 | MYLWLK**LLAFGFAFL**DTE**VFV**TG |
| P01732 | CD8A_HUMAN | T-cell surface glycoprotein CD8 alpha chain | 21 | MALP**VTALLL**PL**ALLL**HAARP |
| P10966 | CD8B_HUMAN | T-cell surface glycoprotein CD8 beta chain | 21 | MRPRLW**LLLAAQLTVL**HGNSV |
| P06729 | CD2_HUMAN | T-cell surface antigen CD2 | 24 | MSFPCK**FVASF**LLIFNVSSKGAVS |
| P06127 | CD5_HUMAN | T-cell surface glycoprotein CD5 | 24 | MPMGSLQP**LATL**Y**LLGMLVASCL**G |
| P09564 | CD7_HUMAN | T-cell antigen CD7 | 25 | MAGPPR**LLLLPLLLA**LARGLPGALA |
| P17643 | TYRP1_HUMAN | 5,6-dihydroxyindole-2-carboxylic acid oxidase | 24 | MSAPK**LLSLGCIFFPLLLF**QQARA |

(continued)

| Accession Number | Entry Name | Protein Name | Length | Signal Sequence (hydrophobic core) |
|---|---|---|---|---|
| P00709 | LALBA_HUMAN | Alpha-lactalbumin | 19 | MR**FFVPLFLVGILF**PAILA |
| P16278 | BGAL_HUMAN | Beta-galactosidase | 23 | MPG**FLVRIL**PLLL**VLLLLG**PTRG |
| P31358 | CD52_HUMAN | CAMPATH-1 antigen | 24 | MKR**FLFLLLTISLLVM**VQIQTGLS |
| Q6YHK3 | CD109_HUMAN | CD109 antigen | 21 | MQGPP**LLTAA**H**LLCVCTAAL**A |
| P01024 | CO3_HUMAN | Complement C3 | 22 | MGPTSGPS**LLLLLLT**HLPLALG |
| P10144 | GRAB_HUMAN | Granzyme B | 18 | MQP**ILLLLAFLLL**PRADA |
| P04434 | KV310_HUMAN | Ig kappa chain V-III region VH | 20 | MEAPAQ**LLFLLLL**WLPDTTR |
| P06312 | KV401_HUMAN | Ig kappa chain V-IV region | 20 | MVLQTQ**VFISLLL**WISGAYG |
| P06319 | LV605_HUMAN | Ig lambda chain V-VI region EB4 | 19 | MAWAP**LLLTLLA**HCTDCWA |
| P31785 | IL2RG_HUMAN | Cytokine receptor common gamma chain | 22 | MLKPSLPFTS**LLFL**QLPLL**GV**G |
| Q8N4F0 | BPIL1_HUMAN | Bactericidal/permeability-increasing protein-like 1 | 20 | MAWASR**LGLLLALLL**PVVGA |
| P55899 | FCGRN_HUMAN | IgG receptor FcRn large subunit p51 | 23 | MGVPRPQPW**ALGLLL**FLLPGSLG |

The mutated signal peptide comprises one or more mutation(s) such that it has fewer hydrophobic amino acids than the wild-type signal peptide from which it is derived. The term "wild type" means the sequence of the signal peptide which occurs in the natural protein from which it is derived. For example, the signal peptide described in the examples is the signal peptide from the murine Ig kappa chain V-III region, which has the wild-type sequence: METDTLILWVLLLLVPGSTG.

The term "wild-type" also includes signal peptides derived from a naturally occurring protein which comprise one or more amino acid mutations in the n- or c- region. For example it is common to modify a natural signal peptide with a conserved amino acid substitution on the N-terminus to introduce a restriction site. Such modified signal peptide sequences (which do not comprise any mutations in the h-region) are considered "wild-type" for the purposes of the present invention.

The present disclosure also relates to synthetic signal peptide sequences, which cannot be defined with reference to a wild-type sequence. Here, the signal peptide of the one polypeptide comprises fewer hydrophobic amino acids than the signal sequence of the other polypeptide. The two signal sequences may be derived from the same synthetic signal peptide sequence, but differ in the number of hydrophobic amino acids in the core region.

ANTIGEN BINDING DOMAIN

The antigen binding domain is the portion of the CAR which recognizes antigen. Numerous antigen-binding domains are known in the art, including those based on the antigen binding site of an antibody, antibody mimetics, and T-cell receptors. For example, the antigen-binding domain may comprise: a single-chain variable fragment (scFv) derived from a monoclonal antibody; a natural ligand of the target antigen; a peptide with sufficient affinity for the target; a single domain antibody; an artificial single binder such as a Darpin (designed ankyrin repeat protein); or a single-chain derived from a T-cell receptor.

The antigen binding domain may comprise a domain which is not based on the antigen binding site of an antibody. For example the antigen binding domain may comprise a domain based on a protein/peptide which is a soluble ligand for a tumour cell surface receptor (e.g. a soluble peptide such as a cytokine or a chemokine); or an extracellular domain of a membrane anchored ligand or a receptor for which the binding pair counterpart is expressed on the tumour cell.

The antigen binding domain may be based on a natural ligand of the antigen. For example, the antigen binding domain may comprise APRIL, the natural ligand of BCMA.

The antigen binding domain may comprise an affinity peptide from a combinatorial library or a de novo designed affinity protein/peptide.

SPACER DOMAIN

CARs comprise a spacer sequence to connect the antigen-binding domain with the transmembrane domain and spatially separate the antigen-binding domain from the endodomain. A flexible spacer allows the antigen-binding domain to orient in different directions to facilitate binding.

The first and second CARs encoded by the nucleic acid construct of the invention comprise different size spacer molecules. For example, the spacer sequence may, for example, comprise an IgG1 Fc region, an IgG1 hinge or a human CD8 stalk or the mouse CD8 stalk. The spacer may alternatively comprise an alternative linker sequence which has similar length and/or domain spacing properties as an IgG1 Fc region, an IgG1 hinge or a CD8 stalk. A human IgG1 spacer may be altered to remove Fc binding motifs.

Examples of amino acid sequences for these spacers are given below:

SEQ ID No. 7 (hinge-CH2CH3 of human IgG1)

AEPKSPDKTHTCPPCPAPPVAGPSVFLFPPKPKDTLMIARTPEVTCVVVDVSHEDPEVKFN

WYVDGVEVHNAKTKPREEQYNSTYRVVSVLTVLHQDWLNGKEYKCKVSNKALPAPIEKTIS

KAKGQPREPQVYTLPPSRDELTKNQVSLTCLVKGFYPSDIAVEWESNGQPENNYKTTPPVL

DSDGSFFLYSKLTVDKSRWQQGNVFSCSVMHEALHNHYTQKSLSLSPGKKD

SEQ ID No. 8 (human CD8 stalk):
TTTPAPRPPTPAPTIASQPLSLRPEACRPAAGGAVHTRGLDFACDI

SEQ ID No. 9 (human IgG1 hinge):
AEPKSPDKTHTCPPCPKDPK

SEQ ID No. 10 (CD2 ectodomain)

KEITNALETWGALGQDINLDIPSFQMSDDIDDIKWEKTSDKKKIAQFRKEKETFKEKDTYKLF

KNGTLKIKHLKTDDQDIYKVSIYDTKGKNVLEKIFDLKIQERVSKPKISWTCINTTLTCEVMNG

TDPELNLYQDGKHLKLSQRVITHKWTTSLSAKFKCTAGNKVSKESSVEPVSCP

EKGLD

SEQ ID no. 11 (CD34 ectodomain)

SLDNNGTATPELPTQGTFSNVSTNVSYQETTTPSTLGSTSLHPVSQHGNEATTNITETTVKF

TSTSVITSVYGNTNSSVQSQTSVISTVFTTPANVSTPETTLKPSLSPGNVSDLSTTSTSLATS

PTKPYTSSSPILSDIKAEIKCSGIREVKLTQGICLEQNKTSSCAEFKKDRGEGLARVLCGEEQ

ADADAGAQVCSLLLAQSEVRPQCLLLVLANRTEISSKLQLMKKHQSDLKKLGILDFTEQDVA

SHQSYSQKT

Since CARs are typically homodimers (see Figure 1a), cross-pairing may result in a heterodimeric chimeric antigen receptor. This is undesirable for various reasons, for example: (1) the epitope may not be at the same "level" on the target cell so that a cross-paired CAR may only be able to bind to one antigen; (2) the VH and VL from the two different scFv could swap over and either fail to recognize target or worse recognize an unexpected and unpredicted antigen.

For the AND gate it is important that the spacer of the first CAR has a different length, and/or charge and/or shape and/or configuration and/or glycosylation, such that when both first and second CARs bind their target antigen, the difference in spacer charge or dimensions results in spatial separation of the two types of CAR to different parts of the membrane to result in activation as predicted by the kinetic separation model. In the construct of the present invention, therefore, the different length, shape and/or configuration of the spacers is carefully chosen bearing in mind the size and binding epitope on the target antigen to allow differential segregation upon cognate target recognition. For example the IgG1 Hinge, CD8 stalk, IgG1 Fc, ectodomain of CD34, ectodomain of CD45 are expected to differentially segregate.

Examples of spacer pairs which differentially segregate and are therefore suitable for use with the AND gate are shown in the following Table:

| Stimulatory CAR spacer | Inhibitory CAR spacer |
| --- | --- |
| Human-CD8STK | Human-IgG-Hinge-CH2CH3 |
| Human-CD3z ectodomain | Human-IgG-Hinge-CH2CH3 |
| Human-IgG-Hinge | Human-IgG-Hinge-CH2CH3 |
| Human-CD28STK | Human-IgG-Hinge-CH2CH3 |
| Human-CD8STK | Human-IgM-Hinge-CH2CH3CD4 |
| Human-CD3z ectodomain | Human-IgM-Hinge-CH2CH3CD4 |
| Human-IgG-Hinge | Human-IgM-Hinge-CH2CH3CD4 |
| Human-CD28STK | Human-IgM-Hinge-CH2CH3CD4 |

The relative dimensions of some commonly used spacers are illustrated in Figure 13. Figure 14 shows a matrix of spacer pairs and their suitability for use with an AND gate.

All the spacer domains mentioned above form homodimers. However the mechanism is not limited to using homodimeric receptors and should work with monomeric receptors as long as the spacer is sufficiently rigid. An example of such a spacer is CD2 or truncated CD22.

TRANSMEMBRANE DOMAIN

The transmembrane domain is the sequence of the CAR that spans the membrane.

A transmembrane domain may be any protein structure which is thermodynamically stable in a membrane. This is typically an alpha helix comprising of several hydrophobic residues. The transmembrane domain of any trans-membrane protein can be used to supply the transmembrane portion of the invention. The presence and span of a transmembrane domain of a protein can be determined by those skilled in the art using the TMHMM algorithm (http://www.cbs.dtu.dk/services/TMHMM-2.0/). Further, given that the transmembrane domain of a protein is a relatively simple structure, i.e a polypeptide sequence predicted to form a hydrophobic alpha helix of sufficient length to span the membrane, an artificially designed TM domain may also be used (US 7052906 B1 describes synthetic transmembrane components).

The transmembrane domain may be derived from CD28, which gives good receptor stability.

ACTIVATING ENDODOMAIN

The endodomain is the signal-transmission portion of the CAR. After antigen recognition, receptors cluster, native CD45 and CD148 are excluded from the synapse and a signal is transmitted to the cell. The most commonly used endodomain component is that of CD3-zeta which contains 3 ITAMs. This transmits an activation signal to the T cell after antigen is bound. CD3-zeta may not provide a fully competent activation signal and additional co-stimulatory signaling may be needed. For example, chimeric CD28 and OX40 can be used with CD3-Zeta to transmit a proliferative / survival signal, or all three can be used together.

In the "AND gate" of the present invention, the cell comprises at least two CARs, at least one of which is an activating CAR which comprises or associates with an activating endodomain. An activating endodomain may, for example, comprise the CD3-Zeta endodomain alone, the CD3-Zeta endodomain with that of either CD28 or OX40 or the CD28 endodomain and OX40 and CD3-Zeta endodomain.

Any endodomain which contains an ITAM motif can act as an activating endodomain in this invention. Several proteins are known to contain endodomains with one or more ITAM motifs. Examples of such proteins include the CD3 epsilon chain, the CD3 gamma chain and the CD3 delta chain to name a few. The ITAM motif can be easily recognized as a tyrosine separated from a leucine or isoleucine by any two other amino acids, giving the signature YxxL/I. Typically, but not always, two of these motifs are separated by between 6 and 8 amino acids in the tail of the molecule (YxxL/Ix(6-8)YxxL/I). Hence, one skilled in the art can readily find existing proteins which contain one or more ITAM to transmit an activation signal. Further, given the motif is simple and a complex secondary structure is not required, one skilled in the art can design polypeptides containing artificial ITAMs to transmit an activation signal (see WO 2000063372, which relates to synthetic signalling molecules).

The transmembrane and intracellular T-cell signalling domain (endodomain) of a CAR with an activating endodomain may comprise the sequence shown as SEQ ID No. 12, 13 or 14 or a variant thereof having at least 80% sequence identity.

SEQ ID No. 12 comprising CD28 transmembrane domain and CD3 Z endodomain

FWVLVVVGGVLACYSLLVTVAFIIFWVRRVKFSRSADAPAYQQGQNQLYNELNLGRREEY
DVLDKRRGRDPEMGGKPRRKNPQEGLYNELQKDKMAEAYSEIGMKGERRRGKGHDGLY
QGLSTATKDTYDALHMQALPPR

SEQ ID No. 13 comprising CD28 transmembrane domain and CD28 and CD3 Zeta endodomains

FWVLVVVGGVLACYSLLVTVAFIIFWVRSKRSRLLHSDYMNMTPRRPGPTRKHYQPYAPP
RDFAAYRSRVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDALHMQALP
PR

SEQ ID No. 14 comprising CD28 transmembrane domain and CD28, OX40 and CD3 Zeta endodomains.

FWVLVVVGGVLACYSLLVTVAFIIFWVRSKRSRLLHSDYMNMTPRRPGPTRKHYQPYAPP
RDFAAYRSRDQRLPPDAHKPPGGGSFRTPIQEEQADAHSTLAKIRVKFSRSADAPAYQQG
QNQLYNELNLGRREEYDVLDKRRGRDPEMGGKPRRKNPQEGLYNELQKDKMAEAYSEIG
MKGERRRGKGHDGLYQGLSTATKDTYDALHMQALPPR

A variant sequence may have at least 80%, 85%, 90%, 95%, 98% or 99% sequence identity to SEQ ID No. 12, 13 or 14, provided that the sequence provides an effective trans-membrane domain and an effective intracellular T cell signaling domain.

"LIGATION-OFF" INHIBITORY ENDODOMAIN

In the AND gate of the present invention, one of the CARs comprises an inhibitory endodomain such that the inhibitory CAR inhibits T-cell activation by the activating CAR in the absence of inhibitory CAR ligation, but does not significantly inhibit T-cell activation by the activating CAR when the inhibitory CAR is ligated. This is termed a "ligation-off" inhibitory endodomain.

In this case, the spacer of the inhibitory CAR is of a different size from the spacer of the activating CAR, such that when both receptors are ligated, the difference in spacer dimensions results in isolation of the activating CARs and the inhibitory CARs in different membrane compartments of the immunological synapse, so that the activating endodomain is released from inhibition by the inhibitory endodomain.

The inhibitory endodomains for use in a ligation-off inhibitory CAR may therefore comprise any sequence which inhibits T-cell signaling by the activating CAR when it is in the same membrane compartment (i.e. in the absence of the antigen for the inhibitory CAR) but which does not significantly inhibit T cell signaling when it is isolated in a separate part of the membrane from the inhibitory CAR.

The ligation-off inhibitory endodomain may be or comprise a tyrosine phosphatase, such as a receptor-like tyrosine phosphatase. An inhibitory endodomain may be or comprise any tyrosine phosphatase that is capable of inhibiting the TCR signalling when only the stimulatory receptor is ligated. An inhibitory endodomain may be or comprise any tyrosine phosphatase with a sufficiently fast catalytic rate for phosphorylated ITAMs that is capable of inhibiting the TCR signalling when only the stimulatory receptor is ligated.

For example, the inhibitory endodomain of an AND gate may comprise the endodomain of CD148 or CD45.

CD148 and CD45 have been shown to act naturally on the phosphorylated tyrosines up-stream of TCR signalling.

CD148 is a receptor-like protein tyrosine phosphatase which negatively regulates TCR signaling by interfering with the phosphorylation and function of PLCγ1 and LAT.

CD45 present on all hematopoetic cells, is a protein tyrosine phosphatase which is capable of regulating signal transduction and functional responses, again by phosphorylating PLC γ1.

An inhibitory endodomain may comprise all of part of a receptor-like tyrosine phosphatase. The phospatase may interfere with the phosphorylation and/or function of elements involved in T-cell signalling, such as PLCγ1 and/or LAT.

The transmembrane and endodomain of CD45 and CD148 is shown as SEQ ID No. 15 and No.16 respectively.

## SEQ ID 15 - CD45 trans-membrane and endodomain sequence

ALIAFLAFLIIVTSIALLVVLYKIYDLHKKRSCNLDEQQELVERDDEKQLMNVEPIHADILLETYK

RKIADEGRLFLAEFQSIPRVFSKFPIKEARKPFNQNKNRYVDILPYDYNRVELSEINGDAGSN

YINASYIDGFKEPRKYIAAQGPRDETVDDFWRMIWEQKATVIVMVTRCEEGNRNKCAEYWP

SMEEGTRAFGDVVVKINQHKRCPDYIIQKLNIVNKKEKATGREVTHIQFTSWPDHGVPEDPH

LLLKLRRRVNAFSNFFSGPIVVHCSAGVGRTGTYIGIDAMLEGLEAENKVDVYGYVVKLRRQ

RCLMVQVEAQYILIHQALVEYNQFGETEVNLSELHPYLHNMKKRDPPSEPSPLEAEFQRLP

SYRSWRTQHIGNQEENKSKNRNSNVIPYDYNRVPLKHELEMSKESEHDSDESSDDDSDSE

EPSKYINASFIMSYWKPEVMIAAQGPLKETIGDFWQMIFQRKVKVIVMLTELKHGDQEICAQ

YWGEGKQTYGDIEVDLKDTDKSSTYTLRVFELRHSKRKDSRTVYQYQYTNWSVEQLPAEP

KELISMIQVVKQKLPQKNSSEGNKHHKSTPLLIHCRDGSQQTGIFCALLNLLESAETEEVVDI

FQVVKALRKARPGMVSTFEQYQFLYDVIASTYPAQNGQVKKNNHQEDKIEFDNEVDKVKQ

DANCVNPLGAPEKLPEAKEQAEGSEPTSGTEGPEHSVNGPASPALNQGS

## SEQ ID 16 - CD148 trans-membrane and endodomain sequence

AVFGCIFGALVIVTVGGFIFWRKKRKDAKNNEVSFSQIKPKKSKLIRVENFEAYFKKQQADSN

CGFAEEYEDLKLVGISQPKYAAELAENRGKNRYNNVLPYDISRVKLSVQTHSTDDYINANYM

PGYHSKKDFIATQGPLPNTLKDFWRMVWEKNVYAIIMLTKCVEQGRTKCEEYWPSKQAQD

YGDITVAMTSEIVLPEWTIRDFTVKNIQTSESHPLRQFHFTSWPDHGVPDTTDLLINFRYLVR

DYMKQSPPESPILVHCSAGVGRTGTFIAIDRLIYQIENENTVDVYGIVYDLRMHRPLMVQTED

QYVFLNQCVLDIVRSQKDSKVDLIYQNTTAMTIYENLAPVTTFGKTNGYIA

An inhibitory CAR may comprise all or part of SEQ ID No 15 or 16 (for example, it may comprise the phosphatase function of the endodomain). It may comprise a variant of the sequence or part thereof having at least 80%, 85%, 90% or 95% sequence identity, as long as the variant retains the capacity to basally inhibit T cell signalling by the activating CAR.

Other spacers and endodomains may be tested for example using the model system exemplified herein. Target cell populations can be created by transducing a suitable cell line such as a SupT1 cell line either singly or doubly to establish cells negative for both antigens (the wild-type), positive for either and positive for both (e.g. CD19-CD33-, CD19+CD33-, CD19-CD33+ and CD19+CD33+). T cells such as the mouse T cell line BW5147 which releases IL-2 upon activation may be transduced with pairs of CARs and their ability to function in a logic gate measured through measurement of IL-2 release (for example by ELISA). For example, it is shown in Example 4 that both CD148 and CD45 endodomains can function as inhibitory CARs in combination with an activating CAR containing a CD3 Zeta endodomain. These CARs rely upon a short/non-bulky CD8 stalk spacer on one CAR and a bulky Fc spacer on the other CAR to achieve AND gating. When both receptors are ligated, the difference in spacer dimensions results in isolation of the different receptors in different membrane compartments, releasing the CD3 Zeta receptor from inhibition by the CD148 or CD45 endodomains. In this way, activation only occurs once both receptors are activated. It can be readily seen that this modular system can be used to test alternative spacer pairs and inhibitory endodomains. If the spacers do not achieve isolation following ligation of both receptors, the inhibition would not be released and so no activation would occur. If the inhibitory endodomain under test is ineffective, activation would be expected in the presence of ligation of the activating CAR irrespective of the ligation status of the inhibitory CAR.

CLEAVAGE SITE

The present nucleic acid construct comprises a sequence encoding a cleavage site positioned between nucleic acid sequences which encode first and second CARs, such that the first and second CARs can be expressed as separate entities.

The cleavage site may be any sequence which enables the polypeptide comprising the first and second CARs to become separated.

The term "cleavage" is used herein for convenience, but the cleavage site may cause the first and second CARs to separate into individual entities by a mechanism other than classical cleavage. For example, for the Foot-and-Mouth disease virus (FMDV) 2A self-cleaving peptide (see below), various models have been proposed for to account for the "cleavage" activity: proteolysis by a host-cell proteinase, autoproteolysis or a translational effect (Donnelly et al (2001) J. Gen. Virol. 82:1027-1041). The exact mechanism of such "cleavage" is not important for the purposes of the present invention, as long as the cleavage site, when positioned between nucleic acid sequences which encode first and second CARs, causes the first and second CARs to be expressed as separate entities.

The cleavage site may be a furin cleavage site.

Furin is an enzyme which belongs to the subtilisin-like proprotein convertase family. The members of this family are proprotein convertases that process latent precursor proteins into their biologically active products. Furin is a calcium-dependent serine endoprotease that can efficiently cleave precursor proteins at their paired basic amino acid processing sites. Examples of furin substrates include proparathyroid hormone, transforming growth factor beta 1 precursor, proalbumin, pro-beta-secretase, membrane type-1 matrix metalloproteinase, beta subunit of pro-nerve growth factor and von Willebrand factor. Furin cleaves proteins just downstream of a basic amino acid target sequence (canonically, Arg-X-(Arg/Lys)-Arg') and is enriched in the Golgi apparatus.

The cleavage site may be a Tobacco Etch Virus (TEV) cleavage site.

TEV protease is a highly sequence-specific cysteine protease which is chymotrypsin-like proteases. It is very specific for its target cleavage site and is therefore frequently used for the controlled cleavage of fusion proteins both *in vitro* and *in vivo*. The consensus TEV cleavage site is ENLYFQ\S (where '\' denotes the cleaved peptide bond). Mammalian cells, such as human cells, do not express TEV protease. Thus in embodiments in which the present nucleic acid construct comprises a TEV cleavage site and is expressed in a mammalian cell - exogenous TEV protease must also expressed in the mammalian cell.

The cleavage site may encode a self-cleaving peptide.

A 'self-cleaving peptide' refers to a peptide which functions such that when the polypeptide comprising the first and second CARs and the self-cleaving peptide is produced, it is immediately "cleaved" or separated into distinct and discrete first and second CARs without the need for any external cleavage activity.

The self-cleaving peptide may be a 2A self-cleaving peptide from an aphtho- or a cardiovirus. The primary 2A/2B cleavage of the aptho- and cardioviruses is mediated by 2A "cleaving" at its own C-terminus. In apthoviruses, such as foot-and-mouth disease viruses (FMDV) and equine rhinitis A virus, the 2A region is a short section of about 18 amino acids, which, together with the N-terminal residue of protein 2B (a conserved proline residue) represents an autonomous element capable of mediating "cleavage" at its own C-terminus.

The C-terminal 19 amino acids of the longer cardiovirus protein, together with the N-terminal proline of 2B mediate "cleavage" with an efficiency approximately equal to the apthovirus FMDV 2a sequence. Cardioviruses include encephalomyocarditis virus (EMCV) and Theiler's murine encephalitis virus (TMEV).

Mutational analysis of EMCV and FMDV 2A has revealed that the motif DxExNPGP is intimately involved in "cleavage" activity (Donelly et al (2001) as above).

The cleavage site of the present invention may comprise the amino acid sequence:

$Dx_1Ex_2NPGP$, where $x_1$ and $x_2$ are any amino acid. $X_1$ may be selected from the following group: I, V, M and S. $X_2$ may be selected from the following group: T, M, S, L, E, Q and F.

For example, the cleavage site may comprise one of the amino acid sequences shown in Table 6.

Table 6

| Motif | Present in: |
|---|---|
| DIETNPGP | Picornaviruses EMCB, EMCD, EMCPV21 |

(continued)

| Motif | Present in: |
|---|---|
| DVETNPGP | Picornaviruses MENGO and TMEBEAN; Insect virus DCV, ABPV |
| DVEMNPGP | Picornaviruses TMEGD7 and TMEBEAN |
| DVESNPGP | Picornaviruses FMDA10, FMDA12, FMDC1, FMD01K, FMDSAT3, FMDVSAT2, ERAV; Insect virus CrPV |
| DMESNPGP | Picornavirus FMDV01G |
| DVELNPGP | Picornavirus ERBV; Porcine rotavirus |
| DVEENPGP | Picornavirus PTV-1; Insect virus TaV; Trypanosoma TSR1 |
| DIELNPGP | Bovine Rotavirus, human rotavirus |
| DIEQNPGP | Trypanosoma AP endonuclease |
| DSEFNPGP | Bacterial sequence *T. maritima* |

The cleavage site, based on a 2A sequence may be, for example 15-22 amino acids in length. The sequence may comprise the C-terminus of a 2A protein, followed by a proline residue (which corresponds to the N-terminal proline of 2B).

Mutational studies have also shown that, in addition to the naturally occurring 2A sequences, some variants are also active. The cleavage site may correspond to a variant sequence from a naturally occurring 2A polypeptide, have one, two or three amino acid substitutions, which retains the capacity to induce the "cleavage" of a polyprotein sequence into two or more separate proteins.

The cleavage sequence may be selected from the following which have all been shown to be active to a certain extent (Donnelly *et al* (2001) as above):

<div align="center">

LLNFDLLKLAGDVESNPGP

LLNFDLLKLAGDVQSNPGP

LLNFDLLKLAGDVEINPGP

LLNFDLLKLAGDVEFNPGP

LLNFDLLKLAGDVESHPGP

LLNFDLLKLAGDVESEPGP

LLNFDLLKLAGDVESQPGP

LLNFDLLKLAGDVESNPGG

</div>

Based on the sequence of the DxExNPGP "a motif, "2A-like" sequences have been found in picornaviruses other than aptho- or cardioviruses, 'picornavirus-like' insect viruses, type C rotaviruses and repeated sequences within *Trypanosoma spp* and a bacterial sequence (Donnelly et al (2001) as above). The cleavage site may comprise one of these 2A-like sequences, such as:

YHADYYKQRLIHDVEMNPGP

HYAGYFADLLIHDIETNPGP

QCTNYALLKLAGDVESNPGP

ATNFSLLKQAGDVEENPGP

AARQMLLLLSGDVETNPGP

RAEGRGSLLTCGDVEENPGP

TRAEIEDELIRAGIESNPGP

TRAEIEDELIRADIESNPGP

AKFQIDKILISGDVELNPGP


SSIIRTKMLVSGDVEENPGP

CDAQRQKLLLSGDIEQNPGP

YPIDFGGFLVKADSEFNPGP

The cleavage site may comprise the 2A-like sequence RAEGRGSLLTCGDVEENPGP.

It has been shown that including an N-terminal "extension" of between 5 and 39 amino acids can increase activity (Donnelly et al (2001) as above). In particular, the cleavage sequence may comprise one of the following sequences or a variant thereof having, for example, up to 5 amino acid changes which retains cleavage site activity:


VTELLYRMKRAETYCPRPLAIHPTEARHKQKIVAPVKQTLNFDLLKLAGDVESNPGP

LLAIHPTEARHKQKIVAPVKQTLNFDLLKLAGDVESNPGP

EARHKQKIVAPVKQTLNFDLLKLAGDVESNPGP

APVKQTLNFDLLKLAGDVESNPGP


INTRACELLULAR RETENTION SIGNAL

The nucleic acid construct of the present invention may comprise a sequence which encodes a CAR comprising an intracellular retention signal.

Protein targeting or protein sorting is the biological mechanism by which proteins are transported to the appropriate destinations in the cell or outside of it. Proteins can be targeted to the inner space of an organelle, different intracellular membranes, plasma membrane, or to exterior of the cell via secretion. This delivery process is carried out based on sequence information contain in the protein itself.

Proteins synthesised in the rough endoplasmic reticulum (ER) of eukaryotic cells use the exocytic pathway for transport to their final destinations. Proteins lacking special sorting signals are vectorially transported from the ER via the Golgi and the trans-Golgi network (TGN) to the plasma membrane. Other proteins have targeting signals for incorporation into specific organelles of the exocytic pathway, such as endosomes and lysosomes.

Lysosomes are acidic organelles in which endogenous and internalised macromolecules are degraded by luminal hydolases. Endogenous macromolecules reach the lysosome by being sorted in the TGN from which they are transported to endosomes and then lysosomes.

The targeting signals used by a cell to sort proteins to the correct intracellular location may be exploited by the present invention. The signals may be broadly classed into the following types:

 i) endocytosis signals
 ii) Golgi retention signals
 iii) TGN recycling signals
 iv) ER retention signals
 v) lysosomal sorting signals

'Intracellular retention signal' refers to an amino acid sequence which directs the protein in which it is encompassed to a cellular compartment other than the cell surface membrane or to the exterior of the cell.

The intracellular retention signal causes a reduction in the amount of the CAR expressed on the surface of a cell compared to an equivalent, control CAR or other transmembrane protein which does not comprise an intracellular retention signal.

In other words, the proportion of translated CAR comprising an intracellular retention signal which is expressed on at the cell surface is less than the proportion of an equivalent amount of an equivalent, translated control CAR or other transmembrane protein which does not comprise an intracellular retention signal.

For example, the amount of the CAR comprising an intracellular retention signal which is expressed on the surface of a cell may be less than 75%, less than 50%, less than 25% or less than 10% of the amount of an equivalent control CAR or other transmembrane protein which does not comprise an intracellular retention signal.

Constructs which express a polyprotein that is subsequently cleaved by a protease are generally limited by the fact the expression of the peptides from the polyprotein is limited to a 1:1 ratio. However, in the present invention, the inclusion of an intracellular retention signal in the CAR means that its expression on the cell surface can be modulated (e.g. reduced compared to an equivalent control CAR or other transmembrane protein which does not comprise an intracellular retention signal). As such the ratio of the CAR which comprises the intracellular retention signal expressed on the cell surface compared to the expression of the second CAR expressed in the polyprotein may be, for example about 1:1.5, of from 1:1.5-1:2, 1:2-1:3, 1:3-1:4, 1:4-1:5, or more than 1:5.

The amount of a CAR expressed on the surface of a cell may be determined using methods which are known in the art, for example flow cytometry or fluorescence microscopy.

The intracellular retention signal may direct the CAR away from the secretory pathway during translocation from the ER.

The intracellular retention signal may direct the CAR to an intracellular compartment or complex. The intracellular retention signal may direct the CAR to a membrane-bound intracellular compartment.

For example, the intracellular retention signal may direct the CAR to a lysosomal, endosomal or Golgi compartment (trans-Golgi Network, 'TGN').

Within a normal cell, proteins arising from biogenesis or the endocytic pathway are sorted into the appropriate intracellular compartment following a sequential set of sorting decisions. At the plasma membrane, proteins can either remain at the cell surface or be internalised into endosomes. At the TGN, the choice is between going to the plasma membrane or being diverted to endosomes. In endosomes, proteins can either recycle to the plasma membrane or go to lysosomes. These decisions are governed by sorting signals on the proteins themselves.

Lysosomes are cellular organelles that contain acid hydrolase enzymes that break down waste materials and cellular debris. The membrane around a lysosome allows the digestive enzymes to work at the pH they require. Lysosomes fuse with autophagic vacuoles (phagosomes) and dispense their enzymes into the autophagic vacuoles, digesting their contents.

An endosome is a membrane-bounded compartment inside eukaryotic cells. It is a compartment of the endocytic membrane transport pathway from the plasma membrane to the lysosome and provides an environment for material to be sorted before it reaches the degradative lysosome. Endosomes may be classified as early endosomes, late endosomes, or recycling endosomes depending on the time it takes for endocytosed material to reach them. The intracellular retention signal used in the present invention may direct the protein to a late endosomal compartment.

The Golgi apparatus is part of the cellular endomembrane system, the Golgi apparatus packages proteins inside the cell before they are sent to their destination; it is particularly important in the processing of proteins for secretion.

There is a considerable body of knowledge which has arisen from studies investigating the sorting signals present in known proteins, and the effect of altering their sequence and/or position within the molecule (Bonifacino and Traub (2003) Ann. Rev. Biochem. 72:395-447; Braulke and Bonifacino (2009) Biochimica and Biophysica Acta 1793:605-614; Griffith (2001) Current Biology 11:R226-R228; Mellman and Nelson (2008) Nat Rev Mol Cell Biol. 9:833-845; Dell'Angelica and Payne (2001) Cell 106:395-398; Schafer et al (1995) EMBO J. 14:2424-2435; Trejo (2005) Mol. Pharmacol. 67:1388-1390). Numerous studies have shown that it is possible to insert one or more sorting signals into a protein of interest in order to alter the intracellular location of a protein of interest (Pelham (2000) Meth. Enzymol. 327:279-283).

It is therefore perfectly possible to select a sorting signal having a desired localisation property and include it within a protein of interest in order to direct the intracellular location of that protein. In connection with the present application, it is therefore possible to select a sorting signal having the desired amount of reduction of expression at the plasma membrane.

The optimal position of the sorting signal in the nascent protein of interest may depend on the type of transmembrane protein (i.e. types I-IV) and whether the C-terminus is on the luminal or the cytoplasmic side of the membrane (Goder and Spiess (2001) FEBS Lett 504:87-93). This may readily be determined by considering the position of the sorting signal in its natural protein.

Examples of endocytosis signals include those from the transferrin receptor and the asialoglycoprotein receptor.

Examples of signals which cause TGN-endosome recycling include those form proteins such as the CI- and CD-MPRs, sortilin, the LDL-receptor related proteins LRP3 and LRP10 and β-secretase, GGA1-3, LIMP-II, NCP1, mucolipn-1, sialin, GLUT8 and invariant chain.

Examples of TGN retention signals include those from the following proteins which are localized to the TGN: the prohormone processing enzymes furin, PC7, CPD and PAM; the glycoprotein E of herpes virus 3 and TGN38.

Examples of ER retention signals include C-terminal signals such as KDEL, KKXX or KXKXX and the RXR(R) motif of potassium channels. Known ER proteins include the adenovirus E19 protein and ERGIC53.

Examples of lysosomal sorting signals include those found in lysosomal membrane proteins, such as LAMP-1 and LAMP-2, CD63, CD68, endolyn, DC-LAMP, cystinosin, sugar phosphate exchanger 2 and acid phosphatase.

TUNABILITY

The relative expression of one or both CARs may be fine tuned using the methods described herein by various methods, such as

    a) altering the position of the intracellular retention signal in the protein molecule; and/or
    b) selecting a particular intracellular retention signal.

Option a) is discussed in more detail below.

With regard to option b), a range of intracellular retention signals is available from the large number of naturally occurring proteins which are sorted to distinct cellular locations inside eukaryotic cells. It is also possible to use "synthetic" intracellular retention signals which comprise one or more of the motifs found in naturally occurring proteins (see next section) and have a similar sorting signal function.

A cascade of signal strength is available, depending on the intracellular location to which the sorting signal sends the relevant protein. Broadly speaking, the more "intracellular" the location directed by the sorting signal, the "stronger" the signal is in terms of lowering the relative expression of the protein.

When a sorting signal directs a protein to the lysosomal compartment, the protein is internalised and degraded by the cell, resulting in relatively little escape to the cell surface. The protein is degraded and lost from the system once it enters the lysosome. Therefore lysosomal sorting signals, such as LAMP1, are the "strongest" in terms of reducing relative expression at the cell surface.

When a sorting signal directs a protein to be retained in the ER, only a very small proportion of the protein gets to the cell surface. Hence ER retention or recycling signals, such as ERGIC-53 and KKFF signal are the next most strong, in terms of reducing relative expression at the cell surface.

When a sorting signal directs a protein to the endosomal, Golgi or TGN compartments, then the protein is likely to recycle to some extent between the TGN, the endosomal compartment, and the plasma membrane. These signals provide a more limited level of reduction of expression as a significant proportion of the protein will still reach the plasma membrane.

In general the reduction in expression seen with known sorting signals can be summarised as follows: Lysosomal sorting signals>ER retention/recycling signals>TGN retention/recycling signals>endocytosis signals.

The tunability using different sorting signals and/or different positions of sorting signals within the protein is especially useful when one considers the expression of multiple proteins, each with their own relative expression. For example, consider a nucleic acid construct having the following structure:
A-X-B-Y-C in which

    A, B and C are nucleic acid sequences encoding polypeptides; and
    X and Y are nucleic acid sequences encoding cleavage sites.

The nucleic acid construct will encode three proteins *A*, *B* and *C*, any or all of which may be CARs. For example, *B* and *C* may be CARs which comprise an intracellular retention signal. If it is desired for A, B and C to be expressed such that the relative levels are *A>B>C*, then the nucleic acid sequence A may have no intracellular retention signal, the nucleic acid sequence B may have an intracellular retention signal that causes a small proportion of protein *B* to be retained in the cell (i.e. not to be expressed at the cell surface), and the nucleic acid sequence C may have an intracellular retention signal that causes a large proportion of protein *C* to be retained in the cell.

As explained below, differential amounts of intracellular retention, leading to different amounts of cell surface expression may be achieved by:

    (a) using different intracellular retention signals for the proteins; and/or

(b) having the intracellular retention signal located at a different position in the proteins.

SIGNAL TYPES

Numerous proteins which include an intracellular retention signal and are directed to an intracellular compartment are known in the art.

The intracellular retention signal may be a retention signal from a protein which resides in the lysosomal, endosomal or Golgi compartment.

Intracellular retention signals are well known in the art (see, for example, Bonifacino & Traub; Annu. Rev. Biochem.; 2003; 72; 395-447).

The intracellular retention signal may be a tyrosine-based sorting signal, a dileucine-based sorting signal, an acidic cluster signal, a lysosomal avoidance signal, an NPFX'(1,2)D-Type signal, a KDEL, a KKX'X' or a KX'KX'X' signal (wherein X' is any amino acid).

Tyrosine-based sorting signals mediate rapid internalization of transmembrane proteins from the plasma membrane and the targeting of proteins to lysosomes (Bonifacino & Traub; as above). Two types of tyrosine-based sorting signals are represented by the NPX'Y and YX'X'Z' consensus motifs (wherein Z' is an amino acid with a bulky hydrophobic side chain).

NPX'Y signals have been shown to mediate rapid internalization of type I transmembrane proteins, they occur in families such as members of the LDL receptor, integrin β, and β-amyloid precursor protein families.

Examples of NPX'Y signals are provided in Table 7.

Table 7 - NPX'Y signals

| Protein | Species | Sequence |
|---|---|---|
| LDL receptor | Human | Tm-10-INFD**NP**V**Y**QKTT-29 |
| LRP1 (1) | Human | Tm-21-VEIG**NP**T**Y**KMYE-64 |
| LRP1 (2) | Human | Tm-55-TNFT**NP**V**Y**ATLY-33 |
| LRP1 | *Drosophila* | Tm-43-GNFA**NP**V**Y**ESMY-38 |
| LRP1 (1) | *C. elegans* | Tm-54-TTFT**NP**V**Y**ELED-91 |
| LRP1 (2) | *C. elegans* | Tm-140-LRVD**NP**L**Y**DPDS-4 |
| Megalin (1) | Human | Tm-70-IIFE**NP**M**Y**SARD-125 |
| Megalin (2) | Human | Tm-144-TNFE**NP**I**Y**AQME-53 |
| Integrin 13-1 (1) | Human | Tm-18-DTGE**NP**I**Y**KSAV-11 |
| Integrin 13-1 (2) | Human | Tm-30-TTVV**NP**K**Y**EGK |
| Integrin 13 (1) | *Drosophila* | Tm-26-WDTE**NP**I**Y**KQAT-11 |
| Integrin 13 (2) | *Drosophila* | Tm-35-STFK**NP**M**Y**AGK |
| APLP1 | Human | Tm-33-HGYE**NP**T**Y**RFLE-3 |
| APP | Human | Tm-32-NGYE**NP**T**Y**KFFE-4 |
| APP-like | *Drosophila* | Tm-38-NGYE**NP**T**Y**KYFE-3 |
| Insulin receptor | Human | Tm--36-YASS**NP**E**Y**LSAS-379 |
| EGR receptor (1) | Human | Tm-434-GSVQ**NP**V**Y**HNQP-96 |
| EGR receptor (2) | Human | Tm-462-TAVG**NP**E**Y**LNTV-68 |
| EGR receptor (3) | Human | Tm-496-ISLD**NP**D**Y**QQDF-34 |

Numbers in parentheses indicate motifs that are present in more than one copy within the same protein. The signals in this and other tables should be considered examples. Key residues are indicated in bold type. Numbers of amino acids before (i.e., amino-terminal) and after (i.e., carboxy-terminal) the signals are indicated. Abbreviations: Tm, transmembrane; LDL, low density lipoprotein; LRP1, LDL receptor related protein 1; APP, 13-amyloid precursor protein; APLP1, APP-like protein 1.

YX'X'Z'-type signals are found in endocytic receptors such as the transferrin receptor and the asialoglycoprotein receptor, intracellular sorting receptors such as the CI- and CD-MPRs, lysosomal membrane proteins such as LAMP-1 and LAMP-2, and TGN proteins such as TGN38 and furin, as well as in proteins localized to specialized endosomal-lysosomal organelles such as antigen-processing compartments (e.g., HLA-DM) and cytotoxic granules (e.g., GMP-17). The YX'X'Z'-type signals are involved in the rapid internalization of proteins from the plasma membrane. However, their function is not limited to endocytosis, since the same motifs have been implicated in the targeting of transmembrane proteins to lysosomes and lysosome-related organelles.

Examples of YX'X'Z'-type signals are provided in Table 8.

Table 8 - YX'X'Z'-type signals

| Protein | Species | Sequence |
|---|---|---|
| LAMP-1 | Human | Tm-RKRSHAG**YQTI** |
| LAMP-2a | Human | Tm-KHHHAG**YEQF** |
| LAMP-2a | Chicken | Tm-KKHHNTG**YEQF** |
| LAMP-2b | Chicken | Tm-RRKSRTG**YQSV** |
| LAMP-2c | Chicken | Tm-RRKSYAG**YQTL** |
| LAMP | *Drosophila* | Tm-RRRSTSRG**YMSF** |
| LAMP | Earthworm | Tm-RKRSRRG**YESV** |
| CD63 | Human | Tm-KSIRSG**YEVM** |
| GMP-17 | Human | Tm-HCGGPRPG**YETL** |
| GMP-17 | Mouse | Tm-HCRTRRAE**YETL** |
| CD68 | Human | Tm-RRRPSA**YQAL** |
| CDlb | Human | Tm-RRRS**YQNIP** |
| CDlc | Human | Tm-KKHCS**YQDIL** |
| CD1d | Mouse | Tm-RRRSA**YQDIR** |
| CD1 | Rat | Tm-RKRRRS**YQDIM** |
| Endolyn | Rat | Tm-KFCKSKERN**YHTL** |
| Endolyn | *Drosophila* | Tm-KFYKARNERN**YHTL** |
| TSC403 | Human | Tm-KIRLRCQSSG**YQRI** |
| TSC403 | Mouse | Tm-KIRQRHQSSA**YQRI** |
| Cystinosin | Human | Tm-HFCLYRKRPG**YDQLN** |
| Putative solute carrier | Human | Tm-12-SLSRGSG**YKEI** |
| TRP-2 | Human | Tm-RRLRKG**YTPL**MET-11 |
| HLA-DM⌀ | Human | Tm-RRAGHSS**YTPL**PGS-9 |
| LmpA | Dictyostelium | Tm-KKLRQQKQQG**YQAI**INNE |
| Putative lysosomal protein | Dictyostelium | Tm-RSKSNQNQS**YNLI**QL |
| LIMP-II | Dictyostelium | Tm-RKTFYNNNQ**YNGY**NIIN |
| Transferrin receptor | Human | 16-PLS**YTRF**SLA-35-Tm |
| Asialoglycoprotein receptor H1 | Human | MTKE**YQDL**QHL-29-Tm |
| CI-MPR | Human | Tm-22-SYK**YSKV**NKE-132 |
| CD-MPR | Human | Tm-40-PAA**YRGV**GDD-16 |
| CTLA-4 | Human | Tm-10-TGV**YVKM**PPT-16 |
| Furin | Human | Tm-17-LIS**YKGL**PPE-29 |
| TGN38 | Rat | Tm-23-ASD**YQRL**NLKL |
| gp41 | HIV-1 | Tm-13-RQG**YSPL**SFQT-144 |
| Acid phosphatase | Human | Tm-RMQAQPPG**YRHV**ADGEDHA |
| See legend to Table 7 for explanation of signal format | | |

Dileucine-based sorting signals ([DE]X'X'X'LL[LI]) play critical roles in the sorting of many type I, type II, and multispanning transmembrane proteins. Dileucine-based sorting signals are involved in rapid internalization and lysosomal degradation of transmembrane proteins and the targeting of proteins to the late endosomal-lysosomal compartments. Transmembrane proteins that contain constitutively active forms of this signal are mainly localised to the late endosomes and lysosomes.

Examples of [DE]X'X'X'LL[LI] sorting signals are provided in Table 9.

Table 9 - [DE]X'X'X'LL[LI] sorting signals

| Protein | Species | Signal |
|---|---|---|
| CD3-Y | Human | Tm-8-S**D**KQT**LL**PN-26 |

(continued)

| Protein | Species | Signal |
|---|---|---|
| LIMP-II | Rat | Tm-11-D**E**RAPL**I**RT |
| Nmb | Human | Tm-37-Q**E**KDP**LL**KN-7 |
| QNR-71 | Quail | Tm-37-T**E**RNP**LL**KS-5 |
| Pmel17 | Human | Tm-33-G**E**NSP**LL**SG-3 |
| Tyrosinase | Human | Tm-8-E**E**KQP**LL**ME-12 |
| Tyrosinase | Medaka fish | Tm--16--G**E**RQP**LL**QS--13 |
| Tyrosinase | Chicken | Tm-8-P**E**IQP**LL**TE-13 |
| TRP-1 | Goldfish | Tm-7-E**G**RQP**LL**GD-15 |
| TRP-1 | Human | Tm-7-E**A**NQP**LL**TD-20 |
| TRP-1 | Chicken | Tm-7-E**L**HQP**LL**TD-20 |
| TRP-2 | Zebrafish | Tm-5-R**E**FEP**LL**NA-11 |
| VMAT2 | Human | Tm-6-E**E**KMA**IL**MD-29 |
| VMAT1 | Human | Tm-6-E**E**KLA**IL**SQ-32 |
| VAchT | Mouse | Tm-10-S**E**RDV**LL**DE-42 |
| VAMP4 | Human | 19-S**E**RRN**LL**ED-88-Tm |
| Neonatal FcR | Rat | Tm-16-D**D**SGD**LL**PG-19 |
| CD4 | Human | Tm-12-S**Q**IKR**LL**SE-17 |
| CD4 | Cat | Tm-12-S**H**IKR**LL**SE-17 |
| GLUT4 | Mouse | Tm-17-R**R**TPS**LL**EQ-17 |
| GLUT4 | Human | Tm-17-H**R**TPS**LL**EQ-17 |
| IRAP | Rat | 46-EP**R**GSR**LL**VR-53-Tm |
| Ii | Human | MD**D**QRD**LI**SNNEQLP**ML**GR-11-Tm |
| Ii | Mouse | MD**D**QRD**LI**SNHEQLPI**L**GN-10-Tm |
| Ii | Chicken | MAE**E**QRD**LI**SSDGSSG**VL**PI-12-Tm |
| Ii-1 | Zebrafish | MEPDH**Q**NES**LI**QRVPSAET**IL**GR-12-Tm |
| Ii-2 | Zebrafish | MSSEG**N**ETP**LI**SDQSSVNMGPQP-8-Tm |
| Lamp | Trypanosome | Tm-RPRRRT**E**EDE**LL**PEEAEG**LI**DPQN |
| Menkes protein | Human | Tm-74-P**D**KHS**LL**VGDFREDDDTAL |
| NPC1 | Human | Tm-13-T**E**RER**LL**NF |
| AQP4 | Human | Tm-32-V**E**TDD**LI**L-29 |
| RME-2 | *C. elegans* | Tm-104-F**E**NDS**LL** |
| Vam3p | *S. cerevisiae* | 153-N**E**QSP**LL**HN-121-Tm |
| ALP | *S. cerevisiae* | 7-S**E**QTR**LV**P-18-Tm |
| Gap1p | *S. cerevisiae* | Tm-23-E**V**QLD**LL**K-24 |

See legend to Table 7 for explanation of signal format.

DX'X'LL signals constitute a distinct type of dileucine-based sorting signals. These signals are present in several transmembrane receptors and other proteins that cycle between the TGN and endosomes, such as the Cl- and CD-MPRs, sortilin, the LDL-receptor-related proteins LRP3 and LRP10, and β-secretase.

Examples of DX'X'LL sorting signals are provided in Table 10.

Table 10 - DX'X'LL sorting signals

| Protein | Species | Sequence |
|---|---|---|
| CI-MPR | Human | Tm-151-SFHDDS**D**ED**LL**HI |
| CI-MPR | Bovine | Tm-150-TFHDDS**D**ED**LL**HV |
| CI-MPR | Rabbit | Tm-151-SFHDDS**D**ED**LL**NI |
| CI-MPR | Chicken | Tm-148-SFHDDS**D**ED**LL**NV |
| CD-MPR | Human | Tm-54-EESEER**D**DH**LL**PM |
| CD-MPR | Chicken | Tm-54-DESEER**D**DH**LL**PM |

(continued)

| Protein | Species | Sequence |
|---|---|---|
| Sortilin | Human | Tm-41-GYHDDS**D**ED**LL**E |
| SorLA | Human | Tm-41-ITGFSD**D**VP**MV**IA |
| Head-activator BP | Hydra | Tm-41-INRFSD**D**EP**LVV**A |
| LRP3 | Human | Tm-237-MLEASD**D**EA**LL**VC |
| ST7 | Human | Tm-330-KNETSD**D**EA**LL**LC |
| LRP10 | Mouse | Tm-235-WVVEAE**D**EP**LL**A |
| LRP10 | Human | Tm-237-WVAEAE**D**EP**LL**T |
| Beta-secretase | Human | Tm-9-HDDFAD**D**IS**LL**K |
| Mucolipin-1 | Mouse | Tm-43-GRDSPE**D**HS**LL**VN |
| Nonclassical MHC-I | Deer mouse | Tm-6-VRCHPE**D**DR**LL**G |
| FLJ30532 | Human | Tm-83-HRVSQD**D**LD**LL**TS |
| GGA1 | Human | 350-ASVSLL**D**DEL**M**SL-275 |
| GGA1 | Human | 415-ASSGLD**D**LD**LL**GK-211 |
| GGA2 | Human | 408-VQNPSA**D**RN**LL**DL-192 |
| GGA3 | Human | 384-NALSWL**D**EE**LL**CL-326 |
| GGA | *Drosophila* | 447-TVDSID**D**VP**LL**SD-116 |

See legend to Table 7 for explanation of signal format. Serine and threonine residues are underlined.

Another family of sorting motifs is provided by clusters of acidic residues containing sites for phosphorylation by CKII. This type of motif is often found in transmembrane proteins that are localized to the TGN at steady state, including the prohormone-processing enzymes furin, PC6B, PC7, CPD, and PAM, and the glycoprotein E of herpes virus 3.

Examples of acidic cluster signals are provided in Table 11.

Table 11 - Acidic cluster sorting signals

| Protein | Species | Sequence |
|---|---|---|
| Furin | Mouse | Tm-31-Q**EE**CPS**D**S**EEDE**G-14 |
| PC6B (1)[a] | Mouse | Tm-39-R**D**RD**Y**D**EDDEDD**I-36 |
| PC6B (2) | Mouse | Tm-69-L**DETEDDE**LEY**DDE**S-4 |
| PC7 | Human | Tm-38-K**D**P**D**EVE**TE**S-47 |
| CPD | Human | Tm-36-H**E**FQ**DETD**T**EEE**T-6 |
| PAM | Human | Tm-59-Q**EKEDD**GS**ESEEE**Y-12 |
| VMAT2 | Human | Tm-35-G**EDEE**S**E**S**D** |
| VMAT1 | Human | Tm-35-G**E**DS**DEE**P**D**HEE |
| VAMP4 | Human | 25-L**EDD**S**DEEED**F-81-Tm |
| Glycoprotein B | HCMV | Tm-125-K**D**S**DEEE**NV |
| Glycoprotein E | Herpes virus 3 | Tm-28-F**ED**S**E**S**TD**T**EEE**F-21 |
| Nef | HIV-1 (AAL65476) | 55-L**E**AQ**EEEE**V-139 |
| Kex1p (1) | *S. cerevisiae* | Tm-29-A**DD**L**E**SGLGA**EDD**L**E**Q**DE**QL**E**G-40 |
| Kex1p (2) | *S. cerevisiae* | Tm-79-T**E**I**DE**S**FE**MT**D**F |
| Kex2p | *S. cerevisiae* | Tm-36-T**E**P**EE**V**ED**F**D**F**D**LS**DED**H-61 |
| Vps10p | *S. cerevisiae* | Tm-112-F**E**I**EEDD**VPT**L**EEE**H-37 |

See legend to Table 7 for explanation of signal format. Serine and threonine residues are underlined. "The number in parentheses is the motif number.

The KDEL receptor binds protein in the ER-Golgi intermediate compartment, or in the early Golgi and returns them to the ER. Although the common mammalian signal is KDEL, it has been shown that the KDEL receptor binds the sequence HDEL more tightly (Scheel et al; J. Biol. Chem. 268; 7465 (1993)). The intracellular retention signal may be HDEL.

KKX'X' and KX'KX'X' signals are retrieval signals which can be placed on the cytoplasmic side of a type I membrane protein. Sequence requirements of these signals are provided in detail by Teasdale & Jackson (Annu. Rev. Cell Dev. Biol.; 12; 27 (1996)).

The intracellular retention signal may be selected from the group of: NPX'Y, YX'X'Z, [DE]X'X'X'L[LI], DX'X'LL, DP[FW], FX'DX'F, NPF, LZX'Z[DE], LLDLL, PWDLW, KDEL, HDEL, KKX'X' or KX'KX'X'; wherein X' is any amino acid and Z' is an amino acid with a bulky hydrophobic side chain.

The intracellular retention signal may be any sequence shown in Tables 7 to 11.

The intracellular retention signal may comprise the Tyrosinase-related protein (TYRP)-1 intracellular retention signal. The intracellular retention signal may comprise the TYRP-1 intracellular domain. The intracellular retention signal may comprise the sequence NQPLLTD (SEQ ID No. 1).

TYRP1 is a well-characterized melansomal protein which is retained in the melanosome (a specialized lysosome) at >99% efficiency. TYRP1 is a 537 amino acid transmembrane protein with a lumenal domain (1-477aa), a transmembrane domain (478-501), and a cytoplasmic domain (502-537). A di-leucine signal residing on the cytoplasmic domain causes retention of the protein. This di-leucine signal has the sequence shown as SEQ ID No. 1 (NQPLLTD).

The intracellular retention signal may be in the endodomain of the CAR. In other words, the intracellular retention signal may be in the domain of the transmembrane protein which would be on the intracellular side of the cell membrane if the protein was correctly expressed at the cell surface.

The intracellular retention signal may be proximal to the transmembrane domain, for instance being immediately connected to it. The intracellular retention signal may be distal to the transmembrane domain - for instance at the carboxy-terminus of the endodomain. The positioning of the retention signal modulates its activity allowing "tuning" of the relative expression of two proteins. For instance in the case of the TYRP1 di-leucine motif, proximal placement results in low-level surface expression, while distal placement results in intermediate surface expression, as shown in the Examples.

POLYPEPTIDE OF INTEREST

Any or all of A or B; or A, B or C of the nucleic acid sequences in the constructs defined herein may encode a CAR which may or may not comprise an intracellular retention signal and/or an altered signal peptide.

The nucleic acid construct may comprise one or more further nucleic acid sequence(s) which encode polypeptide of interest (POIs). For example, the POI(s) may be an intracellular protein such as a nucleic protein, a cytoplasmic protein or a protein localised to a membrane-bound compartment; a secretory protein or a transmembrane protein.

The POI may be a suicide gene and/or a marker gene.

SUICIDE/MARKER GENE

A suicide gene is a gene encoding a polypeptide which, when expressed by a cell enables that cell to be deleted.

A marker gene is a gene encoding a polypeptide which enables selection of a cell expressing that polypeptide.

Various suicide and marker genes are known in the art. WO2013/153391 describes compact polypeptide which comprises both a marker moiety and a suicide moiety. The polypeptide may be co-expressed with a therapeutic transgene, such as a gene encoding a CAR.

The marker moiety comprises a minimal epitope of CD34 which allows efficient selection of transduced cells using, for example, the Miltenyi CD34 cliniMACS system.

The suicide moiety comprises a minimal epitope based on the epitope from CD20. Cells expressing a polypeptide comprising this sequence can be selectively killed using a lytic antibody such as Rituximab.

The combined marker and suicide polypeptide is stably expressed on the cell surface after, for example, retroviral transduction of its encoding sequence.

The marker/suicide polypeptide may have the formula:

St-R1-S1-Q-S2-R2 wherein

St is a stalk sequence which, when the polypeptide is expressed at the surface of a target cell, causes the R and Q epitopes to be projected from the cell surface;

R1 and R2 are a Rituximab-binding epitopes;

S1 and S2 are optional spacer sequences, which may be the same or different; and

Q is a QBEnd10-binding epitope.

The polypeptide may comprise the sequence shown as SEQ ID No.17, or a variant thereof which has at least 80% identity with the sequence shown as SEQ ID No. 17 and which (i) binds QBEND10; (ii) binds Rituximab and (iii) when expressed on the surface of a cell, induces complement-mediated killing of the cell in the presence of Rituximab.

CPYSNPSLCSGGGGGSELPTQGTFSNVSTNVSPAKPTTTACPYSNPSLCSGGGGGSPAPRPP
TPAPTIASQPLSLRPEACRPAAGGAVHTRGLDFACDIYIWAPLAGTCGVLLLSLVITLYCNHR
NRRRVCKCPRPVV (SEQ ID No. 17)

Described herein is a method for deleting a cell which expresses such a marker/suicide gene, which comprises the step of exposing the cells to rituximab.

CELL

The present invention relates to a cell which co-expresses a first CAR and a second CAR at the cell surface. The cell expresses a nucleic acid construct according to the first aspect of the invention.

The cell may be any eukaryotic cell capable of expressing a CAR at the cell surface, such as an immunological cell.

In particular the cell may be an immune effector cell such as a T cell or a natural killer (NK) cell.

T cells or T lymphocytes are a type of lymphocyte that play a central role in cell-mediated immunity. They can be distinguished from other lymphocytes, such as B cells and natural killer cells (NK cells), by the presence of a T-cell receptor (TCR) on the cell surface. There are various types of T cell, as summarised below.

Helper T helper cells (TH cells) assist other white blood cells in immunologic processes, including maturation of B cells into plasma cells and memory B cells, and activation of cytotoxic T cells and macrophages. TH cells express CD4 on their surface. TH cells become activated when they are presented with peptide antigens by MHC class II molecules on the surface of antigen presenting cells (APCs). These cells can differentiate into one of several subtypes, including TH1, TH2, TH3, TH17, Th9, or TFH, which secrete different cytokines to facilitate different types of immune responses.

Cytotoxic T cells (TC cells, or CTLs) destroy virally infected cells and tumor cells, and are also implicated in transplant rejection. CTLs express the CD8 at their surface. These cells recognize their targets by binding to antigen associated with MHC class I, which is present on the surface of all nucleated cells. Through IL-10, adenosine and other molecules secreted by regulatory T cells, the CD8+ cells can be inactivated to an anergic state, which prevent autoimmune diseases such as experimental autoimmune encephalomyelitis.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - naturally occurring Treg cells and adaptive Treg cells.

Naturally occurring Treg cells (also known as CD4+CD25+FoxP3+ Treg cells) arise in the thymus and have been linked to interactions between developing T cells with both myeloid (CD11c+) and plasmacytoid (CD123+) dendritic cells that have been activated with TSLP. Naturally occurring Treg cells can be distinguished from other T cells by the presence of an intracellular molecule called FoxP3. Mutations of the FOXP3 gene can prevent regulatory T cell development, causing the fatal autoimmune disease IPEX.

Adaptive Treg cells (also known as Tr1 cells or Th3 cells) may originate during a normal immune response.

The T cell of the invention may be any of the T cell types mentioned above, in particular a CTL.

Natural killer (NK) cells are a type of cytolytic cell which forms part of the innate immune system. NK cells provide rapid responses to innate signals from virally infected cells in an MHC independent manner

NK cells (belonging to the group of innate lymphoid cells) are defined as large granular lymphocytes (LGL) and constitute the third kind of cells differentiated from the common lymphoid progenitor generating B and T lymphocytes. NK cells are known to differentiate and mature in the bone marrow, lymph node, spleen, tonsils and thymus where they then enter into the circulation.

The CAR cells of the invention may be any of the cell types mentioned above.

CAR- expressing cells, such as CAR-expressing T or NK cells may either be created *ex vivo* either from a patient's own peripheral blood (1st party), or in the setting of a haematopoietic stem cell transplant from donor peripheral blood (2nd party), or peripheral blood from an unconnected donor (3rd party).

The present invention also provide a cell composition comprising CAR-expressing cells, such as CAR-expressing T and/orNK cells, according to the present invention. The cell composition may be made by transducing a blood-sample *ex vivo* with a nucleic acid construct according to the present invention.

Alternatively, CAR-expressing cells may be derived from *ex vivo* differentiation of inducible progenitor cells or embryonic progenitor cells to the relevant cell type, such as T cells. Alternatively, an immortalized cell line such as a T-cell line which retains its lytic function and could act as a therapeutic may be used.

In all these embodiments, CAR cells may be generated by introducing DNA or RNA coding for the CARs by one of many means including transduction with a viral vector, transfection with DNA or RNA.

A CAR T cell of the invention may be an *ex vivo* T cell from a subject. The T cell may be from a peripheral blood mononuclear cell (PBMC) sample. T cells may be activated and/or expanded prior to being transduced with CAR-encoding nucleic acid, for example by treatment with an anti-CD3 monoclonal antibody.

A CAR T cell of the invention may be made by:

(i) isolation of a T cell-containing sample from a subject or other sources listed above; and
(ii) transduction or transfection of the T cells with a nucleic acid construct encoding the first and second CAR.

The T cells may then by purified, for example, selected on the basis of co-expression of the first and second CAR.

VECTOR

The present invention also provides a vector which comprises a CAR-encoding nucleic acid construct as defined herein. Such a vector may be used to introduce the nucleic acid sequence(s) into a host cell so that it expresses the first and second CARs.

The vector may, for example, be a plasmid or a viral vector, such as a retroviral vector or a lentiviral vector, or a transposon based vector or synthetic mRNA.

The vector may be capable of transfecting or transducing a T cell.

PHARMACEUTICAL COMPOSITION

The present invention also relates to a pharmaceutical composition containing a plurality of CAR-expressing cells, such as T cells or NK cells, of the invention. The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

METHOD OF TREATMENT

The cells of the present invention may be capable of killing target cells, such as cancer cells. The target cell may be recognisable by a defined pattern of antigen expression, for example the expression of antigen A AND antigen B.

The cells of the present invention may be used for the treatment of an infection, such as a viral infection.

The cells of the invention may also be used for the control of pathogenic immune responses, for example in autoimmune diseases, allergies and graft-vs-host rejection.

The cells of the invention may be used for the treatment of a cancerous disease, such as bladder cancer, breast cancer, colon cancer, endometrial cancer, kidney cancer (renal cell), leukemia, lung cancer, melanoma, non-Hodgkin lymphoma, pancreatic cancer, prostate cancer and thyroid cancer.

It is particularly suited for treatment of solid tumours where the availability of good selective single targets is limited.

The cells of the invention may be used to treat: cancers of the oral cavity and pharynx which includes cancer of the tongue, mouth and pharynx; cancers of the digestive system which includes oesophageal, gastric and colorectal cancers; cancers of the liver and biliary tree which includes hepatocellular carcinomas and cholangiocarcinomas; cancers of the respiratory system which includes bronchogenic cancers and cancers of the larynx; cancers of bone and joints which includes osteosarcoma; cancers of the skin which includes melanoma; breast cancer; cancers of the genital tract which include uterine, ovarian and cervical cancer in women, prostate and testicular cancer in men; cancers of the renal tract which include renal cell carcinoma and transitional cell carcinomas of the utterers or bladder; brain cancers including gliomas, glioblastoma multiforme and medullobastomas; cancers of the endocrine system including thyroid cancer, adrenal carcinoma and cancers associated with multiple endocrine neoplasm syndromes; lymphomas including Hodgkin's lymphoma and non-Hodgkin lymphoma; Multiple Myeloma and plasmacytomas; leukaemias both acute and chronic, myeloid or lymphoid; and cancers of other and unspecified sites including neuroblastoma.

Treatment with the cells of the invention may help prevent the escape or release of tumour cells which often occurs with standard approaches.

The invention will now be further described by way of Examples, which are meant to serve to assist one of ordinary skill in the art in carrying out the invention and are not intended in any way to limit the scope of the invention.

EXAMPLES

**Example 1** - Creation of target cell populations

For the purposes of proving the principle of the invention, receptors based on anti-CD19 and anti-CD33 were arbitrarily chosen. Using retroviral vectors, CD19 and CD33 were cloned. These proteins were truncated so that they do not signal and could be stably expressed for prolonged periods. Next, these vectors were used to transduce the SupT1 cell line either singly or doubly to establish cells negative for both antigen (the wild-type), positive for either and positive for both. The expression data are shown in Figure 3.

**Example 2** - Design and function of the AND gate

The AND gate combines a simple activating receptor with a receptor which basally inhibits activity, but whose inhibition is turned off once the receptor is ligated. This was achieved by combining a standard 1st generation CAR with a short / non-bulky CD8 stalk spacer and a CD3 Zeta endodomain with a second receptor with a bulky Fc spacer whose endodomain contained either CD148 or CD45 endodomains. When both receptors are ligated, the difference in spacer dimensions results in isolation of the different receptors in different membrane compartments, releasing the CD3 Zeta receptor from inhibition by the CD148 or CD45 endodomains. In this way, activation only occurs once both receptors are activated. CD148 and CD45 were chosen for this as they function in this manner natively: for instance, the very bulky CD45 ectodomain excludes the entire receptor from the immunological synapse. The expression cassette is depicted in Figure 4 and the subsequent proteins in Figure 5.
Surface staining for the different specificity showed that both receptor pairs could be effectively expressed on the cell surface shown in Figure 6. Function in BW5147 shows that the T-cell is only activated in the presence of both antigens (Figure 7).

**Example 3:** Demonstration of Generalizability of the AND gate

To ensure that the observations were not a manifestation of some specific characteristic of CD19 / CD33 and their binders which had been used, the two targeting scFvs were swapped such that now, the activation (ITAM) signal was transmitted upon recognition of CD33, rather than CD19; and the inhibitory (CD148) signal was transmitted upon recognition of CD19, rather than of CD33. Since CD45 and CD148 endodomains are considered to be functionally similar, experimentation was restricted to AND gates with CD148 endodomain. This should still result in a functional AND gate. T-cells expressing the new logic gate where challenged with targets bearing either CD19 or CD33 alone, or both. The T-cells responded to targets expressing both CD19 and CD33, but not to targets expressing only one or none of these antigens. This shows that the AND gate is still functional in this format (Figure 8B).
On the same lines, it was sought to establish how generalizable our AND gate is: the AND gate should be generalizable across different targets. While there may be lesser or greater fidelity of the gate given relative antigen density, cognate scFv binding kinetics and precise distance of the scFv binding epitope, one would expect to see some AND gate manifestations with a wide set of targets and binders. To test this, three additional AND gates were generated. Once again, experimentation was restricted to the CD148 version of the AND gate. The second scFv from the original CD148 AND gate was replaced with the anti-GD2 scFv huK666, or with the anti-CD5 scFv, or the anti-EGFRvIII scFv MR1.1 to generate the following CAR AND gates: CD19 AND GD2; CD19 AND CD5; CD19 AND EGFRvIII. The following artificial antigen expressing cell lines were also generated: by transducing SupT1, and our SupT1.CD19 with GM3 and GD2 synthases SupT1.GD2 and SupT1.CD19.GD2 were generated. By transducing SupT1 and SupT1.CD19 with a retroviral vector coding for EGFRvIII SupT1.EGFRvIII and SupT1.CD19.EGFRvIII were generated. Since CD5 is expressed on SupT1 cells, a different cell line was used to generate the target cells: 293T cells were generated which express CD19 alone, CD5 alone and both CD5 and CD19 together. Expression was confirmed by flow-cytometry (Figure 9). T-cells expressing the three new CAR AND gates were challenged with SupT1.CD19 and respective cognate double positive and single positive target cells. All three AND gates demonstrated reduced activation by the double positive cell lines in comparison with the single positive targets (Figure 10). This demonstrates generalizability of the AND gate design to arbitrary targets and cognate binders.

**Example 4:** Experimental proof of Kinetic segregation model of CAR AND gate

The aim was to prove the model that differential segregation caused by different spacers is the central mechanism behind the ability to generate these logic CAR gates. The model is that if only the activating CAR is ligated, the potent inhibiting 'ligation off' type CAR is in solution in the membrane and can inhibit the activating CAR. Once both CARs are ligated, if both CAR spacers are sufficiently different, they will segregate within the synapse and not co-localize. Hence, a key requirement is that the spacers are sufficiently different. If the model is correct, if both spacers are sufficiently similar so they co-localize when both receptors are ligated, the gate will fail to function. To test this, the "bulky" Fc spacer in the original CAR we replaced with a murine CD8 spacer. It was predicted that this has the similar length, bulk and charge as human CD8 but so should not cross-pair with it. Hence, the new gate had a first CAR which recognizes CD19, a human CD8 stalk spacer and an activatory endodomain; while the second CAR recognizes CD33, has a mouse CD8 stalk spacer and a CD148 endodomain (Figure 8C). T-cells were transduced to express this new CAR gate. These T-cells were then challenged with SupT1 cells expressing CD19 alone, CD33 alone or CD19 and CD33 together. T-cells did not respond to SupT1 cells expressing either antigen alone as per the original AND gate. However, CAR T-cells failed to respond to SupT1 cells expressing both antigens, thereby confirming the model (Figure 8C). A functional AND gate requires both CARs to have spacers sufficiently different so that they do not co-localize within an immunological synapse (Figure 11A and B).

**Example 5:** Functional analysis of the AND gate in primary cells

PBMCs were isolated from blood and stimulated using PHA and IL-2. Two days later the cells were transduced on retronectin coated plates with retro virus containing the CD19:CD33 AND gate construct. On day 5 the expression level of the two CARs translated by the AND gate construct was evaluated via flow cytometry and the cells were depleted of CD56+ cells (predominantly NK cells). On day 6 the PBMCs were placed in a co-culture with target cells at a 1:2 effector to target cell ratio. On day 8 the supernatant was collected and analysed for IFN-gamma secretion via ELISA (Figure 12).
These data demonstrate that the AND gate functions in primary cells.

**Example** 6 - Dissection of TYRP1 lysozomal retention signals

The ability of the Tyrosinase-related protein 1 (TYRP1) retention signal to cause retention of a polypeptide when in the context of a more complex endodomain was determined using a number of constructs (Figure 16). The wild-type construct was compared with constructs where enhanced Green Fluorescent Protein (eGFP) was added or replaced the TYRP1 endodomain. Where eGFP was added, it was placed either after or before the native endodomain so the retention signal was either in its native location (just under the membrane), or distal to it.
All constructs are co-expressed with IRES.CD34. Staining of transduced SupT1 cells is shown with intracellular and surface staining in Figure 16.
It was found that replacement of the endodomain resulted in very bright surface expression, introduction of eGFP after the retention signal to almost no surface expression and introduction before the retention signal to intermediate surface expression (Figure 16).

**Example** 7 - Modulation of the relative expression of a transmembrane protein co-expressed from a single expression cassette with a separate protein

An expression cassette encoding two CAR transmembrane proteins was modified such that one of the CAR proteins had the lysozomal retention signal from TYRP1 introduced either proximal or distal to the membrane. Expression of each of these two new variants at the cell surface was compared with that of the original unmodified CAR protein.
PBMCs were isolated from blood and stimulated using PHA and IL-2. Two days later the cells were transduced on retronectin coated plates with retro virus containing the CD19:CD33 CAR construct. On day 5 the expression level of the two CARs translated by the construct was evaluated via flow cytometry and the cells were depleted of CD56+ cells (predominantly NK cells). On day 6 the PBMCs were placed in a co-culture with target cells at a 1:2 effector to target cell ratio. On day 8 the supernatant was collected and analysed for IFN-gamma secretion via ELISA.
The pattern observed with Tyrp1-eGFP fusions was observed with some reduction of expression of modified transmembrane protein with the distal retention signal and marked reduction in the case of proximal retention signal. As expected, expression of the second transmembrane protein from the cassette was not altered (Figure 17).

**Example 8** - Modulation of expression using a retention signal from the Adenoviral E3/19K protein

The human adenovirus E3/19K protein is a type I transmembrane glycoprotein of the Endoplasmic Reticulum/Golgi that abrogates cell surface transport of major histocompatibility complex class I (MHC-I) and MHC-I-related chain A and B (MICA/B) molecules. The retention motif was identified to be depended on the cytosolic tail of the adenovirus E3/19K protein. More specifically, the last 6aa DEKKMP was found to be the most important for retention. The optimal positioning was found to be at the c-terminus of the protein.

An expression cassette encoding two CAR transmembrane proteins, as described in Example 7, was modified such that one of the CAR proteins had the retention motif from adenovirus E3/19K protein. In this experiment, the retention motif on the second CAR in the expression cassette (the anti-CD33 inhibitory CAR).

Constructs were generated comprising either the entire cytosolic tail of adenovirus E3/19K protein or only the last 6aa from E3/19K (DEKKMP), which were found to be critical for its Golgi/ER retention ability (Figure 18). These constructs were transfected into 293T cells and stained primarily with a chimeric soluble CD19-Rabbit Fc and a chimeric soluble CD33-Mouse Fc proteins. These cells were then subsequently stained with an anti-Rabbit Fc-FITC and an anti-Mouse Fc-APC (Figure 19). These cells show a clear retention when the full length adenovirus E3/19K protein, or the DEKKMP motif, was placed on the anti-CD33 receptor but had no effect on anti-CD19 receptor expression levels.

**Example 9** - Modulation of expression by altering the signal peptide - swapping in the murine Ig kappa chain V-III signal sequence

PCT/GB2014/053452 describes a vector system encoding two chimeric antigen receptors (CARs), one against CD19 and one against CD33. The signal peptide used for the CARs in that study was the signal peptide from the human CD8a signal sequence. For the purposes of this study, this was substituted with the signal peptide from the murine Ig kappa chain V-III region, which has the sequence: METD**TLILWVLLLLV**PGSTG (hydrophobic residues hightlited in bold). In order to establish that the murine Ig kappa chain V-III signal sequence functioned as well as the signal sequence from human CD8a, a comparative study was performed. For both signal sequences, functional expression of the anti-CD33 CAR and the anti-CD19 CAR was observed. This substituted signal sequence and all subsequent mutations thereof were transiently transfected into 293T cells. Three days after transfection the 293T cells were stained with both soluble chimeric CD19 fused with rabbit Fc chain and soluble chimeric CD33 fused with mouse Fc chain. All cells were then stained with anti-Rabbit Fc-FITC and anti-mouse Fc-APC. Flow cytometry plots show the substituted signal sequence as a comparison with non-transfected (NT) and the construct with Cd8 signal sequences (Figure 22). The murine Ig kappa chain V-III signal sequence was found to function as well as the signal sequence from human CD8a.

**Example 10** - Altering relative expression by deleting hydrophobic residues in the signal peptide.

Hydrophobic residues were deleted in a stepwise fashion and the effect on the relative expression of the anti-CD33 CAR and the anti-CD19 CAR was observed. The effect of one, two, three and four amino acid deletions was investigated and the results are shown in Figures 23 to 26 respectively.

All mutant constructs showed a decrease in relative expression of the anti-CD19 CAR compared to the anti-CD33 CAR. The relative decrease of anti-CD19 CAR expression was greater with a greater number of amino acid deletions from 1 to 3, but then plateaued out (four deletions gave a similar decrease in expression as three deletions). Although the invention has been described in connection with specific preferred embodiments, it should be understood that the invention as claimed should not be unduly limited to such specific embodiments.

**Claims**

1. A nucleic acid construct comprising the following structure:

   A-X-B

   in which

   X is a nucleic acid sequence which encodes a cleavage site; and
   A and B are nucleic acid sequences encoding a first and a second chimeric antigen receptor (CAR), each CAR comprising:

   (i) an antigen-binding domain;
   (ii) a spacer
   (iii) a trans-membrane domain; and
   (iv) an endodomain

   wherein the antigen binding domains of the first and second CARs bind to different antigens, wherein the spacer of the first CAR has a different size to the spacer of the second CAR such that when the first CAR and the second CAR bind their respective target antigens at the cell surface, the first CAR and second CAR become spatially separated on the cell membrane; wherein one of the first or second CARs is an activating CAR comprising an activating endodomain and the other CAR is an inhibitory CAR comprising a ligation-off inhibitory endodomain, which inhibitory CAR inhibits T-cell activation by the activating CAR in the absence of inhibitory CAR ligation, but does not significantly inhibit T-cell activation by the activating CAR when the inhibitory CAR is ligated; and wherein:

   (a) the first and/or second CAR comprises an intracellular retention signal; and/or
   (b) the signal peptide of the first or second CAR comprises one or more mutation(s) such that it has fewer hydrophobic amino acids.

2. A nucleic acid construct according to claim 1, wherein the first and/or second CAR comprises an intracellular retention signal which directs the CAR to a lysozomal, endosomal or Golgi compartment.

3. A nucleic acid construct according to claim 1, wherein the first and/or second CAR comprises an intracellular retention signal selected from the following group: an endocytosis signal; a Golgi retention signal; a trans-Golgi network (TGN) recycling signal; an endoplasmic reticulum (ER) retention signal; and a lysosomal sorting signal.

4. A nucleic acid construct according to claim 1, wherein the signal peptide of the first or second CAR comprises one or more mutation(s) such that it has fewer hydrophobic amino acids than a) its wild-type sequence or b) the signal peptide of the other CAR.

5. A nucleic acid construct according to claim 4, wherein the signal peptide of the activating CAR comprises one or more mutation(s) such that it has fewer hydrophobic amino acids, such that when the nucleic acid construct is expressed in a cell, the relative expression level of the activating CAR at the cell surface is reduced in comparison with the inhibitory CAR.

6. A nucleic acid construct according to claim 4, wherein the hydrophobic amino acid(s) removed or replaced by mutation is/are selected from the group: Alanine (A); Valine (V); Isoleucine (I); Leucine (L); Methionine (M); Phenylalanine (P); Tyrosine (Y); Tryptophan (W).

7. A nucleic acid construct according to claim 4, wherein the hydrophobic amino acid(s) removed or replaced by mutation is/are selected from the group: Valine (V); Isoleucine (I); Leucine (L); and Tryptophan (W).

8. A nucleic acid construct according to claim 1, wherein the signal peptide of the first and second CAR differ in their number of hydrophobic amino acids and wherein one signal peptide comprises up to five more hydrophobic amino acids than the other signal peptide.

**9.** A vector comprising a nucleic acid construct according to any preceding claim.

**10.** A cell comprising a nucleic acid construct according to any one of claims 1 to 8 or a vector according to claim 9.

**11.** A method for making a cell according to claim 10, which comprises the step of introducing: a nucleic acid construct according to any of claims 1 to 8 or a vector according to claim 9, into a cell *ex vivo.*

**12.** A pharmaceutical composition comprising a plurality of cells according to claim 10.

**13.** A pharmaceutical composition according to claim 12 for use in treating and/or preventing a disease.


**Patentansprüche**

**1.** Nukleinsäurekonstrukt, umfassend die folgende Struktur:

$$A - X - B,$$

in der

X für eine Nukleinsäuresequenz steht, die eine Spaltungsstelle codiert; und
A und B für Nukleinsäuresequenzen stehen, die einen ersten und einen zweiten chimären Antigenrezeptor (CAR) codieren, wobei jeder CAR Folgendes umfasst:

(i) eine antigenbindende Domäne;
(ii) einen Spacer,
(iii) eine Transmembrandomäne; und
(iv) eine Endodomäne,

wobei die Antigenbindungsdomänen des ersten und zweiten CAR an unterschiedliche Antigene binden, wobei der Spacer des ersten CAR vom Spacer des zweiten CAR verschieden ist, so dass bei Bindung des ersten CAR und des zweiten CAR an ihre jeweiligen Zielantigene an der Zelloberfläche der erste CAR und zweite CAR auf der Zellmembran räumlich getrennt werden; wobei es sich bei dem ersten oder dem zweiten CAR um einen aktivierenden CAR, der eine aktivierende Endodomäne umfasst, und bei dem jeweils anderen CAR um einen Inhibitor-CAR, der eine Ligation-off-Inhibitor-Endodomäne umfasst, handelt, wobei der Inhibitor-CAR T-Zell-Aktivierung durch den aktivierenden CAR in Abwesenheit von Inhibitor-CAR-Ligation hemmt, jedoch T-Zell-Aktivierung durch den aktivierenden CAR nicht signifikant hemmt, wenn der Inhibitor-CAR ligiert ist;
und wobei:

(a) der erste und/oder zweite CAR ein Intrazelluläre-Retention-Signal umfasst; und/oder
(b) das Signalpeptid des ersten oder zweiten CAR eine oder mehrere Mutation(en) umfasst, so dass es weniger hydrophobe Aminosäuren aufweist.

**2.** Nukleinsäurekonstrukt nach Anspruch 1, wobei der erste und/oder zweite CAR ein Intrazelluläre-Retention-Signal umfasst, das den CAR zu einem Lysosomen-, Endosomen- oder Golgi-Kompartiment leitet.

**3.** Nukleinsäurekonstrukt nach Anspruch 1, wobei der erste und/oder der zweite CAR ein Intrazelluläre-Retention-Signal umfasst, das aus der folgenden Gruppe ausgewählt ist: einem Endozytosesignal; einem Golgi-Retention-Signal; einem TGN(Trans-Golgi Network)-Recycling-Signal; einem Endoplasmatisches-Retikulum(ER)-Retention-Signal; und einem Lysosomensortierungssignal.

**4.** Nukleinsäurekonstrukt nach Anspruch 1, wobei das Signalpeptid des zweiten CAR eine oder mehrere Mutation (en) umfasst, so dass es weniger hydrophobe Aminosäuren als a) seine Wildtypsequenz oder b) das Signalpeptid des anderen CAR aufweist.

5. Nukleinsäurekonstrukt nach Anspruch 4, wobei das Signalpeptid des aktivierenden CAR eine oder mehrere Mutation(en) umfasst, so dass bei Expression des Nukleinsäurekonstrukts in einer Zelle das relative Expressionsniveau des aktivierenden CAR an der Zelloberfläche im Vergleich mit dem Inhibitor-CAR reduziert ist.

6. Nukleinsäurekonstrukt nach Anspruch 4, wobei die durch Mutation entfernte(n) oder ersetzte(n) hydrophobe(n) Aminosäure (n) aus der folgenden Gruppe ausgewählt ist/sind: Alanin (A); Valin (V); Isoleucin (I); Leucin (L); Methionin (M) ; Phenylalanin (P); Tyrosin (Y); Tryptophan (W).

7. Nukleinsäurekonstrukt nach Anspruch 4, wobei die durch Mutation entfernte(n) oder ersetzte(n) hydrophobe(n) Aminosäure (n) aus der folgenden Gruppe ausgewählt ist/sind: Valin (V); Isoleucin (I); Leucin (L); und Tryptophan (W).

8. Nukleinsäurekonstrukt nach Anspruch 1, wobei sich das Signalpeptid des ersten und des zweiten CAR in ihrer Anzahl hydrophober Aminosäuren unterscheiden und wobei ein Signalpeptid bis zu fünf hydrophobe Aminosäuren mehr als das andere Signalpeptid umfasst.

9. Vektor, umfassend ein Nukleinsäurekonstrukt nach einem vorhergehenden Anspruch.

10. Zelle, umfassend ein Nukleinsäurekonstrukt nach einem der Ansprüche 1 bis 8 oder einen Vektor nach Anspruch 9.

11. Verfahren zur Erzeugung einer Zelle nach Anspruch 10, das den Schritt umfasst, bei dem ein Nukleinsäurekonstrukt nach einem der Ansprüche 1 bis 8 oder ein Vektor nach Anspruch 9 in eine Zelle ex vivo eingeführt wird.

12. Pharmazeutische Zusammensetzung, umfassend mehrere Zellen nach Anspruch 10.

13. Pharmazeutische Zusammensetzung nach Anspruch 12 zur Behandlung und/oder Vorbeugung einer Krankheit.


**Revendications**

1. Construction d'acide nucléique, comprenant la structure suivante :

A - X - B

dans laquelle

X est une séquence d'acide nucléique qui code pour un site de clivage ; et
A et B sont des séquences d'acide nucléique qui codent pour un premier et un deuxième récepteur antigénique chimérique (CAR), chaque CAR comprenant :

(i) un domaine de fixation à l'antigène ;
(ii) un espaceur ;
(iii) un domaine transmembranaire ; et
(iv) un endo-domaine ;

où les domaines de fixation à l'antigène du premier et du deuxième CAR se fixent à des antigènes différents, où l'espaceur du premier CAR possède une taille différente de l'espaceur du deuxième CAR de sorte que, lorsque le premier CAR et le deuxième CAR se fixent à leurs antigènes cibles respectifs au niveau de la surface cellulaire, le premier CAR et le deuxième CAR deviennent séparés sur le plan spatial sur la membrane cellulaire ; où l'un parmi le premier ou le deuxième CAR est un CAR d'activation comprenant un endo-domaine d'activation et l'autre CAR est un CAR d'inhibition comprenant un endo-domaine d'inhibition en l'absence de ligature, lequel CAR d'inhibition inhibe l'activation des lymphocytes T par le CAR d'activation en l'absence d'une ligature du CAR d'inhibition, mais n'inhibe pas de manière significative l'activation des lymphocytes T par le CAR d'activation lorsque le CAR d'inhibition est ligaturé ;
et où :

(a) le premier et/ou le deuxième CAR comprend un signal de rétention intracellulaire ; et/ou
(b) le peptide signal du premier ou du deuxième CAR comprend une ou plusieurs mutations de façon à posséder moins d'acides aminés hydrophobes.

**2.** Construction d'acide nucléique selon la revendication 1, dans laquelle le premier et/ou le deuxième CAR comprend un signal de rétention intracellulaire qui dirige le CAR vers un compartiment lysosomal, endosomal ou du Golgi.

**3.** Construction d'acide nucléique selon la revendication 1, dans laquelle le premier et/ou le deuxième CAR comprend un signal de rétention intracellulaire choisi parmi le groupe suivant : un signal d'endocytose ; un signal de rétention dans le Golgi ; un signal de recyclage dans le réseau trans-golgien (TGN) ; un signal de rétention dans le réticulum endoplasmique (RE) ; et un signal de triage lysosomal.

**4.** Construction d'acide nucléique selon la revendication 1, dans laquelle le peptide signal du premier ou du deuxième CAR comprend une ou plusieurs mutations, de façon à posséder moins d'acides aminés hydrophobes que a) sa séquence de type sauvage ou b) le peptide signal de l'autre CAR.

**5.** Construction d'acide nucléique selon la revendication 4, dans laquelle le peptide signal du CAR d'activation comprend une ou plusieurs mutations, de façon à posséder moins d'acides aminés hydrophobes, de sorte que, lorsque la construction d'acide nucléique est exprimée dans une cellule, le taux d'expression relatif du CAR d'activation au niveau de la surface cellulaire est réduit par rapport au CAR d'inhibition.

**6.** Construction d'acide nucléique selon la revendication 4, dans laquelle le ou les acides aminés hydrophobes éliminés ou remplacés par mutation sont choisis dans le groupe constitué par : Alanine (A) ; Valine (V) ; Isoleucine (I) ; Leucine (L) ; Méthionine (M) ; Phénylalanine (P) ; Tyrosine (Y) ; Tryptophane (W) .

**7.** Construction d'acide nucléique selon la revendication 4, dans laquelle le ou les acides aminés hydrophobes éliminés ou remplacés par mutation sont choisis dans le groupe constitué par : Valine (V) ; Isoleucine (I) ; Leucine (L) ; et Tryptophane (W).

**8.** Construction d'acide nucléique selon la revendication 1, dans laquelle les peptides signaux du premier et du deuxième CAR diffèrent au niveau de leur nombre d'acides aminés hydrophobes, et où l'un des peptides signaux comprend jusqu'à cinq acides aminés hydrophobes supplémentaires par rapport à l'autre peptide signal.

**9.** Vecteur, comprenant une construction d'acide nucléique selon l'une quelconque des revendications précédentes.

**10.** Cellule, comprenant une construction d'acide nucléique selon l'une quelconque des revendications 1 à 8 ou un vecteur selon la revendication 9.

**11.** Méthode de préparation d'une cellule selon la revendication 10, comprenant l'étape consistant à introduire une construction d'acide nucléique selon l'une quelconque des revendications 1 à 8 ou un vecteur selon la revendication 9, dans une cellule *ex vivo*.

**12.** Composition pharmaceutique, comprenant une pluralité de cellules selon la revendication 10.

**13.** Composition pharmaceutique selon la revendication 12, pour une utilisation destinée au traitement et/ou à la prévention d'une maladie.

FIG. 1

FIG. 2

FIG. 3

FIG. 4

EP 3 288 970 B1

FIG. 5

FIG. 6

a)

b)

FIG. 7

FIG. 8

B. scFv swap

Signalling CAR

Inhibitory CAR

FIG. 8 (Continued)

EP 3 288 970 B1

EP 3 288 970 B1

C. Similar spacers

FIG. 8 (Continued)

FIG. 9

FIG. 9 (Continued)

FIG. 10

FIG. 10 (Continued)

FIG. 11

EP 3 288 970 B1

FIG. 12

(a)

CD3z

(b)

Hinge

(c)

Stalk from:
CD8  CD28

-S-S-

(d)

Human IgG1
HCH2CH3

-S-S-
-S-S-
-S-S-  -S-S-
-S-S-  -S-S-

(e)

Human IgM
HCH2CH3CH4

-S-S-
-S-S-
-S-S-  -S-S-
-S-S-  -S-S-
-S-S-  -S-S-

FIG. 13

aCD33-(spacer)-dCD148

|  | Hinge | huCD8STK | huCD28STK | huIgG-Fc | huIgM-Fc |
|---|---|---|---|---|---|
| Hinge | NO STIM | ? | ? | AND | AND |
| huCD8STK | ? | NO STIM | NO STIM | AND | AND |
| huCD28STK | ? | NO STIM | NO STIM | AND | AND |
| huIgG-Fc | ? | AND | ? | NO STIM | NO STIM |
| huIgM-Fc | ? | ? | ? | ? | NO STIM |

aCD19-(spacer)-CD28tmZ

FIG. 14

(a)

(b)

IRES

(c)

2A

FIG. 15

FIG. 16

FIG. 16 (Continued)

Extracellular Staining

A. SupT1.NT

B. SupT1.Tyrp1_WT

C. SupT1.Tyrp1-L-eGFP

D. SupT1.Tyrp1
(LM-TM)-L-eGFP

E. SupT1.Tyrp1(LM-
TM)-L-eGFP-Tyrp1(CP)

TA99-PE

aCD34-APC

FIG. 16 (Continued)

FIG. 17

FIG. 17 (Continued)

C

FIG. 17 (Continued)

EP 3 288 970 B1

EP 3 288 970 B1

FIG. 18

293T cells:

FIG. 19

1) processing of signal peptide by signal peptide peptidase

2) membrane-inserted signal peptide

signal sequences affect protein targeting, insertion and their cleavage in a qualitative and quantitative manner

3) release of a signal peptide from the ER membrane

FIG. 20

FIG. 21

FIG. 22

FIG. 23

FIG. 24

FIG. 25

FIG. 26

EP 3 288 970 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2014053452 W
- US 7052906 B1
- WO 2000063372 A
- WO 2013153391 A
- GB 1507115 A

### Non-patent literature cited in the description

- **LAMERS et al.** *Mol. Ther.,* 2013, vol. 21, 904-912
- **WLIKIE et al.** *J Clin Immunol,* 2012, vol. 32, 1059-1070
- **KLOSS et al.** *Nature Biotechnol.,* 2013, vol. 31, 71-75
- **ROSSIG.** *Blood,* 15 March 2002, vol. 99 (6), 2009-16
- **WILKIE et al.** *J. Clin. Immunol.,* 2012, vol. 32, 1059-1070
- **KLOSS et al.** *Nat. Biotechnol.,* 2013, vol. 31, 71-75
- **CABEZUDO et al.** *Haematologica,* 1999, vol. 84, 413
- **DONNELLY et al.** *J. Gen. Virol.,* 2001, vol. 82, 1027-1041
- **BONIFACINO ; TRAUB.** *Ann. Rev. Biochem.,* 2003, vol. 72, 395-447
- **BRAULKE ; BONIFACINO.** *Biochimica and Bio-physica Acta,* 2009, vol. 1793, 605-614
- **GRIFFITH.** *Current Biology,* 2001, vol. 11, R226-R228
- **MELLMAN ; NELSON.** *Nat Rev Mol Cell Biol.,* 2008, vol. 9, 833-845
- **DELL'ANGELICA ; PAYNE.** *Cell,* 2001, vol. 106, 395-398
- **SCHAFER et al.** *EMBO J.,* 1995, vol. 14, 2424-2435
- **TREJO.** *Mol. Pharmacol.,* 2005, vol. 67, 1388-1390
- **PELHAM.** *Meth. Enzymol.,* 2000, vol. 327, 279-283
- **GODER ; SPIESS.** *FEBS Lett,* 2001, vol. 504, 87-93
- Bonifacino & Traub. *Annu. Rev. Biochem.,* 2003, vol. 72, 395-447
- **SCHEEL et al.** *J. Biol. Chem.,* 1993, vol. 268, 7465
- **TEASDALE ; JACKSON.** *Annu. Rev. Cell Dev. Biol.,* 1996, vol. 12, 27